# EUROPÄISCHE PATENTSCHRIFT

(11) **EP 1 692 105 B1**
(45) Veröffentlichungstag und Bekanntmachung des Hinweises auf die Patenterteilung: **14.05.2008**
(21) Anmeldenummer: 04803188.4
(22) Anmeldetag: 19.11.2004
(51) Int. Cl.: C07D 209/46, C07C 233/66

(54) **VERFAHREN ZUR HERSTELLUNG VON (3-OXO-2,3-DIHYDRO-1H-ISOINDOL-1-Yl)-ACETYLGUANIDIN-DERIVATEN**
METHOD FOR PRODUCING (3-OXO-2,3-DIHYDRO-1H-ISOINDOL-1-YL) ACETYLGUANIDINE DERIVATIVES
PROCEDES POUR PRODUIRE DES DERIVES DE (3-OXO-2,3-DIHYDRO-1H-ISOINDOL-1-YL)-ACETYLGUANIDINE

(30) Priorität: 02.12.2003 DE 10356717
(43) Veröffentlichungstag der Anmeldung: 23.08.2006
(73) Patentinhaber: Sanofi-Aventis Deutschland GmbH, 65929 Frankfurt am Main (DE)
(72) Erfinder: SCHUBERT, Gerrit, 65779 Kelkheim (DE); RIEKE-ZAPP, Joerg, 60325 Frankfurt (DE); KEIL, Johannes, 65929 Frankfurt (DE); KLEEMANN, Heinz-Werner, 65474 Bischofsheim (DE); HANNA, Reda, Allentown, PA 18103 (US); HUANG, Bao-Guo, Bridgewater, NJ 08807 (US); WU, Xiao-Dong, Bridgewater, NJ 08807 (US); GOURAUD, Yves, F-93370 Montfermeil (FR)
(86) Internationale Anmeldenummer: PCT/EP2004/013153
(87) Internationale Veröffentlichungsnummer: WO 2005/054195

(56) Entgegenhaltungen:
- WO-A-02/081443
- WO-A-03/101450

## Beschreibung

Die vorliegende Erfindung betrifft Verfahren zur Herstellung von (3-Oxo-2,3-dihydro-1H-isoindol-1-yl)-acetylguanidin-Derivaten über 3-Hydroxy-2,3-dihydro-1H-isoindol-1-on-Derivate oder 3-(2-Carbamoyl-phenyl)-acrylsäureester-Derivate als Zwischenstufen, ein Verfahren zur Racematspaltung, sowie Zwischenprodukte der erfindungsgemäßen Verfahren.

Dokument WO02/081443 beschreibt (3-Oxo-2,3-dihydro-1 H-isoindol-5-yl)guanidin-Derivate als MC4-R-Agonisten und die zur Behandlung von Diabetes verwendet werden.

(3-Oxo-2,3-dihydro-1H-isoindol-1-yl)-acetylguanidin-Derivate der Formel I 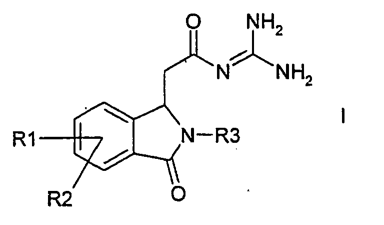 sind NHE1-Inhibitoren und sind in WO 03/101450 beschrieben. Die dort beschriebenen Synthesen führen jedoch zu racemischen Regioisomerengemischen, was aufwendige Trennverfahren erfordert und die Ausbeute an der gewünschten Verbindung vermindert. Bisher war der Zugang zu den Enantiomeren nur durch eine aufwendige chromatographische Trennung an chiralen Trägern möglich. Der Substanzdurchsatz bei chromatographischen Trennungen ist jedoch beschränkt.

Es bestand daher ein großes Interesse, regioselektive Herstellungsverfahren für (3- . Oxo-2,3-dihydro-1H-isoindol-1-yl)-acetylguanidin-Derivate sowie Verfahren zur Gewinnung der Enantiomeren zu finden. Die verbesserte, regioselektive Herstellung der racemischen (3-Oxo-2,3-dihydro-1 H-isoindol-1-yl)-acetylguanidin-Derivate gelang auf zwei unabhängigen Wegen, die in Schema 1 und Schema 3 dargestellt sind. Die Spaltung der Racemate gelang durch Kristallisation als Salze von 2,3-O-acylierten D- oder L-Weinsäuren, wie in Schema 5 dargestellt. Durch schonende basenkatalysierte Racemisierung des jeweils unerwünschten Enantiomeren ist dabei eine weitgehende Umwandlung des Racemats in das gewünschte Enantiomer möglich. Die genannten

Verfahren ermöglichen die einfache Herstellung von enantiomer angereicherten oder enantiomerenreinen (3-Oxo-2,3-dihydro-1H-isoindol-1-yl)-acetylguanidin-Derivaten. Durch die neuen Verfahren ist nun die einfache Herstellung großer Substanzmengen der Verbindungen der Formel I im industriellen Maßstab möglich.

Die vorliegende Erfindung betrifft somit ein Verfahren zur Herstellung von Verbindungen der Formel I, 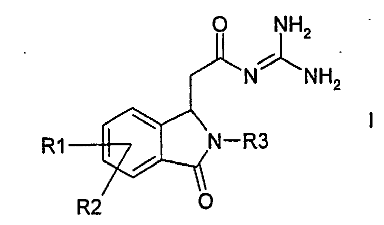 wobei bedeuten
- R1 und R2: unabhängig voneinander Wasserstoff, F, Cl, Trifluormethoxy, 2,2,2-Trifluorethoxy, Trifluormethyl, 2,2,2-Trifluorethyl oder Alkyl mit 1, 2, 3 oder 4 C-Atomen;
- R3: Alk-R4 oder Trifluormethyl;
Alk Alkyl mit 1, 2, 3 oder 4 C-Atomen;
R4 Wasserstoff, Trifluormethyl oder Cycloalkyl mit 3, 4, 5, 6 oder 7 C-Atomen;
sowie deren Salzen;
dadurch gekennzeichnet, dass wie in Schema 1 dargestellt 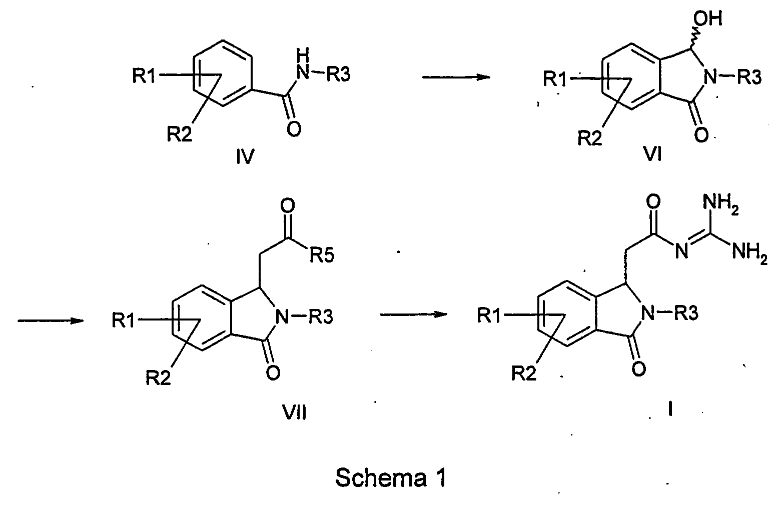
a) das Amid der Formel IV formyliert und dann zur Verbindung der Formel VI cyclisiert wird,
b) die Verbindung der Formel VI mit einem Alkoxycarbonylmethylentriphenylphosphoran, mit einem 1-Alkoxy-1-trimethylsiloxyethylen oder mit einem Trialkylphosphonoacetat zur Verbindung der Formel VII umgesetzt wird, und
c) die Verbindung der Formel VII mit Guanidin zur Verbindung der Formel I umgesetzt wird,
wobei in den Verbindungen der Formeln IV, VI und VII
R1 bis R3 definiert sind wie in Formel I und
R5 Alkoxy mit 1, 2, 3 oder 4 C-Atomen ist;
sowie deren Salze.

Gegenstand der Erfindung ist auch ein Verfahren zur Herstellung von Verbindungen der Formel I,
wobei bedeuten
- R1 und R2: unabhängig voneinander Wasserstoff, F, Cl, Trifluormethoxy, 2,2,2-Trifluorethoxy, Trifluormethyl, 2,2,2-Trifluorethyl oder Alkyl mit 1, 2, 3 oder 4 C-Atomen;
- R3: Alk-R4 oder Trifluormethyl;
Alk Alkyl mit 1, 2, 3 oder 4 C-Atomen;
R4 Wasserstoff, Trifluormethyl oder Cycloalkyl mit 3, 4, 5, 6 oder 7 C-Atomen;
sowie deren Salzen;
dadurch gekennzeichnet, dass wie in Schema 2 dargestellt 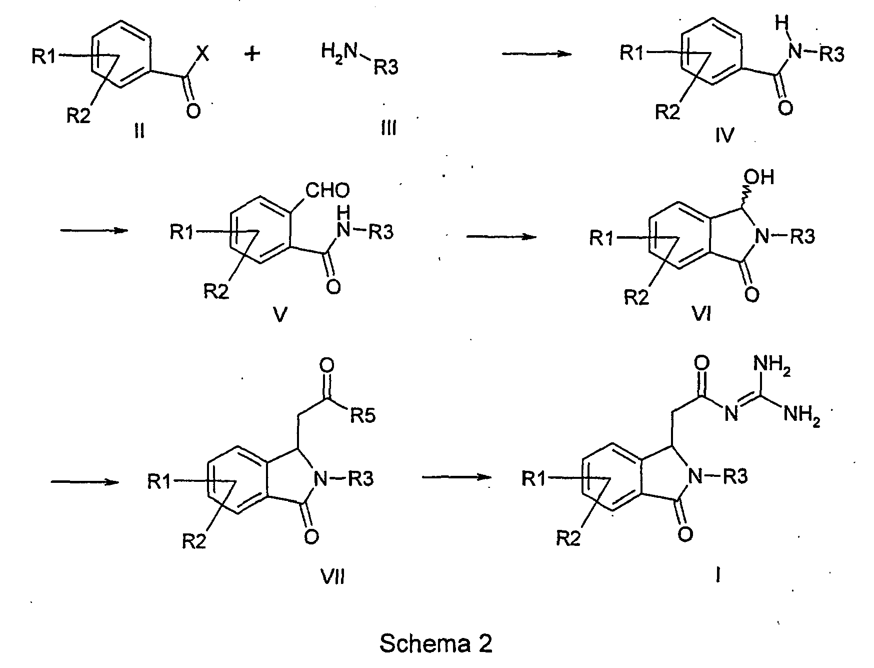
a) die Verbindung der Formel II mit dem Amin der Formel III zum Amid der Formel IV umgesetzt wird,
b) das Amid der Formel IV an ortho-Position zur Amidfunktion zu dem Formyl-amid der Formel V formyliert wird,
c) das Formyl-amid der Formel V zur Verbindung der Formel VI cyclisiert wird,
d) die Verbindung der Formel VI mit einem Alkoxycarbonylmethylentriphenylphosphoran, mit einem 1-Alkoxy-1-trimethyfsiloxyethyfen oder mit einem Trialkylphosphonoacetat zur Verbindung der Formel VII umgesetzt wird und
e) die Verbindung der Formel VII mit Guanidin zur Verbindung der Formel I umgesetzt wird,
wobei in den Verbindungen der Formeln II, III, IV, V, VI und VII
R1 bis R3 definiert sind wie in Formel I,
R5 Alkoxy mit 1, 2, 3 oder 4 C-Atomen ist und
X Cl, Br, OH oder Alkoxy mit 1, 2, 3 oder 4 C-Atomen ist;
sowie deren Salze.

Die Verbindung der Formel 11 wird üblicherweise in einem inerten Lösungsmittel wie einem Ether, Kohlenwasserstoff oder halogeniertem Kohlenwasserstoff, zum Beispiel Dichlormethan, bei-einer Temperatur zwischen -30°C und dem Siedepunkt des Lösungsmittels, bevorzugt bei RT, mit einem Amin der Formel III, gegebenenfalls in Gegenwart eines Aktivierungsmittels, zum Amid der Formel IV umgesetzt.

Die ortho-Formylierung kann zum Beispiel durchgeführt werden, indem eine Alkylmetall-Verbindung, zum Beispiel eine Alkyllithium-Verbindung, bevorzugt t-BuLi, mit einem Komplexliganden, bevorzugt TMEDA, in einem inerten Lösungsmittel wie einem Ether oder Kohlenwasserstoff, zum Beispiel THF, bei einer Temperatur zwischen -100°C und 0°C, bevorzugt zwischen -80 °C und -50 °C , vorgelegt wird. Dann wird das Amid der Formel IV zugegeben und über einen Zeitraum zwischen 10 Minuten und 10 Stunden, bevorzugt zwischen 10 Minuten und 60 Minuten, bei einer Temperatur zwischen -100°C und 0°C, bevorzugt zwischen -80 °C und -50 °C, deprotoniert. Anschließend wird ein Formylierungs-Mittel, bevorzugt DMF, zugegeben und bei einer Temperatur zwischen -100°C und 40°C, bevorzugt zwischen -80 °C und Raumtemperatur, mit dem Anion zur Reaktion gebracht. Bevorzugt läßt man die Lösung nach Zugabe des DMF während eines Zeitraumes von 10 Minuten bis 3 Stunden, zum Beispiel innerhalb 30 Minuten, auf RT kommen. Intermediär entstandenes Amid der Formel V cyclisiert dabei im allgemeinen direkt zum Isoindolon der Formel VI.

Das Isoindolon der Formel VI wird mit einem (C₁-C₄)-Alkoxycarbonylmethylen-triphenylphosphoran in einem inerten Lösungsmittel wie einem Ether, Kohlenwasserstoff oder halogeniertem Kohlenwasserstoff, beispielsweise Toluol, bei einer Temperatur zwischen 0°C und dem Siedepunkt des Lösungsmittels, bevorzugt zwischen 20 °C und dem Siedepunkt des Lösungsmittels, oder mit einem Tri-(C₁-C₄)-alkylphosphonacetat in Gegenwart einer Base, beispielsweise Natriumhydrid, in einem inerten Lösungsmittel wie einem Ether, Kohlenwasserstoff oder halogeniertem Kohlenwasserstoff, beispielsweise 1,2-Dimethoxyethan, bei einer Temperatur zwischen 0°C und dem Siedepunkt des Lösungsmittels, bevorzugt zwischen 20°C und dem Siedepunkt des Lösungsmittels, umgesetzt. Alternativ wird das Isoindolon der Formel VI mit einem 1-(C₁-C₄)-Alkyloxy-1-trimethylsiloxyethylen in Gegenwart einer Lewis-Säure, beispielsweise Titan(IV)chlorid oder Trimethylsilyltriflat, in einem inerten Lösungsmittel wie einem Ether, Kohlenwasserstoff oder halogeniertem Kohlenwasserstoff, beispielsweise Dichlormethan, bei einer Temperatur zwischen -80°C und dem Siedepunkt des Lösungsmittels, bevorzugt bei einer Temperatur zwischen -80 °C und 20 °C, umgesetzt (Synth. Cömmun. 1987, 17,1).

Der Ester der Formel VII kann nach allgemein bekannten Verfahren mit Guanidin zum Acylguanidin der Formel I umgesetzt werden. Die Umsetzung erfolgt vorzugsweise in dem Fachmann bekannter Weise in einem protischen oder aprotischen, polaren, aber inerten organischen Lösungsmittel. Dabei haben sich beispielsweise bei der Umsetzung des Methylesters (Formel VII; R5 = OCH₃) mit Guanidin Lösungsmittel wie Methanol, Isopropanol oder THF bei Temperaturen von 20°C bis zur Siedetemperatur dieser Lösungsmittel bewährt. Bei den meisten Umsetzungen von Verbindungen der Formel VII mit salzfreiem Guanidin wird beispielsweise in aprotischen inerten Lösungsmitteln, zum Beispiel Ethern wie THF, Dimethoxyethan oder Dioxan, gearbeitet. Aber auch Wasser kann unter Gebrauch einer Base wie beispielsweise NaOH als Lösungsmittel bei der Umsetzung von Verbindungen der Formel VII mit Guanidin verwendet werden. Bei der Umsetzung von Verbindungen der Formel VII mit Salzen des Guanidins, wie zum Beispiel Guanidin-Hydrochlorid, erfolgt die Umsetzung üblicherweise in Gegenwart einer Base, zum Beispiel Kalium-tert-butoxid, Natriummethylat oder Natriumethylat in einem inerten Lösungsmittel, wie Dimethylformamid, NMP, 2-Propanol bei einer Temperatur zwischen 20°C und dem Siedepunkt des Lösungsmittels.

Neben den Carbonsäureestern der Formel VII lassen sich auch weitere aktivierte Säurederivate bei der Umsetzung mit Guanidin einsetzen, beispielsweise Carbonsäurechloride, -thioester oder -anhydride. Es kann auch eine Aktivierung der Carbonsäure mit zum Beispiel DCC erfolgen. Die aktivierten Säurederivate lassen sich in dem Fachmann bekannter Weise direkt aus den zugrundeliegenden Carbonsäureestern der Formel VII oder aus den entsprechenden Carbonsäuren herstellen, die aus den Estern durch übliche Hydrolysemethoden erhalten werden können. Eine Reihe geeigneter Methoden zur Herstellung von aktivierten Carbonsäurederivaten sind unter Angabe von Quellenliteratur in J. March, Advanced Organic Chemistry, Third Edition (John Wiley & Sons, 1985, S. 350) angegeben.

Die in Schema 1 und 2 beschriebenen Verfahrensschritte können unabhängig voneinander kontinuierlich oder diskontinuierlich erfolgen. Eine Aufarbeitung des Reaktionsgemisches kann nach jedem der Verfahrensschritte erfolgen. Die Aufarbeitung und gewünschtenfalls die Reinigung der Produkte erfolgt nach den üblichen Methoden wie Extraktion, pH-Trennung, Chromatographie oder Kristallisation und den üblichen Trocknungen.

Die Ausgangsverbindungen der Formeln II und III sind käuflich erhältlich oder können nach oder analog zu in der Literatur beschriebenen, dem Fachmann bekannten Verfahren hergestellt werden.

Beansprucht wird auch ein Verfahren zur Herstellung von Verbindungen der Formel I 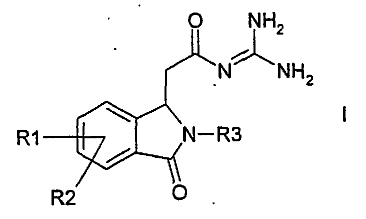 wobei bedeuten
- R1 und R2: unabhängig voneinander Wasserstoff, F, Cl, Trifluormethoxy, 2,2,2-Trifluorethoxy, Trifluormethyl, 2,2,2-Trifluorethyl oder Alkyl mit 1, 2, 3 oder 4 C-Atomen;
- R3: Alk-R4 oder Trifluormethyl; .
Alk Alkyl mit 1,2,3 oder 4 C-Atomen;
R4 Wasserstoff, Trifluormethyl oder Cycloalkyl mit 3, 4, 5, 6 oder 7 C-Atomen;
sowie deren Salzen;
dadurch gekennzeichnet, dass wie in Schema 3 dargestellt 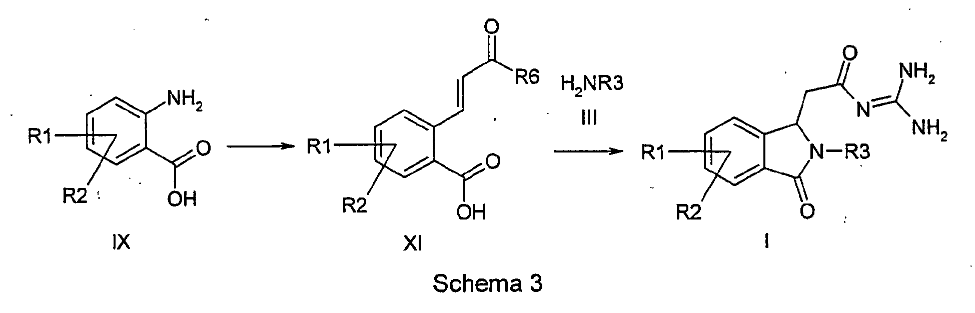
a) das Amin der Formel IX über ein Diazoniumsalz mit einem Acrylsäure-Alkylester zum Zimtsäurederivat der Formel XI umgesetzt wird,
b) die Verbindung der Formel XI mit dem Amin der Formel III und mit Guanidin zum Acylguanidin der Formel I umgesetzt wird,
wobei in den Verbindungen der Formeln III, IX und XI
R1 bis R3 definiert sind wie in Formel I und
R6 Alkoxy mit 1, 2, 3 oder 4 C-Atomen ist;
sowie deren Salze.

Die vorliegende Erfindung betrifft auch ein Verfahren zur Herstellung der Verbindungen der Formel I, 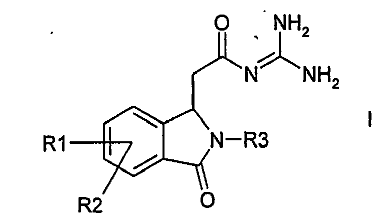 wobei bedeuten
- R1 und R2: unabhängig voneinander Wasserstoff, F, Cl, Trifluormethoxy, 2,2,2-Trifluorethoxy, Trifluormethyl, 2,2,2-Trifluorethyl oder Alkyl mit 1, 2, 3 oder 4 C-Atomen;
- R3: Alk-R4 oder Trifluormethyl;
Alk Alkyl mit 1, 2, 3 oder 4 C-Atomen;
R4 Wasserstoff, Trifluormethyl oder Cycloalkyl mit 3, 4, 5, 6 oder 7 C-Atomen;
sowie deren Salze;
dadurch gekennzeichnet, dass wie in Schema 4 dargestellt 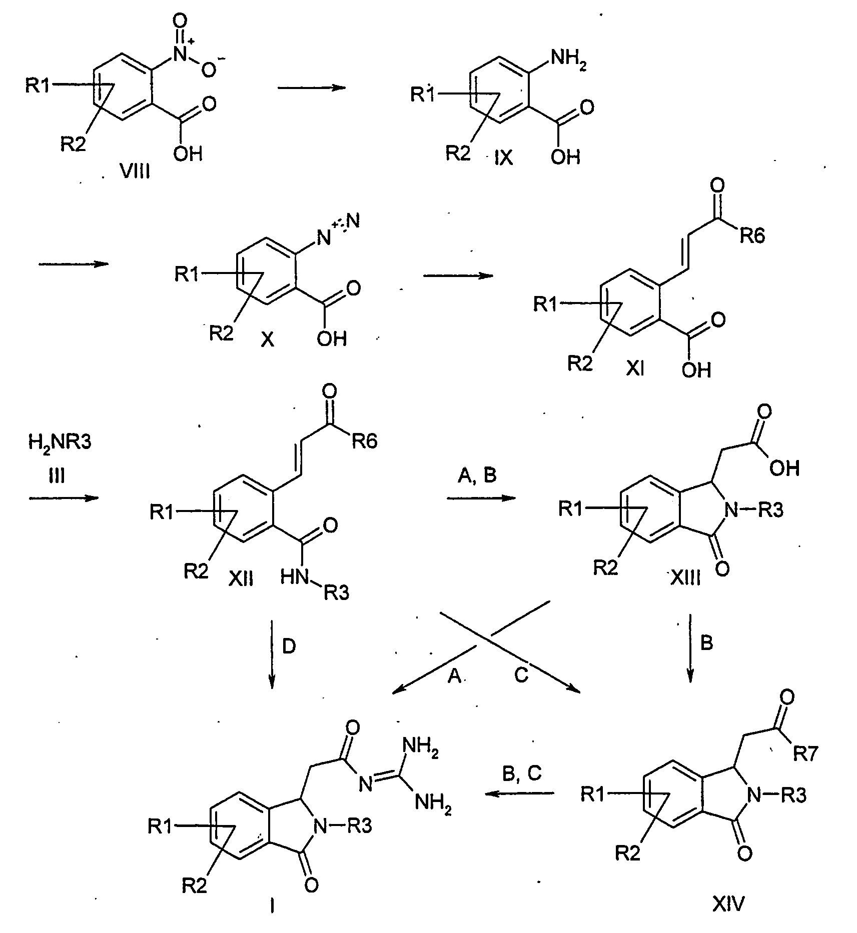 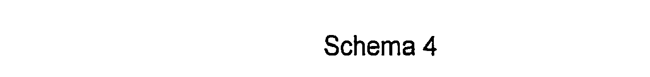
a) die Nitroverbindung der Formel VIII zum Amin der Formel IX umgesetzt wird,
b) das Amin der Formel IX zum Diazoniumsalz der Formel X umgesetzt wird,
c) das Diazoniumsalz der Formel X mit einem Acrylsäure-Alkylester zum Zimtsäurederivat der Formel XI umgesetzt wird,
d) die Verbindung der Formel XI zum Amid der Formel XII umgesetzt wird und
e) die Verbindung der Formel XII zum Acylguanidin der Formel I umgesetzt wird, entweder durch Umsetzung der Verbindung der Formel XII in Gegenwart einer Base zum Isoindolon-Derivat der Formel XIII und anschließend durch-Umsetzung mit Guanidin unter Aktivierung zum Acylguanidin der Formel I (Alternative A), oder nach Bildung des Isoindolon-Derivats der Formel XIII in Gegenwart einer Base aus der Verbindung der Formel XII durch Umwandlung der Verbindung der Formel XIII in den Ester der Formel XIV und anschließend durch Umsetzung mit Guanidin zum Acylguanidin der Formel I (Alternative B), oder
durch Umsetzung der Verbindung der Formel XII in Gegenwart einer starken Base zum Ester der Formel XIV und anschließend durch Umsetzung mit Guanidin zum Acylguanidin der Formel I (Alternative C), oder
durch direkte Umsetzung der Verbindung der Formel XII mit Guanidin in Gegenwart einer Base unter gleichzeitig erfolgender Guanylierung und Cyclisierung zum Isoindolon der Formel I (Alternative D),
wobei in den Verbindungen der Formeln VIII, IX, X, XI, XII, XIII und XIV
R1 bis R3 definiert sind wie in Formel I und
R6 und R7 unabhängig voneinander Alkoxy mit 1, 2, 3 öder 4 C-Atomen sind;
sowie deren Salze.

Die Nitroverbindung der Formel VIII kann nach bekannten Methoden (wie zum Beispiel beschrieben in "Houben-Weyl, Methoden der organischen Chemie", Band XI/1, Stickstoffverbindungen II, Georg Thieme Verlag Stuttgart, 1957, S. 360ff) zum Anilin der Formel IX reduziert werden. Bevorzugt ist die katalytische Hydrierung, zum Beispiel mit Pd/C, beispielsweise mit 5% Pd/C oder 10% Pd/C, in einem Lösungsmittel wie zum Beispiel einem Alkohol, bevorzugt Ethanol, unter einer WasserstoffAtmosphäre von 1 bar bis 200 bar Druck, bevorzugt 1 bar bis 10 bar Druck.

Die anschließende Diazotierung des Anilins der Formel IX erfolgt in einem inerten Lösungsmittel, bevorzugt Ethanol, in Gegenwart einer Säure, deren Anion nicht selbst das Diazoniumion substituiert, wie zum Beispiel HBF₄ oder HPF₆, bevorzugt HBF₄, oder zu Beispiel H₂SO₄ und in Gegenwart eines Nitrits, bevorzugt NaNO₂, bei einer Temperatur zwischen -30°C und dem Siedepunkt des Lösungsmittels, bevorzugt zwischen 0°C bis 30°C.

Das Diazoniumsalz der Formel X wird bevorzugt direkt umgesetzt mit einem Acrylsäure-(C₁-C₄)-Alkylester, bevorzugt Acrylsäure-ethylester, in Gegenwart eines Palladium-Katalysators, bevorzugt Pd(OAc)₂, bei einer Temperatur zwischen 0°C und dem Siedepunkt des Lösungsmittels, bevorzugt zwischen 45°C bis 55°C, zum Zimtsäurederivat der Formel XI.

Die Benzoesäurefunktion der Verbindung der Formel XI kann nach dem Fachmann bekannten Methoden in das Amid der Formel XII überführt werden, bevorzugt über das Säurechlorid oder mit Hilfe von DCC. Diese Reaktion kann auch so geführt werden, dass das Amid der Formel XII im Reaktionsgemisch direkt zum Ester der Formel XIV cyclisiert wird, also die Reaktion von der Verbindung der Formel XI zum Ester der Formel XIV in einem Schritt durchgeführt wird. Dies kann entweder schon unter den basischen Reaktionsbedingungen der Amidbildung geschehen, oder die Cyclisierung kann durch den Zusatz einer Base wie beispielsweise Triethylamin, Hünig-Base oder Kalium-tertiärbutylat bewirkt werden. Eine weitere Alternative besteht darin, die Verbindung der Formel XI direkt in die Verbindung der Formel I umzusetzen, indem nacheinander Amidbildung, Cyclisierung und Guanidierung im selben Reaktionsgefäß durchgeführt werden, wobei die Reaktion ohne Isolierung von Zwischenstufen erfolgen kann.

Für die weitere Umsetzung der Verbindung der Formel XII zum Acylguanidin der Formel I ergeben sich 4 Alternativen:
Alternative A: Die Umsetzung des Amids der Formel XII erfolgt vorzugsweise mit wässriger Alkalilösung, bevorzugt wässriger NaOH-Lösung, in einem Lösungsmittel wie einem Alkohol, bevorzugt Methanol oder Ethanol, bei einer Temperatur zwischen -30°C und dem Siedepunkt des Lösungsmittels, bevorzugt bei RT. Es findet sowohl die Verseifung der Esterfunktion als auch die Cyclisierung zum Isoindolon-Derivat der Formel XIII statt. Die Verbindung der Formel XIII wird nach allgemein bekannten Verfahren (und wie zu Schema 1 beschrieben) zur Acylierung aktiviert, zum Beispiel über das Säurechlorid oder mit DCC, und man erhält das Acylguanidin der Formel I.
Alternative B: Wie bei Alternative A erfolgt die Synthese der Carbonsäure der Formel XIII. Anschließend wird mit Standard-Verfahren zur Esterherstellung, bevorzugt mit SOCl₂ in einem Alkohol wie Methanol oder Ethanol, beispielsweise der Methyl- oder der Ethylester der Formel XIV hergestellt. Die anschließende Umsetzung des Esters der Formel XIV erfolgt wie zu Schema 1 beschrieben zum Acylguanidin der Formel I.
Alternative C: Die Umsetzung des Amids der Formel XII erfolgt in einer Lösung einer starken Base, bevorzugt Methylat oder t-Butylat in einem Alkohol wie Methanol oder Ethanol, und man erhält den Methyl- oder den Ethylester der Formel XIV. Die Umsetzung des Esters der Formel XIV zum Acylguanidin der Formel I erfolgt wie zu Schema 1 beschrieben.
Alternative D: Das Amid der Formel XII wird unter üblichen Bedingungen zur Acylierung von Guanidin umgesetzt. Als Lösungsmittel wird ein inertes Lösungsmittel wie ein Ether, Kohlenwasserstoff oder halogenierter Kohlenwasserstoff, bevorzugt DMF, eingesetzt. Üblicherweise wird zunächst ein Guanidiniumsalz mit einer starken Base, bevorzugt KOtBu, umgesetzt, wobei das freie Guanidin freigesetzt wird. Das Gemisch wird zur Lösung der Verbindung der Formel XII in einem Lösungsmittel wie einem Alkohol, Ether, Kohlenwasserstoff oder halogeniertem Kohlenwasserstoff, beispielsweise DMF, NMP oder 2-Propanol, gegeben. Dabei treten gleichzeitig die Guanylierung und die Cyclisierung zum Isoindolon der Formel I ein. Eine Variante besteht darin, zeitlich aufeinanderfolgend mit einer katalytischen Menge einer starken Base, beispielsweise Kalium-tertiärbutylat oder Natriummethylat oder Natriumethylat in einem Lösungsmittel, beispielsweise DMF, NMP oder 2-Propanol, die Verbindung der Formel XI zur Verbindung der Formel XIV zu cyclisieren und dann in situ zur Verbindung der Formel I umzusetzen.

Bevorzugt ist die Alternative D, bei der man die Umsetzung vom Benzoesäurederivat der Formel XI im Eintopfverfahren bis zum Acylguanidin der Formel I durchführt.

Die in Schema 2 beschriebenen Verfahrensschritte können kontinuierlich oder diskontinuierlich erfolgen. Eine Aufarbeitung des Reaktionsgemisches kann nach jedem der Verfahrensschritte erfolgen. Die Aufarbeitung und gewünschtenfalls die Reinigung der Produkte erfolgt nach den üblichen Methoden wie Extraktion, pH-Trennung, Chromatographie oder Kristallisation und den üblichen Trocknungen.

Die Ausgangsverbindungen der Formeln III und VIII sind käuflich erhältlich oder können nach oder analog zu in der Literatur beschriebenen, dem Fachmann bekannten Verfahren hergestellt werden.

Gegenstand der Erfindung sind auch Verbindungen der Formel XII 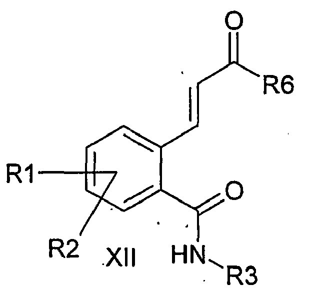 wobei bedeuten
- R1 und R2: unabhängig voneinander Wasserstoff, F, Cl, Trifluormethoxy, 2,2,2-Trifluorethoxy, Trifluormethyl, 2,2,2-Trifluorethyl oder Alkyl mit 1, 2, 3 oder 4 C-Atomen;
- R3: Alk-R4 oder Trifluormethyl;
Alk Alkyl mit 1, 2, 3 oder 4 C-Atomen;
R4 Wasserstoff, Trifluormethyl oder Cycloalkyl mit 3, 4, 5, 6 oder 7 C-Atomen;
- R6: Alkoxy mit 1, 2, 3 oder 4 C-Atomen;
sowie deren Salze.

Beansprucht wird auch die Verwendung der Verbindungen der Formel XII als Syntheseintermediat.

Verbindungen der Formel I in enantiomer angereicherter oder enantiomerenreiner Form können vorteilhaft durch ein neues Racematspaltungsverfahren hergestellt werden, das ebenfalls Gegenstand der vorliegenden Erfindung ist. Hierzu werden die Racemate der Verbindungen der Formel I als Salze von 2,3-O-acylierten D- oder L-Weinsäuren zur Kristallisation gebracht, wobei sich die Enantiomere im Kristallisat bzw. in der Mutterlauge anreichern. Anschließend werden die freien Basen wieder aus den Salzen freigesetzt.

Die vorliegende Erfindung betrifft somit ein Verfahren zur Isolierung der Verbindungen der Formel Ia und Ib 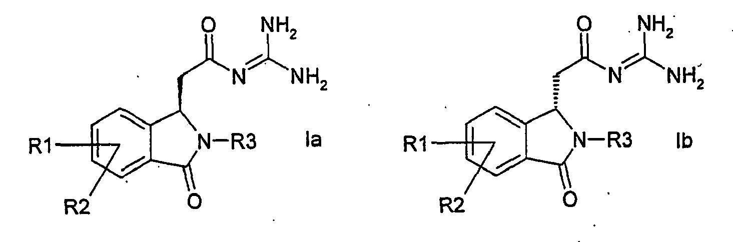 wobei bedeuten
- R1 und R2: unabhängig voneinander Wasserstoff, F; Cl, Trifluormethoxy, 2,2,2-Trifluorethoxy, Trifluormethyl, 2,2,2-Trifluorethyl oder Alkyl mit 1, 2, 3 oder 4 C-Atomen;
- R3: Alk-R4 oder Trifluormethyl;
Alk Alkyl mit 1, 2, 3 oder 4 C-Atomen;
R4 Wasserstoff, Trifluormethyl oder Cycloalkyl mit 3, 4, 5, 6 oder 7 C-Atomen;
sowie deren Salze;
dadurch gekennzeichnet, dass wie in Schema 5 dargestellt 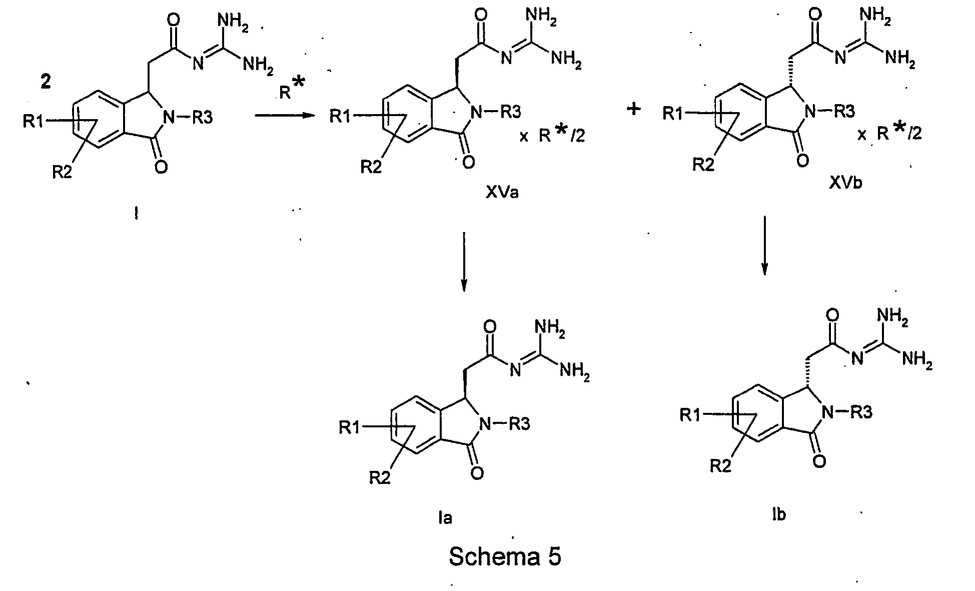
a) die Verbindung der Formel I zu Salzen von einer 2,3-O-acylierten D- oder L-Weinsäure umgesetzt und durch Kristallisation die beiden Salze der Formeln XVa und XVb getrennt erhalten werden, und
b) die freien Basen der Formeln Ia bzw. Ib aus den beiden Salzen der Formeln XVa bzw. XVb freigesetzt werden,
wobei in den Verbindungen der Formeln I, XVa und XVb
R1 bis R3 definiert sind wie in den Formeln Ia und Ib und
R* 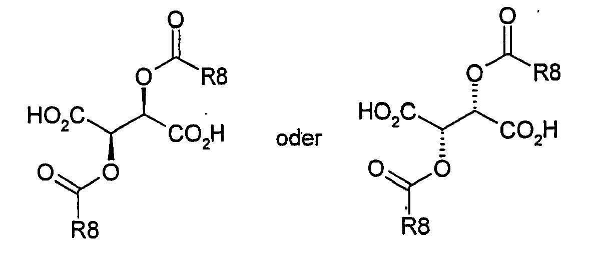 bedeutet und
R8 Alkyl mit 1, 2, 3, 4, 5 oder 6 C-Atomen oder Phenyl, das unsubstituiert oder substituiert ist mit 1, 2 oder 3 Substituenten aus der Gruppe F, Cl, Br, I, Alkyl mit 1, 2, 3 oder 4 C-Atomen oder Alkoxy mit 1, 2, 3 oder 4 C-Atomen, bedeutet.

Es wird auch das oben beschriebene Verfahren beansprucht, bei dem das unerwünschte Enantiomer der Formel Ia oder Ib wieder racemisiert wird.

Das Racemat der Verbindung der Formel I wird mit einem Weinsäurederivat R*, zum Beispiel O,O'-Dibenzoyl-D-Weinsäure, O,O'-Dibenzoyl-L-Weinsäure, Ö,O'-Di(4-methylbenzoyl)-L-Weinsäure, O,O'-Di(4-methylbenzoyl)-D-Weinsäure, O,O'-Di(4-methoxybenzoyl)-L-Weinsäure oder O,O'-Di(4-methoxybenzoyl)-D-Weinsäure, bevorzugt mit O,O'-Dibenzoyl-L-Weinsäure oder O,O'-Dibenzoyl-D-Weinsäure, zur Kristallisation gebracht in einem geeigneten Lösungsmittel wie beispielsweise in einem Ether, zum Beispiel Diethylether, Diisopropylether, Dimethoxyethan, Tetrahydrofuran oder Dioxan, in einem halogenierten Kohlenwasserstoff, zum Beispiel Dichlormethan, Trichlormethan, Tetrachlormethan, 1,2-Dichlorethan oder Trichlorethylen, in einem Alkohol, zum Beispiel Methanol, Ethanol, n-Propanol, 2-Propanol, Butanol, in einem Ester, zum Beispiel Ethylacetat oder Butylacetat, in Wasser, oder in Mischungen von Lösungsmitteln, bevorzugt in 2-Propanol, Dimethoxyethan oder Ethylacetat, bei einer Temperatur zwischen -10 °C und dem Siedepunkt des Lösungsmittels, bevorzugt bei 0 °C bis 40 °C. Eine Variante des Verfahrens besteht darin, Gemische von zwei oder mehreren 2,3-O-acylierter D- oder L-Weinsäuren derselben Konfiguration, die unterschiedliche Acylgruppen tragen, zur Trennung zu verwenden.

Die Salzbildung aus der Verbindung der Formel I und dem Weinsäurederivat R* kann unter Verwendung von äquivalenten Mengen erfolgen, d. h. es können 0,5 Mol des Weinsäurederivats R*, das zwei Carbonsäuregruppen enthält, pro Mol der Verbindung der Formel I eingesetzt werden. Die Verbindung der Formel I kann aber auch mit weniger als 0,5 Mol-Äquivalenten der 2,3-O-acylierten D- oder L-Weinsäure zur Kristallisation gebracht werden, beispielsweise mit 0,25 Mol bis 0,5 Mol Weinsäurederivat R* pro Mol der Verbindung der Formel I, insbesondere mit 0.25 Mol bis 0,3 Mol Weinsäurederivat R* pro Mol der Verbindung der Formel I. Das erwünschte Enantiomer kristallisiert dann in Form des Salzes der Formel XVa bzw. XVb aus und das unerwünschte Enantiomer ist in der Mutterlauge zum großen Teil in Form des Enantiomeren der Formel Ib bzw. la und nicht in Form des Salzes der Formel XVa bzw. XVb enthalten. Die Enantiomerenreinheit der Salze der Formeln XVa und XVb kann durch wiederholte Kristallisation oder durch Rühren der Erstkristalle mit frischem Lösungsmittel bei erhöhter Temperatur und anschließendes Abkühlen gesteigert werden.

Nach Trennung der beiden Salze der Formeln XVa und XVb bzw. Trennung des Salzes der Formel XVa oder XVb vom unerwünschten Enantiomer Ib bzw. la werden die enantiomer angereicherten Verbindungen der Formeln Ia und Ib anschließend üblicherweise durch Zusatz einer Hilfsbase, zum Beispiel einem Amin wie zum Beispiel Triethylamin, einer anorganischen Base wie NaHCO₃ Na₂CO₃ oder deren wässrigen Lösungen, aus den Salzen freigesetzt. Dabei wird üblicherweise in einem geeigneten Lösungsmittel gearbeitet wie beispielsweise in einem Ether, zum Beispiel Diethylether, Diisopropylether, Dimethoxyethan, Tetrahydrofuran oder Dioxan, in einem halogenierten Kohlenwasserstoff, zum Beispiel Dichlormethan, Trichlormethan, Tetrachlormethan, 1,2-Dichlorethan oder Trichlorethylen, in einem Alkohol, zum Beispiel Methanol, Ethanol, n-Propanol, 2-Propanol oder Butanol, in einem Ester, zum Beispiel Ethylacetat oder Butylacetat, oder in Wasser oder in Lösungsmittelmischungen, bevorzugt in Ethylacetat, 2-Propanol, Dichlormethan oder Wasser oder Mischungen davon, wobei das Reaktionsgemisch eine oder mehrere Phasen haben kann, bei einer Temperatur zwischen -10 °C und dem Siedepunkt des Lösungsmittels, bevorzugt bei 10 °C bis 40 °C. Dies kann beispielsweise so geschehen, dass das Salz in wässriger NaHCO₃-Lösung gelöst wird und dann mit einem organischen Lösungsmittel, zum Beispiel Ethylacetat, das Enantiomer der Formel Ia oder Ib extrahiert wird.

Das jeweils unerwünschte Enantiomere Ia oder Ib kann durch ein Racemisierungsverfahren wieder in das Racemat der Formel I überführt werden und steht somit für einen erneuten Racematspaltungsschritt zur Verfügung. Das unerwünschte Enantiomere wird hierbei vorzugsweise in einem Lösungsmittel wie einem Alkohol, zum Beispiel 2-Propanol, bei einer Temperatur zwischen -10 °C und dem Siedepunkt des Lösungsmittels, bevorzugt bei 0 °C bis 40 °C, mit geringen Mengen einer Base, zum Beispiel KOH, behandelt, das Reaktionsgemisch neutralisiert und nach wässrig-extraktiver Aufarbeitung das Racemat isoliert. Dieses Verfahren kann durch geeignete Wahl der Basenmenge und Reaktionstemperatur so durchgeführt werden, dass praktisch ausschließlich Racemisierung und keine chemische Veränderung der Substanz eintritt.

Gegenstand der vorliegenden Erfindung sind weiterhin Verbindungen der Formeln XVa und XVb 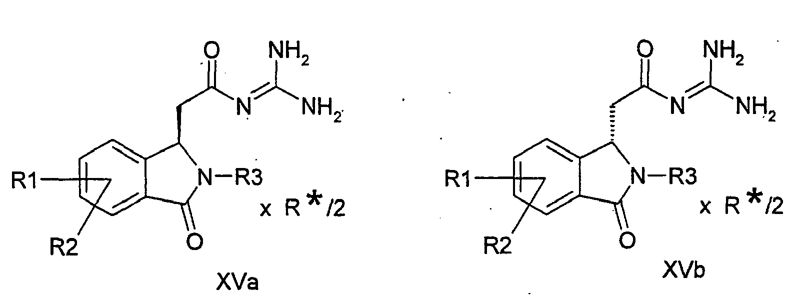 wobei bedeuten
- R1 und R2: unabhängig voneinander Wasserstoff, F, Cl, Trifluormethoxy, 2,2,2-Trifluorethoxy, Trifluormethyl, 2,2,2-Trifluorethyl oder Alkyl mit 1,2,3 oder 4 C-Atomen;
- R3: Alk-R4 oder Trifluormethyl;
Alk Alkyl mit 1, 2, 3 oder 4 C-Atomen;
R4 Wasserstoff, Trifluormethyl oder Cycloalkyl mit 3, 4, 5, 6 oder 7 C-Atomen;
- R*:

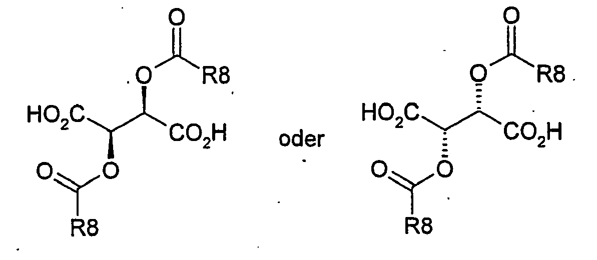

R8 Alkyl mit 1, 2, 3, 4, 5 oder 6 C-Atomen oder Phenyl, das unsubstituiert oder substituiert ist mit 1, 2 oder 3 Substituenten aus der Gruppe F, Cl, Br, I, Alkyl mit 1, 2, 3 oder 4 C-Atomen oder Alkoxy mit 1, 2, 3 oder 4 C-Atomen.

Enthalten die oben beschriebenen Verbindungen, beispielsweise die Verbindungen der Formeln I, Ia, Ib, VII, XIII, XIV, XVa oder XVb, ein oder mehrere Asymmetriezentren, so können diese unabhängig voneinander sowohl S- als auch R-konfiguriert sein, soweit nicht anders angegeben. Die Verbindungen können als optische Isomere, als Diastereomere, als Racemate oder als Gemische derselben vorliegen, soweit sie nicht genauer bezeichnet sind. An Doppelbindungen kann sowohl die E- als auch die Z-Konfiguration vorliegen, soweit nicht anders angegeben. Die vorliegende Erfindung umfasst alle tautomeren Formen der oben beschriebenen Verbindungen, beispielsweise der Verbindungen der Formeln I, Ia, Ib, XVa und XVb.

Alkylreste können geradkettig oder verzweigt sein. Dies gilt auch, wenn sie Substituenten tragen oder als Substituenten anderer Reste auftreten, beispielsweise in Fluoralkylresten oder Alkoxyresten. Beispiele für Alkylreste sind Methyl, Ethyl, n-Propyl, Isopropyl (= 1-Methylethyl), n-Butyl, Isobutyl (= 2-Methylpropyl), sec-Butyl (= 1-Methylpropyl), tert-Butyl (= 1,1-Dimethylethyl), n-Pentyl, Isopentyl, tert-Pentyl, Neopentyl und Hexyl. Bevorzugte Alkylreste sind Methyl, Ethyl, n-Propyl und Isopropyl, besonders bevorzugt Methyl oder Ethyl. In Alkylresten können ein oder mehrere, zum Beispiel 1, 2, 3, 4 oder 5, Wasserstoffatome durch Fluoratome substituiert sein. Beispiele für solche Fluoralkylreste sind Trifluormethyl, 2,2,2-Trifluorethyl und Pentafluorethyl, bevorzugt Trifluormethyl oder 2,2,2-Trifluorethyl. Substituierte Alkylreste können in beliebigen Positionen substituiert sein.

Beispiele für Cycloalkylreste sind Cyclopropyl, Cyclobutyl, Cyclopentyl, Cyclohexyl oder Cycloheptyl.

Phenylreste können unsubstituiert sein oder einfach oder mehrfach, zum Beispiel einfach, zweifach oder dreifach, durch gleiche oder verschiedene Reste substituiert sein. Wenn ein Phenylrest substituiert ist, trägt er bevorzugt einen oder zwei gleiche oder verschiedene Substituenten. In monosubstituierten Phenylresten kann sich der Substituent in der 2-Position, der 3-Position oder der 4-Position befinden. Zweifach substituiertes Phenyl kann in 2,3-Position, 2,4-Position, 2,5-Position, 2,6-Position, 3,4-Position oder 3,5-Position substituiert sein. In dreifach substituierten Phenylresten können sich die Substituenten in 2,3,4-Position, 2,3,5-Position, 2,4.,5-Position, 2,4,6-Position, 2,3,6-Position oder 3,4,5-Position befinden.

Die oben beschriebenen Verbindungen, beispielsweise die Verbindungen der Formeln I, Ia und Ib, können in Form ihrer Salze in die erfindungsgemäßen Verfahren eingesetzt und/oder in Form ihrer Salze isoliert werden. Salze können nach den üblichen Methoden, zum Beispiel durch Umsetzung mit Säuren oder Basen in einem Lösungsmittel oder durch Anionenaustausch oder Kationenaustausch aus anderen Salzen, erhalten werden. Als Säureadditionssalze, beispielsweise der Verbindungen der Formeln I, Ia und Ib, kommen dabei beispielsweise Halogenide, insbesondere Hydrochloride, Hydrobromide, Lactate, Sulfate, Citrate, Tartrate, Acetate, Phosphate, Methylsulfonate, Benzolsulfonate, p-Toluolsulfonate, Adipinate, Fumarate, Gluconate, Glutamate, Glycerolphosphate, Maleinate, Benzoate, Oxalate und Pamoate und Trifluoracetate in Frage. Im Fall der Herstellung von Wirkstoffen sind physiologisch verträgliche Salze und pharmazeutisch verträgliche Salze bevorzugt. Als Beispiele seien Salze von Verbindungen der Formeln I, Ia und Ib mit Fumarsäure genannt, insbesondere Salze, die ein Mol Fumarsäure pro Mol der Verbindung der Formel I, Ia oder Ib enthalten und die somit Hydrogenfumarate oder Hemifumarate sind. Durch vorteilhafte Eigenschaften wie Kristallinität, Stabilität, eine besonders geringe Hygroskopizität, eine geringe Neigung zur Racemisierung und gute Löslichkeit zeichnet sich speziell beispielsweise das (S)-N-{2-[3-Oxo-2-(2,2,2-trifluorethyl)-6-trifluormethyl-2,3-dihydro-1H-isoindol-1-yl]-acetyl}-guänidin Hydrogenfumarat Hydrat der Formel XVI aus, das ebenfalls in allen seinen tautomeren Formen Gegenstand der vorliegenden Verbindung ist.

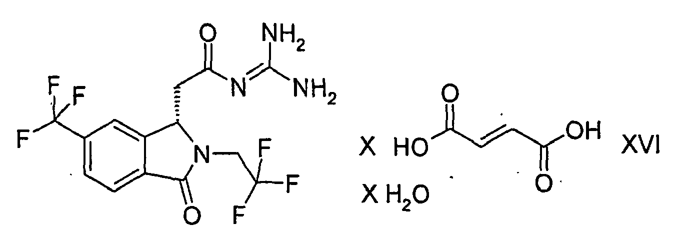

Enthalten die Verbindungen eine Säuregruppe, können sie Salze mit Basen bilden, beispielsweise Alkalimetallsalze, vorzugsweise Natriumsalze oder Kaliumsalze, oder Ammoniumsalze, zum Beispiel Salze mit Ammoniak oder organischen Aminen oder Aminosäuren. Verbindungen, die eine basische Gruppe und eine Säuregruppe enthalten, können auch als Zwitterion vorliegen.

Eine Ausführungsform der vorliegenden Erfindung betrifft Verbindungen, in denen R1 und R2 nicht beide Wasserstoff sind, insbesondere Verbindungen, in denen R1 Wasserstoff und R2 Fluor, Chlor oder Trifluormethyl, speziell Trifluormethyl, bedeutet. In Verbindungen, in denen R1 Wasserstoff bedeutet, befindet sich der Substituent R2 bevorzugt in der para-Position des Benzolringes bezüglich der C=O-Gruppe im Isoindolon-System.

Die Gruppe Alk steht bevorzugt für Alkyl mit 1, 2 oder 3 C-Atomen, insbesondere mit 1 oder 2 C-Atomen, speziell mit 1 C-Atom. R4 steht bevorzugt für Trifluormethyl oder Cycloalkyl mit 3, 5 oder 6 C-Atomen, insbesondere 3 C-Atomen, besonders bevorzugt für Trifluormethyl. Eine Ausführungsform der vorliegenden Erfindung betrifft Verbindungen, in denen R3 Trifluormethyl oder 2,2,2-Trifluorethyl, insbesondere 2,2,2-Trifluorethyl, bedeutet.

Eine spezielle Ausführungsform der vorliegenden Erfindung betrifft die Herstellung von N-{2-[3-Oxo-2-(2,2,2-trifluorethyl)-6-trifluormethyl-2,3-dihydro-1H-isoindol-1-yl]-acetyl}-guanidin und seinen enantiomeren Formen und Salzen davon.

X steht bevorzugt für Chlor oder Methoxy, insbesondere Chlor. R5 steht bevorzugt für Methoxy oder Ethoxy, insbesondere Ethoxy. R6 steht bevorzugt für Methoxy oder Ethoxy, insbesondere Ethoxy. R7 steht bevorzugt für Methoxy oder Ethoxy, insbesondere Ethoxy.

In einer Ausführungsform der vorliegenden Erfindung bedeutet R8 Phenyl, das unsubstituiert oder substituiert ist mit 1, 2 oder 3 Substituenten aus der Gruppe F, Cl, Alkyl mit 1, 2, 3 oder 4 C-Atomen oder Alkoxy mit 1, 2, 3 oder 4 C-Atomen, insbesondere unsubstituiertes Phenyl.

Die Verbindungen der Formeln I, Ia, Ib, XVa und XVb und ihre pharmazeutisch verträglichen Salze sind substituierte Acylguanidine und inhibieren den zellulären Natrium-Protonen-Antiporter (Na⁺/H⁺-Exchanger, NHE), insbesondere den Subtyp NHE-1.

Aufgrund der NHE-inhibitorischen Eigenschaften eignen sich die Verbindungen der Formeln I, Ia, Ib, XVa, XVb und XVI und/oder deren pharmazeutisch verträgliche Salze zur Prävention und Behandlung von Krankheiten, die durch eine Aktivierung bzw. durch einen aktivierten NHE verursacht werden, sowie von Krankheiten, die sekundär durch die NHE-bedingten Schädigungen verursacht werden.
Die Verbindungen der Formel I, Ia, Ib, XVa und XVb können auch zur Behandlung und Prävention von Krankheiten eingesetzt werden, wobei der NHE nur partiell gehemmt wird, beispielsweise durch Verwendung einer niedrigeren Dosierung.

Da NHE-Inhibitoren in überwiegender Weise über ihre Beeinflussung der zellulären pH-Regulation wirken, können diese generell in günstiger Weise mit anderen, den intrazellulären pH-Wert regulierenden Verbindungen kombiniert werden, wobei Inhibitoren der Enzymgruppe der Carboanhydrasen, Inhibitoren der Bicarbonationen transportierenden Systeme wie des Natrium-Bicarbonat-Cotransporters (NBC) oder des Natrium abhängigen Chlorid-Bicarbonat-Austauschers (NCBE), sowie NHE-Inhibitoren mit inhibitorischer Wirkung auf andere NHE-Subtypen, als Kombinationspartner infrage kommen, weil durch sie die pharmakologisch relevanten pH-regulierenden Effekte der hier beschriebenen NHE-Inhibitoren verstärkt oder moduliert werden können.

Die Verwendung der Verbindungen der Formeln I, Ia, Ib, XVa, XVb oder XVI betrifft die Prävention und die Behandlung akuter und chronischer Krankheiten in der Veterinär- und in der Humanmedizin. So eignen sich die erfindungsgemäßen Inhibitoren des NHE zur Behandlung von Krankheiten, die durch Ischämie und durch Reperfusion hervorgerufen werden.

Die hier beschriebenen Verbindungen sind infolge ihrer pharmakologischen Eigenschaften als antiarrhythmische Arzneimittel geeignet. Durch ihre cardioprotektive Komponente eignen sich NHE-Inhibitoren hervorragend zur Infarktprophylaxe und der Infarktbehandlung sowie zur Behandlung der angina pectoris, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäß verwendeten Verbindungen der Formeln I, Ia, Ib, XVa, XVb und XVI und/oder deren pharmazeutisch verträgliche Salze infolge Inhibition des zellulären Na⁺/H⁺-Austauschmechanismus als Arzneimittel zur Behandlung aller akuten oder chronischen durch Ischämie ausgelösten Schäden oder dadurch primär oder sekundär induzierten Krankheiten verwendet werden.

Dies betrifft auch ihre Verwendung als Arzneimittel für chirurgische Eingriffe. So können die Verbindungen bei Organ-Transplantationen verwendet werden, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können.

Die erfindungsgemäßen Verbindungen sind ebenfalls wertvolle, protektiv wirkende Arzneimittel bei der Durchführung angioplastischer operativer Eingriffe beispielsweise am Herzen wie auch an peripheren Organen und Gefäßen.

Weiterhin können die erfindungsgemäßen Verbindungen bei der Durchführung von Bypassoperationen verwendet werden, beispielsweise bei Bypassoperationen an Koronargefäßen und bei Coronary Artery Bypass Graft (CABG).

Entsprechend ihrer Wirkung gegen ischämisch induzierte Schäden können die erfindungsgemäßen Verbindungen der Formel I auch bei Wiederbelebung nach einem Herzstillstand eingesetzt werden.

Die erfindungsgemäßen Verbindungen sind von Interesse für Arzneimittel gegen lebensbedrohliche Arrhythmien. Kammerflimmern wird beendet und der physiologische Sinus-Rhythmus des Herzens wiederhergestellt.

Da NHE1-Inhibitoren menschliches Gewebe und Organe, insbesondere das Herz, nicht nur effektiv gegen Schäden schützen, die durch Ischämie und Reperfusion verursacht werden, sondern auch gegen die cytotoxische Wirkung von Arzneimitteln, wie sie insbesondere in der Krebstherapie und der Therapie von Autoimmunerkrankungen Anwendung finden, ist die kombinierte Verabreichung mit NHE-Inhibitoren geeignet, die cytotoxischen, insbesondere cardiotoxischen Nebenwirkungen der genannten Verbindungen zu inhibieren. Durch die Verminderung der cytotoxischen Effekte, insbesondere der Cardiotoxizität, infolge Ko-Medikation mit NHE1-Inhibitoren kann außerdem die Dosis der cytotoxischen Therapeutika erhöht und/oder die Medikation mit solchen Arzneimitteln verlängert werden. Der therapeutische Nutzen einer solchen cytotoxischen Therapie kann durch die Kombination mit NHE-Inhibitoren erheblich gesteigert werden.

Außerdem können NHE1-Inhibitoren bei einer herzschädigenden Überproduktion von Schilddrüsenhormonen, der Thyreotoxikose, oder bei der externen Zufuhr von Schilddrüsenhormonen Verwendung finden. Die Verbindungen der Formeln I, Ia, Ib, XVa, XVb und XVI und/oder deren pharmazeutisch verträgliche Salze eignen sich somit zur Verbesserung der Therapie mit cardiotoxischen Arzneimitteln.

Entsprechend ihrer protektiven Wirkung gegen ischämisch induzierte Schäden sind die erfindungsgemäßen Verbindungen auch als Arzneimittel zur Behandlung von Ischämien des Nervensystems, insbesondere des Zentralnervensystems, geeignet, wobei sie zum Beispiel zur Behandlung des Schlaganfalls oder des Hirnödems geeignet sind.

NHE-Inhibitoren eignen sich auch zur Therapie und Prophylaxe von Erkrankungen und Störungen, die durch Übererregbarkeit des Zentralnervensystems ausgelöst-werden, insbesondere zur Behandlung von Erkrankungen des epileptischen Formenkreises, zentral ausgelöster klonischer und tonischer Spasmen, psychische Depressionszustände, Angsterkrankungen und Psychosen. Dabei können die hier beschriebenen NHE-Inhibitoren allein angewandt werden oder in Kombination mit anderen antiepileptisch wirkenden Substanzen oder antipsychotischen Wirkstoffen, oder Carboanhydrasehemmern, beispielweise mit Acetazolamid, sowie mit weiteren Inhibitoren des NHE oder des natrium-abhängigen Chlorid-Bicarbonat-Austauschers (NCBE).

Darüber hinaus eignen sich NHE-Inhibitoren ebenfalls zur Behandlung von Formen des Schocks, wie beispielweise des allergischen, cardiogenen, hypovolämischen und des bakteriellen Schocks.

Die Verbindungen der Formeln I, Ia, Ib, XVa, XVb und XVI und/oder deren pharmazeutisch verträgliche Salze können ebenfalls zur Prävention und zur Behandlung thrombotischer Erkrankungen verwendet werden, da sie als NHE-Inhibitoren die Plättchenaggregation selbst inhibieren können. Darüberhinaus können sie die nach Ischämie und Reperfusion stattfindende überschießende Freisetzung von Entzündungs- und Gerinnungsmediatoren, insbesondere des von Willebrand Faktors und der thrombogenen Selectin-Proteine hemmen bzw. verhindern. Damit kann die pathogene Wirkung bedeutender thrombogener Faktoren vermindert und ausgeschaltet werden. Deshalb sind die NHE-Inhibitoren der vorliegenden Erfindung kombinierbar mit weiteren antikoagulativen und/oder thrombolytischen Wirkstoffen wie beispielsweise recombinantem oder natürlichen tissue plasminogen activator, Streptokinase, Urokinase, Acetylsalizylsäure, Thrombinantagonisten, Faktor Xa-Antagonisten, fibrinolytisch wirkenden Arzneistoffen, Thromboxan RezeptorAntagonisten, Phosphodiesterase-Inhibitoren, Factor-VIIa-Antagonisten, Clopidogrel, Ticolopidin,usw. Eine kombinierte Anwendung der vorliegenden NHE-Inhibitoren mit NCBE-Inhibitoren und/oder mit Inhibitoren der Carboanhydrase, wie beispielsweise mit Acetazolamid, ist besonders günstig.

Darüber hinaus zeichnen NHE-Inhibitoren durch starke inhibierende Wirkung auf die Proliferationen von Zellen, beispielsweise der Fibroblasten-Zellproliferation und der Proliferation der glatten Gefäßmuskelzellen, aus. Deshalb kommen die Verbindungen der Formeln I, Ia, Ib, XVa, XVb und XVI und/oder deren pharmazeutisch verträgliche Salze als wertvolle Therapeutika für Krankheiten in Frage, bei denen die Proliferation eine primäre oder sekundäre Ursache darstellt, und können deshalb als Antiatherosklerotika, Mittel gegen das chronische Nierenversagen, Krebserkrankungen, verwendet werden.

Es konnte gezeigt werden, dass durch NHE-Inhibitoren die Zellmigration inhibiert wird. Daher kommen die Verbindungen der Formeln I, Ia, Ib, XVa, XVb und XVI und/oder deren pharmazeutisch verträgliche Salze als wertvolle Therapeutika für Krankheiten in Frage, bei denen die Zellmigration eine primäre oder sekundäre Ursache darstellt, wie beispielsweise Krebserkrankungen mit ausgeprägter Neigung zur Metastasierung.

NHE-Inhibitoren zeichnen sich weiterhin durch eine Verzögerung oder Verhinderung von fibrotischen Erkrankungen aus. Sie eignen sich somit als ausgezeichnete Mittel zur Behandlung von Fibrosen des Herzens, sowie der Lungenfibrose, Leberfibrose, der Nierenfibrose und anderer fibrotischer Erkrankungen. Sie können somit zur Behandlung von Organhypertrophien und -hyperplasien, beispielsweise des Herzens und der Prostata verwendet werden. Sie sind deshalb zur Prävention und zur Behandlung der Herzinsuffizienz (congestive heart failure = CHF) wie auch bei der Behandlung und Prävention der Prostatahyperplasie bzw. Prostatahypertrophie geeignet.

Da der NHE bei essentiellen Hypertonikern signifikant erhöht ist, eignen sich die Verbindungen der Formeln I, Ia, Ib, XVa, XVb und XVI und/oder deren pharmazeutisch verträgliche Salze zur Prävention und zur Behandlung des Bluthochdrucks und zur Behandlung von Herz-Kreislauferkrankungen. Dabei können sie allein oder mit einem geeigneten Kombinations- und Formulierungspartner zur Bluthochdruckbehandlung und von Herz-Kreislauferkrankungen zur Anwendung kommen. So können beispielsweise ein oder mehrere thiazid-artig wirkende Diuretika, Loop-Diuretika, Aldosteron- und Pseudoaldosteron-Antagonisten, wie Hydrochlorothiazid, Indapamid, Polythiazid, Furosemid, Piretanid, Torasemid, Bumetanid, Amilorid, Triamteren, Spironolacton oder Epleron, kombiniert werden. Weiterhin können die NHE-Inhibitoren der vorliegenden Erfindung in Kombination mit Calcium-Antagonisten, wie Verapamil, Diltiazem, Amlodipin oder Nifedipin, sowie mit ACE-Hemmern, wie beispielsweise Ramipril, Enalapril, Lisinopril, Fosinopril oder Captopril verwendet werden. Weitere günstige Kombinationspartner sind auch β-Blocker wie Metoprolol, Albuterol usw., Antagonisten des Angiotensin-Rezeptors und seiner Rezeptor-Subtypen wie Losartan, Irbesartan, Valsartan, Omapatrilat, Gemopatrilat, Endothelin-Antagonisten, Renin-Inhibitoren, Adenosin-Rezeptoragonisten, Inhibitoren und Aktivatoren von Kaliumkanälen wie Glibenclamid, Glimepirid, Diazoxid, Cromakalim, Minoxidil und deren Derivate, Aktivatoren des mitochondrialen ATP-sensitiven Kaliumkanals (mitoK(ATP) Kanal), Inhibitoren des Kv1.5 usw.

Es zeigte sich, dass NHE1-Inhibitoren eine signifikante antiphlogistische Wirkung haben und somit als Antiinflammatorika-verwendet werden können. Dabei fällt die Inhibition der Freisetzung von Entzündungsmediatoren auf. Die Verbindungen können somit allein oder in Kombination mit einem Antiphlogistikum bei der Prävention oder Behandlung chronischer und akuter inflammatorischer Erkrankungen verwendet werden. Als Kombinationspartner werden vorteilhaft steroidale und nicht-steroidale Antiinflammatorika verwendet. Weiterhin könne die erfindungsgemäßen Verbindungen zur Prävention oder Behandlung von Erkrankungen, die durch Protozoen verursacht werden, eingesetzt werden, wie bei Malaria und der Hühnercoccidiose.

Es wurde außerdem gefunden, dass NHE-Inhibitoren eine günstige Beeinflussung der Serumlipoproteine zeigen. Es ist allgemein anerkannt, dass für die Entstehung arteriosklerotischer Gefäßveränderungen,insbesondere der koronaren Herzkrankheit, zu hohe Blutfettwerte, sogenannte Hyperlipoproteinämien, einen wesentlichen Risikofaktor darstellen. Für die Prophylaxe und die Regression von atherosklerotischen Veränderungen kommt daher der Senkung erhöhter SerumLipoproteine eine außerordentliche Bedeutung zu. Neben der Reduktion des Serum-Gesamtcholesterins kommt der Senkung des Anteils spezifischer atherogener Lipidfraktionen dieses Gesamtcholesterins, insbesondere der low density Lipoproteine (LDL) und der very low density Lipoproteine (VLDL) eine besondere Bedeutung zu, da diese Lipidfraktionen einen atherogenen Risikofaktor darstellen. Dagegen wird den high density Lipoproteinen eine Schutzfunktion gegen die koronare Herzkrankheit zugeschrieben. Dementsprechend sollen Hypolipidämika in der Lage sein, nicht nur das Gesamtcholesterin, sondern insbesondere die VLDL und LDL-Serumcholesterinfraktionen zu senken. Es wurde nun gefunden, dass NHE1-Inhibitoren bezüglich der Beeinflussung der Serumlipidspiegel wertvolle therapeutisch verwertbare Eigenschaften zeigen. So erniedrigen sie signifikant die erhöhte SerumKonzentrationen von LDL und VLDL, wie sie beispielweise durch erhöhte diätetische Einnahme einer cholesterin- und lipidreichen Kost oder bei pathologischen Stoffwechselveränderungen, beispielsweise genetisch bedingten Hyperlipidämien zu beobachten sind. Sie können daher zur Prophylaxe und zur Regression von atherosklerotischen Veränderungen herangezogen werden, indem sie einen kausalen Risikofaktor ausschalten. Hierzu zählen nicht nur die primären Hyperlipidämien, sondern auch gewisse sekundäre Hyperlipidämien, wie sie zum Beispiel beim Diabetes vorkommen. Darüber hinaus führen NHE-Inhibitoren zu einer deutlichen Reduktion der durch Stoffwechselanomalien induzierten Infarkte und insbesondere zu einer signifikanten Verminderung der induzierten Infarktgröße und dessen Schweregrades.

Die erfindungsgemäßen Verbindungen der Formeln I, Ia, Ib, XVa, XVb und XVI finden deshalb vorteilhaft Verwendung zur Herstellung eines Medikaments zur Behandlung von Hypercholesterinämie; zur Herstellung eines Medikaments zur Prävention der Atherogenese; zur Herstellung eines Medikaments zur Prävention und Behandlung der Atherosklerose, zur Herstellung eines Medikaments zur Prävention und Behandlung von Krankheiten, die durch erhöhte Cholesterinspiegel ausgelöst werden, zur Herstellung eines Medikaments zur Prävention und Behandlung von Krankheiten, die durch endotheliale Dysfunktion ausgelöst werden, zur Herstellung eines Medikaments zur Prävention und Behandlung von atherosklerose-induzierter Hypertonie, zur Herstellung eines Medikaments zur Prävention und Behandlung von atherosklerose-induzierter Thrombosen, zur Herstellung eines Medikaments zur Prävention und Behandlung von Hypercholesterinämie- und endothelialer Dysfunktion induzierter ischämischer Schäden und postischämischer Reperfusionsschäden, zur Herstellung eines Medikaments zur Prävention und Behandlung von Hypercholesterinämie- und endothelialer Dysfunktion induzierter kardialer Hypertrophien und Cardiomyopathien und der congestiven Herzinsuffizienz (CHF), zur Herstellung eines Medikaments zur Prävention und Behandlung von Hypercholesterinämie- und endothelialer Dysfunktion induzierter koronarer Gefäßspasmen und myocardialer Infarkte, zur Herstellung eines Medikaments zur Behandlung der genannten Leiden in Kombinationen mit blutdrucksenkenden Stoffen, bevorzugt mit Angiotensin Converting Enzyme (ACE)-Hemmern und Angiotensin-Rezeptorantagonisten. Eine Kombination eines NHE-Inhibitors mit einem blutfettspiegelsenkenden Wirkstoff, bevorzugt mit einem HMG-CoA-Reduktaseinhibitor (zum Beispiel Lovastatin oder Pravastatin), wobei letzterer eine hypolipidämische Wirkung herbeiführt und dadurch die hypolipidämischen Eigenschaften des NHE-Inhibitors steigert, erweist sich als günstige Kombination mit verstärkter Wirkung und vermindertem Wirkstoffeinsatz.
So führen NHE-Inhibitoren zu einen wirksamen Schutz gegen Endothelschädigungen unterschiedlicher Genese. Mit diesem Schutz der Gefäße gegen das Syndrom der endothelialen Dysfunktion sind Verbindungen der Formeln I, Ia, Ib, XVa, XVb und XVI und/oder deren pharmazeutisch verträgliche Salze wertvolle Arzneimittel zur Prävention und zur Behandlung koronarer Gefäßspasmen, peripherer Gefäßkrankheiten, insbesondere von claudicatio intermittens, der Atherogenese und der Atherosklerose, der linksventrikulären Hypertrophie und der dilatierten Kardiomyopathie, und thrombotischer Erkrankungen.

Außerdem wurde gefunden, dass NHE-Inhibitoren geeignet in der Behandlung des nicht-insulinabhängigen Diabetes (NIDDM) sind, wobei die Insulinresistenz zurückgedrängt wird. Dabei kann es zur Verstärkung von antidiabetischer Wirksamkeit und Wirkqualität der erfindungsgemäßen Verbindungen günstig sein, diese mit einem Biguanid wie Metformin, mit einem antidiabetischen Sulfonylharnstoff, wie Glyburid, Glimepirid, Tolbutamid usw., einem Glucosidase-Inhibitor, einem PPAR Agonisten, wie Rosiglitazone, Pioglitazone etc., mit einem Insulinpräparat unterschiedlicher Verabreichungsform, mit einem DB4 Inhibitor, mit einem Insulinsensitizer oder mit Meglitinide zu kombinieren.
Neben den akuten antidiabetischen Effekten wirken NHE-Inhibitoren der Entstehung diabetischer Spätkomplikationen entgegen und können deshalb als Arzneimittel zur Prävention und Behandlung diabetischer Spätschäden, wie der diabetischen Nephropathie, der diabetischen Neuropathie, der diabetischen Retinopathie, der diabetischen Cardiomyopathie und anderen, als Folge des Diabetes auftretenden Erkrankungen verwendet werden. Dabei können sie mit den soeben unter NIDDM-Behandlung beschriebenen antidiabetischen Arzneimitteln vorteilhaft kombiniert werden. Der Kombination mit einer günstigen Darreichungsform von Insulin dürfte dabei eine besondere Bedeutung zukommen.

NHE-Inhibitoren zeigen neben den protektiven Effekten gegen akute ischämische Ereignisse und die nachfolgenden ebenso akut belastenden Reperfusionsereignisse, auch noch direkte therapeutisch verwertbare Wirkungen gegen Erkrankungen und Störungen des gesamten Säugetierorganismus, die mit den Erscheinungen des chronisch ablaufenden Alterungsprozesses zusammenhängen und die unabhängig von akuten Mangeldurchblutungszuständen sind und bei normalen, nichtischämischen Bedingungen auftreten. Bei diesen pathologischen, über die lange Zeit des Alterns ausgelösten altersbedingten Erscheinungen wie Krankheit, Siechtum und Tod, die neuerdings mit NHE-Inhibitoren einer Behandlung zugänglich gemacht werden können, handelt es sich um Erkrankungen und Störungen, die maßgeblich durch altersbedingte Veränderungen von lebensnotwendigen Organen und deren Funktion verursacht werden und im alternden Organismus zunehmend an Bedeutung gewinnen.
Erkrankungen, die mit einer altersbedingten Funktionsstörung, mit altersbedingten Verschleißerscheinungen von Organen zusammenhängen, sind beispielsweise die ungenügende Ansprechbarkeit und Reagibilität der Blutgefäße gegenüber Kontraktions- und Reläxationsreaktionen. Diese altersbedingte Abnahme der Gefäßreagibilität auf konstriktorische und relaxierende Reize, die ein essentieller Prozess des Herz-Kreislaufsystems und somit des Lebens und der Gesundheit sind, kann signifikant durch NHE-Inhibitoren aufgehoben bzw. verringert werden: Eine wichtige Funktion und ein Maß für die Aufrechterhaltung der Gefäßreagibilität ist die Blockade bzw. Retardierung der altersbedingt fortschreitenden endothelialen Dysfunktion, die hochsignifikant durch NHE-Inhibitoren aufgehoben werden kann. NHE-Inhibitoren eignen sich somit hervorragend zur Behandlung und Prävention der altersbedingt fortschreitenden endothelialen Dysfunktion, insbesondere von claudicatio intermittens.

Beispiel einer weiteren den Altersprozess charakterisierenden Messgröße ist die Abnahme der Kontraktilität des Herzens und die Abnahme der Anpassung des Herzens an eine geforderte Pumpleistung des Herzens. Diese verminderte Herzleistungsfähigkeit als Folge des Alterungsprozesses ist in den meisten Fällen verbunden mit einer Dysfunktion des Herzens, die unter anderem durch eine Einlagerung von Bindegewebe ins Herzgewebe verursacht wird. Diese Bindegewebseinlagerung ist gekennzeichnet durch eine Zunahme des Herzgewichtes, durch eine Vergrößerung des Herzens und durch eine eingeschränkte Herzfunktion. Es war überraschend, dass eine derartige Alterung des Organs Herz nahezu komplett inhibiert werden konnte. NHE-Inhibitoren eignen sich somit hervorragend zur Behandlung und Prävention der Herzinsuffizienz, des congestive heart failure (CHF).

Durch Proliferationshemmung können nicht nur die bereits eingetretene Krebserkrankung geheilt werden, sondern dass auch die altersbedingte Entstehungshäufigkeit von Krebs durch NHE-Inhibitoren vermindert und hochsignifikant verzögert werden. Besonders bemerkenswert ist der Befund, dass altersbedingt auftretenden Erkrankungen aller Organe und nicht nur bestimmter Krebsformen unterbunden bzw. hochsignifikant verzögert auftreten. NHE-Inhibitoren eignen sich somit zur Behandlung und insbesondere der Prävention von altersbedingten Formen von Krebs.

Mit NHE-Inhibitoren wird eine zeitlich hochsignifikant verschobene Verzögerung des Eintretens altersbedingter Erkrankungen aller untersuchten Organe einschließlich Herz, Gefäße, Leber usw., sowie eine hochsignifikante Verzögerung von Alterskrebs festgestellt. Vielmehr kommt es auch überraschenderweise zu einer Lebensverlängerung in einem Ausmaß, das bislang durch keine andere Medikamentengruppe bzw. durch irgendwelche Naturstoffe erreicht werden konnte. Diese einzigartige Wirkung der NHE-Inhibitoren ermöglicht es auch, neben der alleinigen Wirkstoffanwendung an Mensch und Tier diese NHE-Inhibitoren mit anderen gerontologisch verwendeten Wirkprinzipien, Maßnahmen, Substanzen und Naturstoffen zu kombinieren, denen ein anderer Wirkmechanismus zugrunde liegt. Derartige in der gerontologischen Therapie verwendete Wirkstoffklassen sind: insbesondere Vitamine und antioxidativ wirksame Stoffe. Da eine Korrelation zwischen kalorischer Belastung bzw. Nahrungsaufnahme und Alterungsprozeß besteht, kann die Kombination mit diätetischen Maßnahmen zum Beispiel mit Appetitszüglern erfolgen. Ebenso kann eine Kombination mit blutdrucksenkenden Medikamenten, wie mit ACE-Hemmern, Angiotensinrezeptorantagonisten, Diuretika, Ca⁺²-Antagonisten etc. oder mit stoffwechselnormalisierenden Medikamenten, wie Cholesterinsenkern, gedacht werden.
NHE-Inhibitoren eignen sich somit hervorragend zur Prävention altersbedingter Gewebsveränderungen und zur Lebensverlängerung unter Erhalt einer hohen Lebensqualität.

Die erfindungsgemäßen Verbindungen sind wirkungsvolle Inhibitoren des zellulären Natrium-Protonen-Antiporters (Na/H-Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäß verwendeten Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes und diabetischer Spätkomplikationen, proliferativer Erkrankungen usw.

Beansprucht wird weiterhin ein Heilmittel für die humane, veterinäre oder phytoprotektive Anwendung, das, zusammen mit pharmazeutisch annehmbaren Träger- und Zusatzstoffen, eine wirksame Menge einer oder mehrerer Verbindungen der Formeln XVa, XVb und XVI und/oder deren pharmazeutisch verträglicher Salze enthält, allein oder in Kombination mit anderen pharmakologischen Wirkstoffen oder Arzneimitteln. Arzneimittel, die eine Verbindung der Formeln I, Ia, Ib, XVa, XVb und XVI und/oder deren pharmazeutisch verträgliche Salze enthalten, können dabei zum Beispiel oral, parenteral, intravenös, rektal, perkutan oder durch Inhalation appliziert werden, wobei die bevorzugte Applikation von dem jeweiligen Erscheinungsbild der Erkrankung abhängig ist. Die Verbindungen der Formeln I, Ia, Ib, XVa, XVb und XVI können dabei allein oder zusammen mit galenischen Hilfsstoffen zur Anwendung kommen, und zwar sowohl in der Veterinär- als auch in der Humanmedizin. Die Arzneimittel enthalten Wirkstoffe der Formeln I, Ia, Ib, XVa, XVb und XVI und/oder deren pharmazeutisch verträgliche Salze im allgemeinen in einer Menge von 0.01 mg bis 1 g pro Dosiseinheit.

Welche Hilfsstoffe für die gewünschte Arzneimittelformulierung geeignet sind, ist dem Fachmann auf Grund seines Fachwissens geläufig. Neben Lösungsmitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wässrige, alkoholische oder ölige Lösungen. Als inerte Träger können zum Beispiel Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösungsmittel kommen beispielsweise pflanzliche oder tierische Öle in Betracht, wie Sonnenblumenöl oder Lebertran.

Zur subkutanen, intramuskulären oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen zum Beispiel in Frage: Wasser, physiologische Kochsalzlösung oder Alkohole, zum Beispiel Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet zum Beispiel Lösungen, Suspensionen oder Emulsionen des Wirkstoffes der Formeln I, Ia, Ib, XVa, XVb und XVI und/oder deren pharmazeutisch verträgliche Salze in einem pharmazeutisch unbedenklichen Lösungsmittels, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formeln I, Ia, Ib, XVa, XVb und XVI und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.

Im Durchschnitt beträgt die tägliche Dosis einer Verbindung der Formeln I, Ia, Ib, XVa, XVb und XVI und/oder deren pharmazeutisch verträgliche Salze bei einem etwa 75 kg schweren Patienten mindestens 0,001 mg/kg, beispielsweise 0,01 mg/kg, bis höchstens 10 mg/kg, beispielsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, zum Beispiel bis zu 4 Einzeldosen pro Tag. Insbesondere bei i.v. Anwendung, etwa bei einem Infarktpatienten auf der Intensivstation können beispielsweise bis zu 700 mg pro Tag notwendig werden und können die erfindungsgemäßen Verbindungen durch Infusion verabreicht werden.

### Liste der Abkürzungen:

- DCC: Dicyclohexyl-carbodiimid
- DIP: Diisopropylether
- DC: Dünnschichtchromatographie
- DMF: N, N-Dimethylformamid
- EE: Ethylacetat
- eq.: Äquivalent
- Et₃N: Triethylamin
- Et₂O: Diethylether
- EtOH: Ethanol
- h: Stunde(n)
- HEP: n-Heptan
- HOAc: Essigsäure
- KOtBu: Kalium-2-methyl-propan-2-olat
- MeOH: Methanol
- min: Minute(n)
- mp: Schmelzpunkt
- MTB: tert.-Butyl-methylether
- NMP: 1-Methylpyrrolidin-2-on
- Pd(OAc)₂: Palladium(II)-acetat
- RT: Raumtemperatur
- rt: Retentionszeit
- tBu: tert-Butyl
- THF: Tetrahydrofuran
- TMEDA: N,N,N',N'-Tetramethyl-ethan-1,2-diamin

Die im Folgenden angegebenen Retentionszeiten (rt) beziehen sich auf HPLC-Messungen mit den folgenden Parametern:
Methode A:
   - Stationäre Phase:: Waters Symmetry C8 (5µ) 3.9x150mm
   - Mobile Phase:: isokratisch CH₃CN/0.1 % wässrige CF₃CO₂H 35:65; λ=220nm; 1 ml/min.
Methode B:
   - Stationäre Phase:: Waters Symmetry C8 (5µ) 3.9x150mm
   - Mobile Phase:: isokratisch CH₃CN/0.1% wässrige CF₃CO₂H 40:60; λ=230nm; 1ml/min.
Methode C:
   - Stationäre Phase:: Waters Symmetry C8 (5µ) 3.9x150mm
   - Mobile Phase:: isokratisch CH₃CN/0.1 % wässrige CF₃CO₂H 50:50; λ=220nm; 1 ml/min.

### Beispiel 1

### a) N-(2,2,2-Trifluor-ethyl)-4.-trifluormethyl-benzamid

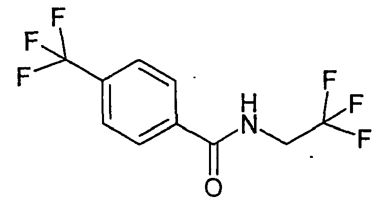

5,0 g (24 mmol) 4-Trifluormethyl-benzoylchlorid und 5,0 ml (36 mmol) Triethylamin wurden in 50 ml CH₂Cl₂ gelöst und bei RT langsam 2,4 g (24 mmol) 2,2,2-Trifluorethylamin zugetropft. 4 h wurde bei RT nachgerührt, anschließend die flüchtigen Bestandteile im Vakuum entfernt. Der Rückstand wurde mit 100 ml MTB aufgenommen und zunächst mit 30 ml einer gesättigten wässrigen Na₂CO₃-Lösung und dann mit 30 ml einer gesättigten wässrigen NaHSO₄-Lösung gewaschen. Über MgSO₄ wurde getrocknet und man erhielt 6,1 g (94%) eines farblosen Harzes, das beim Stehenlassen kristallisierte; mp: 117°C.

| | |
|---|---|
| R_{f} (DIP) = 0.50 | MS (EI) : 271 (M+1)⁺ |

### b) (R,S)-3-Hydroxy-2-(2,2,2-trifluor-ethyl)-5-trifluormethyt-2,3-dihydro-isoindol-1-on

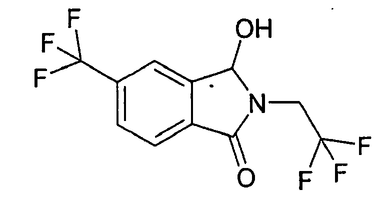

0,37 ml (2,4 mmol) TMEDA und 1,4 ml (2,3 mmol) einer 1,5 M Lösung von t-BuLi in n-Pentan wurden bei -75°C in 2 ml THF (wasserfrei) gelöst und bei -75°C eine Lösung von 0,30 g (1,1 mmol) N-(2,2,2-Trifluor-ethyl)-4-trifluormethyl-benzamid in 2 ml THF zugetropft. 3 h wurde bei -75°C gerührt, anschließend 0,43 ml (5,5 mmol) DMF zugetropft und während 30 Minuten auf RT erwärmt. Das Reaktionsgemisch wurde auf 100 ml einer gesättigten wässrigen NaHCO₃-Lösung gegossen und 3 mal mit je 30 ml EE extrahiert. Über MgSO₄ wurde getrocknet und das Solvens im Vakuum entfernt. Chromatographie an Kieselgel mit DIP lieferte 80 mg des (R,S)-3-Hydroxy-2-(2,2,2-trifluor-ethyl)-5-trifluormethyl-2,3-dihydro-isoindol-1-on neben 110 mg Gemisch mit Ausgangsmaterial. Dieses Gemisch wurde durch reversed phase HPLC (Bedingungen siehe unten) erneut getrennt und man erhielt weitere 40 mg (R,S)-3-Hydroxy-2-(2,2,2-trifluor-ethyl)-5-trifluormethyl-2,3-dihydro-isoindol-1-on; Gesamtausbeute 30%.

HPLC: Gradient, Laufzeit 20 min
Laufmittel: 0.1 % wässrige CF₃CO₂H, Acetonitril (Chromasolv); Fluss: 30ml/min
Säule: Waters Xterra™ MS C₁₈ 5µm, 30x100mm

Gradient:
- 0 - 2,5 min: 10% Acetonitril
- 3,0 min: 25% Acetonitril
- 14,0 min: 75% Acetonitril
- 15,0 min: 95% Acetonitril
- 17,5 min: 10% Acetonitril

| | |
|---|---|
| R_{f} (DIP) = 0.50 | MS (EI) : 299 (M+1)⁺ |

### c) (RS)-[3-Oxo-2-(2,2,2-trifluor-ethyl)-6-trifluormethyl-2,3-dihydro-1 H-isoindol-1-yl]-essigsäure-ethylester

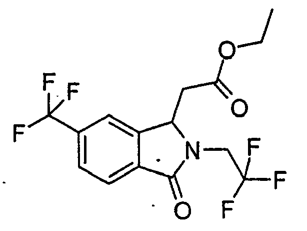

Unter Argon wurde (Diethoxy-phosphoryl)-essigsäureethylester (135 mg, 0,6 mmol) in wasserfreiem Dimethoxyethan (10 ml) gelöst. Zu dieser Lösung gab man bei RT 17,6 mg NaH (60% ig in Öl) und rührte 10 min, bei RT. Danach wurde eine Lösung von (RS)-3-Hydroxy-2-(2,2,2-trifluorethyl)-5-trifluormethyl-2,3-dihydro-isoindol-1-on 120 mg (0,04 mmol) in wasserfreiem Dimethoxyethan (5 ml) zugegeben und anschließend 2 h am Rückfluss gerührt. Die Reaktionslösung wurde abkühlen gelassen, die Reaktionslösung dann auf 50 ml 5 % ige Natriumhydrogencarbonatlösung gegossen, 2 mal mit je 20 ml Ethylacetat extrahiert, die organische Phase über MgSO4 getrocknet, im Vakuum eingedampft und den Rückstand durch Chromatographie an Kieselgel mit DIP als Eluens gereinigt. Man erhielt 90 mg (61%) (RS)-[3-Oxo-2-(2,2,2-trifluor-ethyl)-6-trifluormethyl-2,3-dihydro-1 H-isoindol-1-yl]-essigsäure-ethylester als farbloses Öl, das aus Heptan als beiger Feststoff kristallisierte.
Rf(DIP)= 0,31

Das NMR-Spektrum war identisch mit dem in Beispiel 4 hergestellten Material.

### d) (R,S)-N-{2-[3-Oxo-2-(2,2,2-trifluor-ethyl)-6-trifluormethyl-2,3-dihydro-H-isoindol-1-yl]-acetyl}-guanidin

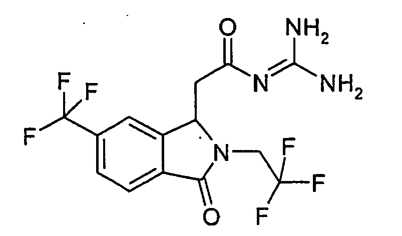

Der (RS)-[3-Oxo-2-(2,2,2-trifluor-ethyl)-6-trifluormethyl=2,3-dihydro-1 H-isoindol-1-yl]-essigsäure-ethylester kann Wie im Beispiel 2g) beschrieben mit Guanidin umgesetzt werden.

### Beispiel 2

### a) 2-Nitro-4-trifluormethyl-benzoesäure

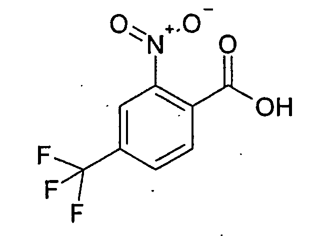

11,97 g 4-Trifluormethyl-benzoesäure (63 mmol) wurden langsam portionsweise zu 48 ml HNO₃ (100%) bei RT zugegeben. Die Mischung wurde anschließend 1 h lang unter Rückfluss erhitzt, dann auf RT abgekühlt und auf etwa 600 g Eis gegossen. 1 h lang wurde das Gemisch gerührt, dann der Niederschlag abfiltriert und mit 1 I Wasser gewaschen. Das Filtrat wurde mit 300 ml CH₂Cl₂ extrahiert, die organische Phase mit dem Niederschlag vereinigt und über Na₂SO₄ getrocknet. Das Solvens wurde im Vakuum entfernt und der Rückstand umkristallisiert, indem in 1 I DIP bei 68°C gelöst wurde, 2 I HEP bei dieser Temperatur zugegeben wurden und schließlich die Lösung langsam auf RT abgekühlt wurde. Das kristallisierte Produkt wurde mit 1 I HEP gewaschen, im Vakuum getrocknet und man erhielt 7,1 g (48%), mp 136°C-138°C.

### b) 2-Amino-4-trifluormethyl-benzoesäure

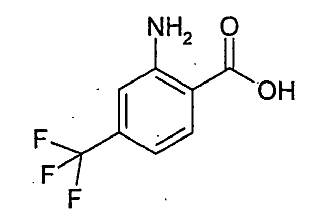

250 g 2-Nitro-4-trifluormethyl-benzoesäure (1.06 mol) wurden in 1 1 EtOH gelöst und 7,5 g Pd/C (5%) zugegeben. Die Mischung wurde unter 1-2.5 bar Wasserstoffdruck hydriert. Während der Wasserstoff-Aufnahme stieg die Temperatur zwischenzeitlich von 10°C auf 104°C. Nach 2 h war die Wasserstoff-Aufnahme beendet. Anschließend wurde der Katalysator abfiltriert, das Solvens im Vakuum entfernt und man erhielt 215 g (99%) eines blassgelben Feststoffs, mp 174-176 °C.

### c) 2-((E)-2-Ethoxycarbonyl-vinyl)-4-trifluormethyl-benzoesäure

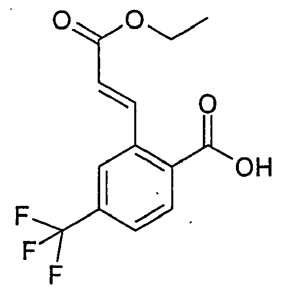

520 mg NaNO₂ (7.6 mmol) wurden in 2 ml Wasser gelöst und zu einer Lösung von 1,3 g 2-Amino-4-trifluormethyl-benzoesäure (6.5 mmol) in 2.6 ml einer 48% wässrigen HBF₄-Lösung und 30 ml Ethanol bei 0°C zugetropft. 10 Minuten wurde bei 0°C gerührt, dann wurde auf RT erwärmt. Weitere 0,3 ml einer 48% wässrigen HBF₄-Lösung wurden zugegeben, dann 30 ml Ethanol, 0,9 g Acrylsäure-ethylester (9,0 mmol) und 26.9 mg Pd(OAc)₂ (0.12 mmol). Anschließend wurde die Mischung 1 h bei 50-60°C gerührt. Dann wurde das Solvens im Vakuum entfernt, der Rückstand mit 25 ml EE aufgenommen und zunächst mit 25 ml einer 1 N wässrigen HCl-Lösung, dann mit 25 ml einer gesättigten wässrigen NaCl-Lösung gewaschen. Die organische Phase wurde über Na₂SO₄ getrocknet und das Solvens im Vakuum entfernt. Der Rückstand wurde in 25 ml Heptan suspendiert und das ausgefallene Produkt abfiltriert. Ausbeute 1,3 g (69%) eines blassbräunlichen Feststoffs. Eine analytische Probe wurde durch Kristallisation aus Heptan/Ethylacetat gereinigt.
Das NMR-Spektrum war identisch mit dem in Beispiel 3a hergestellten Material.

### d) (E)-3-[2-(2,2,2-Trifluor-ethylcarbamoyl)-5-trifluormethyl-phenyl]-acrylsäure-ethylester

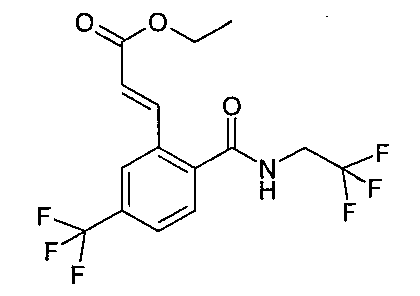

1,3 g 2-(2-Ethoxycarbonyl-vinyl)-4-trifluormethyl-benzoesäure (4,5 mmol) und 453 mg 2,2,2-Trifluor-ethylamin (4,5 mmol) wurden in 5 ml DMF gelöst und 0.93 g DCC zugegeben. Die Mischung wurde 4h lang bei RT gerührt. Das Harnstoff-Nebenprodukt wurde durch Filtration entfernt und dann das Solvens im Vakuum entfernt. Der Rückstand wurde aus DIP umkristallisiert und man erhielt 1,6 g (96%) weißer Kristalle. Das NMR-Spektrum war identisch mit dem in Beispiel 3b hergestellten Material.

### e) (RS)-[3-Oxo-2-(2,2,2-trifluor-ethyl)-6-trifluormethyl-2,3-dihydro-1 H-isoindol-1-yl]-essigsäure

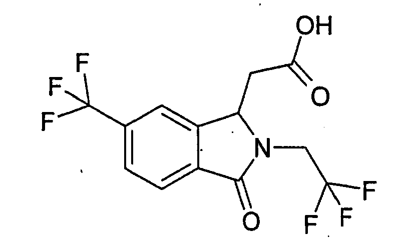

2,2 g (E)-3-[2-(2,2,2-Trifluor-ethylcarbamoyl)-5-trifluormethyl-phenyl]-acrylsäure-ethylester (5,9 mmol) wurden in 10 ml Methanol gelöst und 1,5 ml einer 5 M wässrigen NaOH-Lösung (7,5 mmol) zugegeben. Das Gemisch wurde 18h bei RT gerührt und dann mit wässriger HCl-Lösung auf pH=7 gestellt. Die Solventien wurden im Vakuum entfernt und der Rückstand in 10 ml Wasser suspendiert. Diese Suspension wurde mit einer 2N wässrigen HCl-Lösung auf pH=2 gestellt und 3 mal mit je 10 ml EE extrahiert. Über Na₂SO₄ wurde getrocknet und das Solvens im Vakuum entfernt. Der Rückstand wurde mit Diethylether/DIP kristallisiert, mp: 202-204 °C.
Ausbeute 1.8 g (89%).
¹H-NMR (400 MHz, CDCl₃): δ = 3,07 (dd, J₁=17 Hz, J₂=6 Hz, 1 H), 3.23 (dd, J₁=17 Hz, J₂= 5 Hz, 1 H), 4.27 (m, 1H), 4.58 (m, 1 H), -5.08 (t, J=5 Hz, 1 H), 7.91 (d, J=8 Hz, 1 H), 7.96 (d, J=8 Hz, 1 H), 8.12 (s, 1 H), 12.50 (bs, 1 H) ppm.
Verbrennungsanalyse: C₁₃H₉F₆NO₃ (341.2): ber. C 45.76 H 2.66 N4.10; gef. C 45.71 H 2.43 N 4.11.

### f) (RS)-[3-Oxo-2-(2,2,2-trifluor-ethyl)-6-trifluormethyl-2,3-dihydro-1H-isoindol-1-yl]-essigsäure-ethylester

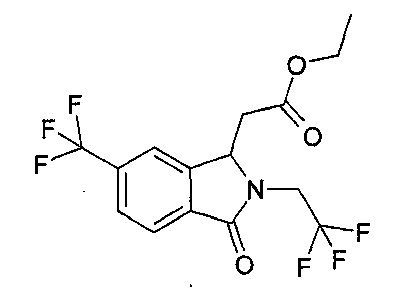

2,6 ml SOCl₂ (35 mmol) wurden in 20 ml Ethanol gelöst und 3,4 g (R,S)-[3-Oxo-2-(2,2,2-trifluor-ethyl)-6-trifluormethyl-2,3-dihydro-1H-isoindol-1-yl]-essigsäure (10 mmol) bei -10°C zugegeben. 18h wurde bei RT gerührt und anschließend die flüchtigen Bestandteile im Vakuum entfernt. Der Rückstand wurde an Kieselgel mit HEP/EE 3:1 chromatographiert. Ausbeute 3,0 g (81 %) eines farblosen Öls, das aus Heptan als beiger Feststoff kristallisierte.
Das NMR-Spektrum war identisch mit dem in Beispiel 4 hergestellten Material.

### g) (RS)-N-{2-[3,-Oxo-2-(2,2,2-trifluor-ethyl)-6-trifluormethyl-2,3-dihydro-H-isoindol-1-yl]-acetyl}-guanidin

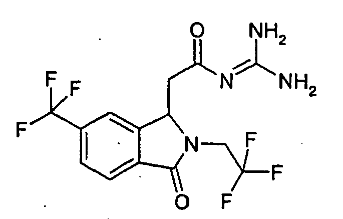

Guanidin-Hydrochlorid (11,5 g, 120 mmol) wurde in NMP (45 ml) gelöst und KOtBu (11,2 g, 100 mmol) unter Rühren zugegeben, 1.5 h bei RT rühren gelassen und das Gemisch filtriert. Das Filtrat wurde zu einer Lösung von (RS)-[3-Oxo-2-(2,2,2-trifluorethyl)-6-trifluormethyl-2,3-dihydro-1H-isoindol-1-yl]-essigsäure-ethylester (7,38 g,20 mmol) in NMP (12 ml) unter Rühren bei RT getropft und weitere 60 min bei RT rühren gelassen. Anschließend wurde Eiswasser (270 ml) zugegeben, mit 2N HCl auf pH 7 gestellt, Ethylacetat (60 ml)-zugegeben und anschließend durch Zugabe von NaHCO₃-Lösung der pH-Wert auf 8-8,5 eingestellt. Das Gemisch wurde 1 h bei RT kräftig gerührt und der gebildete Niederschlag abgesaugt und mit Wasser gewaschen. Man erhielt 7,06 g (83 %) (R,S)-N-{2-[3-Oxo-2-(2,2,2-trifluor-ethyl)-6-trifluormethyl-2,3-dihydro-H-isoindol-1-yl]-acetyl}-guanidin, Einschlussverbindung mit 0,5 Äquivalenten Ethylacetat, als hellgelbe Kristalle, mp.160-161°C bei langsamem Erwärmen, Entweichen von Ethylacetat ab ca. 90°C.
Rf (Ethylacetat/Methanol) = 0.45
¹H-NMR (400 MHz, CDCl₃): δ = 2.54 (dd, J₁=8 Hz, J₂=16 Hz, 1 H), 3.09 (dd, J₁=4 Hz, J₂=16 Hz, 1 H), 4.25 (m, 1 H), 4.64 (m, 1 H), 5.18 (m, 1 H), 6,65 (bs, 2 H), 7.75 (bs, 2 H), 7.88 (d, J=8 Hz, 1 H), 7.95 (d, J=8 Hz, 1 H), 8.02 (s, 1 H) ppm.
C₁₄H₁₂F₆N₄O₂ x ½ C₄H₈O₂ (426.33): ber. C 45.08, H 3.78, N 13,14; gef. C 45.07, H 3.79, N 13.01.

### Beispiel 3

### a) 2-((E)-2-Ethoxycarbonyl-vinyl)-4-trifluormethyl-benzoesäure (Variante des Beispiels 2c)

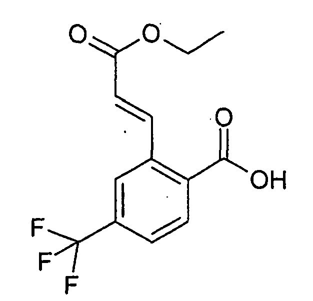

Zu 339 g 2-Amino-4-trifluormethyl-benzoesäure (1,65 mol) in 6.8 I EtOH (wasserfrei) wurden bei RT 658 ml einer 48-50% wässrigen HBF₄-Lösung zugegeben. Dabei stieg die Temperatur von 21 °C auf 26°C. Dann wurde die Mischung auf 0°C gekühlt und eine Lösung von 125 g NaNO₂ in 500 ml Wasser während 17 Minuten zwischen 0°C und 5°C zugetropft. Dabei wurde aus der zunächst blassgelben Lösung zunächst eine orange-rote Suspension und schließlich eine hellgelbe Suspension. Der Verlauf der Reaktion wurde mit HPLC verfolgt (Methode B; rt 2-Amino-4-trifluormethyl-benzoesäure = 6,4 min; Zwischenprodukt 2-Carboxy-5-trifluormethyl-benzoldiazonium-Salz = 1,1 min. Innerhalb von 30 Minuten war die Umsetzung zum 2-Carboxy-5-trifluormethyl-benzoldiazonium-Salz zu >99% beendet. Dann wurden zu der Mischung 231 g Acrylsäure-ethylester (2,31 mol), 11,1 g Pd(OAc)₂ (49 mmol) und 6,81 Ethanol (wasserfrei) zugegeben und das Reaktionsgemisch auf 49-51 °C erwärmt. Dabei wurde eine gleichmäßige, mit Temperaturerhöhung zunehmende Stickstoffentwicklung beobachtet. Die Umsetzung wurde mit HPLC verfolgt (Methode B; rt 2-((E)-2-Ethoxycarbonyl-vinyl)-4-trifluormethyl-benzoesäure = 16,4 min). Nach 45 min war die Umsetzungsrate über 99% . Die Mischung wurde dann auf RT abgekühlt und das Solvens im Vakuum entfernt. Der Rückstand wurde in 3 l EE aufgenommen und abfiltriert. Das Filtrat wurde dann zunächst 3 mal mit je 2.1 l einer wässrigen HCl-Lösung, danach mit 1 l einer gesättigten wässrigen NaCl-Lösung gewaschen. Über Na₂SO₄ wurde getrocknet, das Solvens im Vakuum entfernt und man erhielt 449 g eines hellbraunen Feststoffs. Unter Einbeziehung einer Verunreinigung (4-Trifluormethyl-benzoesäure; 6,3%) und von Lösungsmittelresten (EE; 4%) ergab sich eine Ausbeute von 83%. Eine analytische Probe wurde durch Kristallisation aus Heptan/Ethylacetat gereinigt. Mp: 132-133 °C
¹H-NMR (400 MHz, CDCl₃): δ = 1.36 (t, J=7 Hz, 3 H), 4.31 (q, J=7 Hz, 2 H), 6.41 (d, J=16 Hz, 1 H), 7.72 (d, J=8 Hz, 1H), 7.86 (s, 1 H), 8.21 (d, J=8 Hz, 1 H), 8.51 (d, J=16 Hz, 1 H), 8.5-9.5 (bs, 1 H) ppm.
Verbrennungsanalyse: C₁₃H₁₁F₃O₄ (288,23): ber. C 54.17, H 3.85; gef. C 54.24, H 3.74.

### b) (E)-3-[2-(2,2,2-Trifluorethylcarbamoyl)-5-trifluormethyl-phenyl]-acrylsäure-ethylester

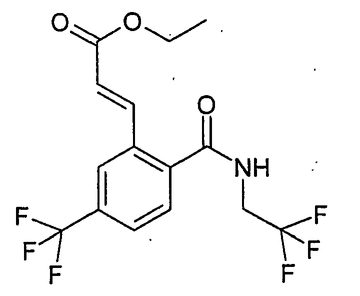

315 g Oxalylchlorid (2,48 mol) wurden bei einer Temperatur zwischen 15 und 18°C während 24 min zu einer Mischung aus 650 g 2-((E)-2-Ethoxycarbonyl-vinyl)-4-trifluormethyl-benzoesäure (2,25 mol), 33 ml DMF und 7,8 l CH₂Cl₂ addiert. Während der Zugabe wurde Gasentwicklung beobachtet. 1 h wurde bei RT gerührt, dann auf 5°C gekühlt und 285 g Et₃N ((2,81 mol) bei einer Temperatur zwischen 5°C und 10°C über einen Zeitraum von 27 min addiert. 10 min wurde bei 5°C nachgerührt, anschließend 279 g 2,2,2-Trifluor-ethylamin (2,81 mol) bei einer Temperatur zwischen 9°C und 20°C über einen Zeitraum von 27 min addiert. 10 min wurde bei RT gerührt, dabei fiel ein dicker Niederschlag aus und zur Verbesserung der Rührbarkeit des Gemisches wurde zusätzlich 1 l CH₂Cl₂ zugegeben. Die Reaktion wurde mit HPLC verfolgt (Methode C; rt: 2-((E)-2-Ethoxycarbonyl-vinyl)-4-trifluormethyl-benzoesäure = 5,9 min; rt: (E)-3-[2-(2,2,2-Trifluorethylcarbamoyl)-5-trifluormethyl-phenyl]-acrylsäure-ethylester = 13,2 min). Nach weiteren 50 min Rührens bei RT war die Umsetzung beendet. Dann wurden flüchtige Bestandteile des Reaktionsgemisches im Vakuum entfernt, der Rückstand mit 12 l EE aufgenommen und 3 mal mit je 2,5 l Wasser, dann 2 mal mit je 2,5 l einer gesättigten wässrigen NaHCO₃-Lösung und schließlich mit 1,5 l einer gesättigten wässrigen NaCl-Lösung gewaschen. Über MgSO₄ wurde getrocknet, das Solvens im Vakuum entfernt und man erhielt 802 g (E)-3-[2-(2,2,2-Trifluorethylcarbamoyl)-5-trifluormethyl-phenyl]-acrylsäure-ethylester als braunen Feststoff. Diese Rohsubstanz wurde mit dem Rohprodukt eines anderen Ansatzes (177 g) vereinigt und in 3 l EE bei 60-70°C gelöst und bei dieser Temperatur 14 l HEP in 1 l-Portionen addiert. Dann wurde die Mischung auf 80°C erwärmt und 1,5 h bei dieser Temperatur gerührt. Diese Mischung wurde dann zu 5,6 170°C warmem HEP addiert und die Mischung dann unter Rühren während eines Zeitraumes von 5h auf RT gekühlt. Dann wurde das Produkt abfiltriert, mit 3 I HEP gewaschen, an der Luft getrocknet und man erhielt 689 g (E)-3-[2-(2,2,2-Trifluorethylcarbamoyl)-5-trifluormethyl-phenyl]-acrylsäure-ethylester (67%) als hellbraunen Feststoff. Mp: 161,5-162 °C.
¹H-NMR (400 MHz, CDCl₃): δ = 1.33 (t, J=7 Hz, 3 H), 4.05 (m, 2 H), 4.26 (q, J=7 Hz, 2 H), 6.19 (bs, 1 H), 6.46 (d, J=16 Hz, 1 H), 7.63 (d, J=8 Hz, 1 H), 7.68 (d, J=8 Hz, 1 H), 7.87 (s, 1 H), 7.90 (d, J=16 Hz, 1 H) ppm.
Verbrennungsanalyse: C₁₅H₁₃F₆NO₃ (369,27): ber. C 48.79, H 3.55, N 3.79; gef. C 48.93, H 3.51, N 3.92.

### c) (R,S)-N-{2-[3-Oxo-2-(2,2,2-trifluor-ethyl)-6-trifluormethyl-2,3-dihydro-H-isoindol-1-yl]-acetyl}-guanidin

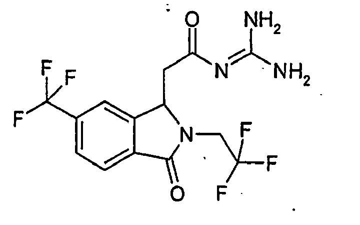

386 g (E)-3-[2-(2,2,2-Trifluorethylcarbamoyl)-5-trifluormethyl-phenyl]-acrylsäure-ethylester (1,05 mol) wurden in 600 ml DMF suspendiert und bei einer Temperatur zwischen 5°C und 15°C portionsweise langsam 4,7 g KOtBu (42 mmol) addiert. Die Cyclisierung zum Isoindolon wurde mit DC verfolgt (HEP/EE = 2:1; Acrylsäure-ethylester: R_{f} = 0.32; Isoindolon: R_{f} = 0,41). Nach einer Stunde war die Umsetzung abgeschlossen. In der Zwischenzeit wurden 587 g KOtBu in 2.2 l DMF suspendiert und 600 g Guanidinium-Chlorid bei einer Temperatur zwischen 20°C und 25°C zugegeben.

Die Mischung wurde 1 h bei 25°C gerührt und dann das KCl abfiltriert. Das Filtrat mit dem freigesetzten Guanidin wurde dann zu dem das Isoindolon enthaltende Reaktionsgemisch addiert und 2 h bei RT gerührt. Die Umsetzung zum Acylguanidin wurde über HPLC verfolgt (Methode B; Wellenlänge 230 nm und 254 nm; Isolindolon: rt = 15.1 min; Acylguanidin: rt = 2,9 min). Anschließend wurde das Reaktionsgemisch auf 14I Eiswasser gegossen, mit wässriger HCl-Lösung auf pH = 8,5-9,0 gestellt und 4 mal mit je 3 l EE extrahiert. Dann wurde 3 mal mit je 3 l einer gesättigten wässrigen NaCl-Lösung gewaschen, über Na₂SO₄ getrocknet und das Solvens im Vakuum entfernt. Man erhielt 329 g (82%) eines braunen Feststoffs. Das Produkt wurde mit 3 anderen Ansätzen desselben Herstellungsverfahrens vereinigt; Menge insgesamt 842 g. Diese 842 g (2,2 mol) wurden in 2 l EE und 5 l Et₂O 2 h bei 30°C digeriert. Dann wurde der Feststoff abfiltriert, 2 mal mit je 2 l Et₂O gewaschen und im Vakuum getrocknet. Man erhielt 693 g (82% Rückgewinnung) eines fast weißen Feststoffs. Die Verbindung kristallisierte aus 2-Propanol als Einschlussverbindung mit 0,5 Äquivalenten 2-Propanol aus.
Das NMR-Spektrum war identisch mit dem in Beispiel 5b hergestellten S-Enantiomer.

### Beispiel 4

### (RS)-(2-(2,2,2-Trifluorethyl)-3-oxo-6-trifluormethyl-2,3-dihydro-1H-isoindol-1-yl)-essigsäureethylester durch Eintopfreaktion ausgehend von 2-((E)-2-Ethoxycarbonylvinyl)-4-trifluormethyl-benzoesäure

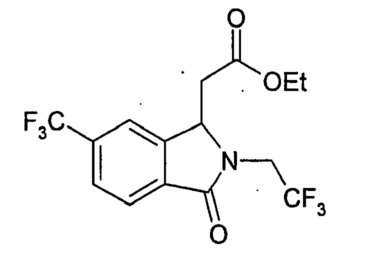

Zu einer Suspension von 2-((E)-2-Ethoxycarbonyl-vinyl)-4-trifluormethyl-benzoesäure (2,9 g, 10,1 mmol) in Toluol (30 ml) wurde bei Raumtemperatur SOCl₂ (1,98 g, 27,2 mmol) zugegeben. Es wurde 5 Min. bei Raumtemperatur gerührt und dann innerhalb von 30 Min. auf 105 °C (Badtemperatur) erwärmt. Bei etwa 70 °C begann die Gasentwicklung. Es wurde 3 h bei 105 °C gerührt, dann abgekühlt auf Raumtemperatur und über eine Kieselgurschicht (2,5 x 0,5 cm) abgesaugt, mit Toluol nachgewaschen und das Filtrat im Vakuum eingedampft. Man erhielt das Säurechlorid in Form eines rotbraunen Öls (3,34 g). 2,2,2-Trifluorethylamin (1,2 g, 12,1 mmol) und Triethylamin (2,58 g, 25,3 mmol) wurden in Dichlormethan (15 ml) bei 5 °C gelöst und unter Eiskühlung das Säurechlorid, gelöst in Dichlormethan (20 ml) so schnell zugetropft, dass die Temperatur zwischen 5 °C und 10 °C gehalten wurde. Dann wurde das Eisbad entfernt und der Überschuss an Trifluorethylamin und ein Teil des Dichlormethans im leichten Vakuum abdestilliert. Anschließend wurde das Gemisch 10 h am Rückfluss zum Sieden erhitzt. Nach dem Abkühlen wurde das Gemisch mit Dichlormethan (50 ml) verdünnt und zweimal mit wässriger 2N HCl-Lösung (je 50 ml) ausgeschüttelt, die vereinigten organischen Phasen mit Wasser (100 ml) gewaschen, über Na₂SO₄ getrocknet und im Vakuum eingedampft. Man erhielt (RS)-(2-(2,2,2-Trifluorethyl)-3-oxo-6-trifluormethyl-2,3-dihydro-1H-isoindol-1-yl)-essigsäureethylester (3,51 g, 94 %) als dunkelbraunes Öl, das durch Kristallisation aus n-Heptan gereinigt wurde. Mp: 54,5-55,5 °C.
¹H-NMR (400 MHz, CDCl₃): δ = 1.15 (t, J=7 Hz, 3 H), 2.85 (dd, J₁=6 Hz, J₂=16 Hz, 1 H), 3.01 (dd, J₁=5 Hz, J₂=16 Hz), 1 H), 3.83 (m, 1 H), 4.12 (q, J=7 Hz, 2 H), 4.73 (m, 1 H), 5.17 (t, J=6 Hz, 1 H), 7.80 (m, 2 H), 8.01 (d, J=8 Hz, 1 H) ppm.
Verbrennungsanalyse: C₁₅H₁₃F₆NO₃ (369,27): ber. C 48.79, H 3.55, N 3.79; gef. C 48.54, H 3.49, N 3.79.

### Beispiel 5

### a) (S)-N-{2-[3-Oxo-2-(2,2,2-trifluorethyl)-6-trifluormethyl-2,3-dihydro-1H-isoindol-1-yl]-acetyl}-guanidin, O,O'-Dibenzoyl-L-weinsäuresalz

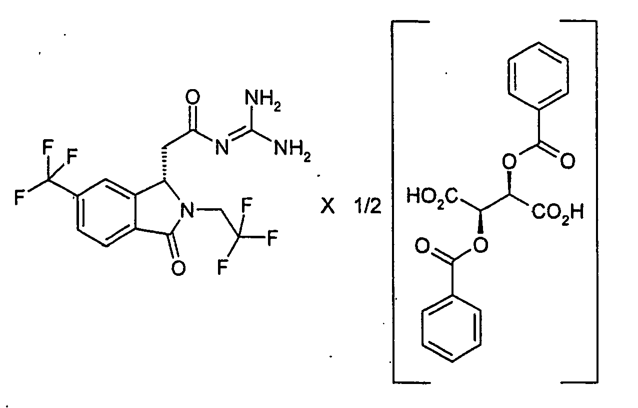

(RS)-N-{2-[3-Oxo-2-(2,2,2-trifluorethyl)-6-trifluormethyl-2,3-dihydro-1H-isoindol-1-yl]-acetyl}-guanidin (Einschlußverbindung mit Ethylacetat, Gehalt 87,06 % laut NMR, 44 g, 100 mmol) und O,O'-Dibenzoyl-L-weinsäure (11,2 g, 31 mmol) wurden als Feststoffe vorgelegt und unter Rühren 2-Propanol (500 ml) zugetropft. Dabei lösten sich die Feststoffe zunächst vollständig auf, dann fiel ein weißer Niederschlag aus. Nach 30 min wurde das Gemisch auf 70 °C erwärmt. Dabei entstand wieder eine fast klare Lösung. Diese wurde innerhalb von 4 h auf Raumtemperatur abkühlen gelassen und anschließend bei dieser Temperatur über Nacht gerührt. Danach wurde noch 4 h bei 10 °C gerührt und anschließend abgesaugt. Der Rückstand wurde zweimal mit 2-Propanol (je 100 ml) gewaschen und an der Luft getrocknet. Man erhielt 28,05 g (S)-N-{2-[3-Oxo-2-(2,2,2-trifluorethyl)-6-trifluormethyl-2,3-dihydro-1H-isoindol-1-yl]-acetyl}-guanidin, O,O'-Dibenzoyl-L-weinsäuresalz (74 % Ausbeute bezogen auf das (S)-Enantiomer), Enantiomerenreinheit 82 % ee laut HPLC (Chiracel OD/21, 250 x 4,6 mm, n-Heptan/Ethanol/Methanol 50:5:2, 1 ml/min, 30 °C) als farblose Kristalle. 20 g (14,6 mmol) dieser Kristalle wurden vorgelegt und 2-Propanol (400 ml) zugetropft. Das Gemisch wurde unter Rühren auf 80 °C erwärmt und dann langsam auf Raumtemperatur abkühlen gelassen. Es wurde noch 2 h bei dieser Temperatur gerührt und dann abgesaugt, der Rückstand zweimal mit 2-Propanol (je 50 ml) gewaschen und an der Luft getrocknet. Man erhielt 16,3 g (100 % Ausbeute bezogen auf das (S)-Enantiomer) (S)-N-{2-[3-Oxo-2-(2,2,2-trifluorethyl)-6-trifluormethyl-2,3-dihydro-1H-isoindol-1-yl]-acetyl}-guanidin, O,O'-Dibenzoyl-L-weinsäuresalz als farblose Kristalle, mp: 192-193 °C, Enantiomerenreinheit >97 % ee laut HPLC (Bedingungen wie oben).
Verbrennungsanalyse: C₁₄H₁₂F₆N₄O₂ x ½ C₁₈H₁₄O₈ (561.43): ber. C 49.21, H 3.41, N 9.98; gef. C 49.17, H 3.30, N 9.97.

### b) (S)-N-{2-[3-Oxo-2-(2,2,2-trifluorethyl)-6-trifluormethyl-2,3-dihydro-1 H-isoindol-1-yl]-acetyl}-guanidin

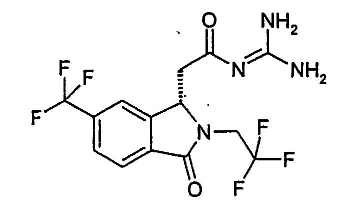

(S)-N-{2-[3-Oxo-2-(2,2,2-trifluorethyl)-6-trifluormethyl-2,3-dihydro-1 H-isoindol-1-yl]-acetyl}-guanidin, O,O'-Dibenzoyl-L-weinsäuresalz (113 mg, 0,20 mmol) wurde in einem Gemisch von Wasser (1 ml) und Ethylacetat (5 ml) gelöst und eine Lösung von NaHCO₃ (50 mg) in Wasser (7,5 ml) zugegeben. Das Gemisch wurde 16 h bei Raumtemperatur rühren gelassen und dann dreimal mit Ethylacetat (je 5 ml) extrahiert. Die vereinigten organischen Phasen wurden einmal mit einer Lösung von NaHCO₃ (50 mg) in Wasser (20 ml) und danach mit reinem Wasser (20 ml) geschüttelt, mit Na₂SO₄ getrocknet und im Vakuum eingedampft. Man erhielt 75 mg (97 %) (S)-N-{2-[3-Oxo-2-(2,2,2-trifluorethyl)-6-trifluormethyl-2,3-dihydro-1H-isoindol-1-yl]-acetyl}-guanidin. Das Produkt kristallisierte aus 2-Propanol als Einschlussverbindung mit 0,5 Äquivalenten 2-Propanol, mp: 80-82 °C. Die Substanz kannn auch aus Ethylacetat umkristallisiert werden und kristallisiert mit 0,5 Äquivalenten Ethylacetat, mp.: 121,5-122 °C. Enantiomerenreinheit > 97 % laut HPLC (Chiracel OD/21, 250 x 4.6 mm, n-Heptan/2-Propanol 4:1, 1 ml/min, 30 °C).
¹H-NMR (400 MHz, CDCl₃): δ = 2.54 (dd, J₁=8 Hz, J₂=16 Hz, 1 H), 3.09 (dd, J₁=4 Hz, J₂=16 Hz, 1 H), 4.25 (m, 1 H), 4.64 (m, 1 H), 5.18 (m, 1 H), 6,65 (bs, 2 H), 7.75 (bs, 2 H), 7.88 (d, J=8 Hz, 1 H), 7.95 (d, J=8 Hz, 1 H), 8.02 (s, 1 H) ppm.
Verbrennungsanalyse (Einschlussverbindung mit 0,5 Äquivalenten 2-Propanol): C₁₄H₁₂F₆N₄O₂ x ½ C₃H₈O (412.32): ber. C 45.15, H 3.91, N 13.59; gef. C 45.23, H 4.27, N 13.10.

### Beispiel 6

### (RS)-N-{2-[3-Oxo-2-(2,2,2-trifluorethyl)-6-trifluormethyl-2,3-dihydro-1 H-isoindol-1-yl]-acetyl}-guanidin durch Racemisierung von (R)-N-{2-[3-Oxo-2-(2,2,2-trifluorethyl)-6-trifluormethyl-2,3-dihydro-1 H-isoindol-1-yl]-acetyl}-guanidin

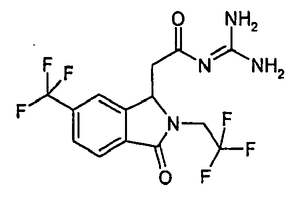

(R)-N-{2-[3-Oxo-2-(2,2,2-trifluorethyl)-6-trifluormethyl-2,3-dihydro-1H-isoindol-1-yl]-acetyl}-guanidin (Einschlussverbindung mit 0,5 Äquivalenten 2-Propanol (M=412.3), 43 g, 104 mmol; erhalten durch Einengen der Mutterlauge, die bei der Fällung des (S)-N-{2-[3-Oxo-2-(2,2,2-trifluorethyl)-6-trifluormethyl-2,3-dihydro-1 H-isoindol-1-yl]-acetyl}-guanidin, O,O'-Dibenzoyl-L-weinsäuresalz im -Beispiel 5a) anfiel, und Behandlung mit NaHCO₃ wie im Beispiel 5b) beschrieben) wurde in 2-Propanol (1,8 I) gelöst und bei Raumtemperatur unter Rühren eine Lösung von KOH (85proz., 660 mg, 10 mmol) in 2-Propanol (400 ml) zugegeben. Das Gemisch wurde bei Raumtemperatur 24 h gerührt und dann mit Eisessig (720 mg, 1,5 ml) angesäuert, im Vakuum bei max. 40 °C Badtemperatur eingedampft und der Rückstand zwischen Wasser (500 ml) und Ethylacetat (400 ml) verteilt. Die wässrige Phase wurde zweimal mit Ethylacetat (je 300 ml) ausgeschüttelt. Die vereinigten organischen Phasen wurden mit einer Lösung von NaHCO₃ (10 g) in Wasser (500 ml) und danach nochmals mit reinem Wasser geschüttelt. Die organische Phase wurde mit Na₂SO₄ getrocknet und im Vakuum bei max. 40 °C Badtemperatur eingedampft. Man erhielt 39,8 g (RS)-N-{2-[3-Oxo-2-(2,2,2-trifluorethyl)-6-trifluormethyl-2,3-dihydro-1H-isoindol-1-yl]-acetyl}-guanidin, Gehalt 87 % laut NMR, Einschlussverbindung mit 0,5 Äquivalenten Ethylacetat, als blassgelbe Kristalle, Ausbeute 87 %. Mp: 164-166 °C bei langsamem Erwärmen, Entweichen von Ethylacetat ab ca. 100 °C.
Enantiomerenverhältnis 49:51 laut HPLC (Chiracel OD/21, 250 x 4.6 mm, n-Heptan/2-Propanol 4:1, 1mL/min, 30 °C).

### Beispiel 7

### (S)-N-{2-[3-Oxo-2-(2,2,2-trifluorethyl)-6-trifluormethyl-2,3-dihydro-1 H-isoindol-1-yl]-acetyl}-guanidin Hydrogenfumarat Hydrat

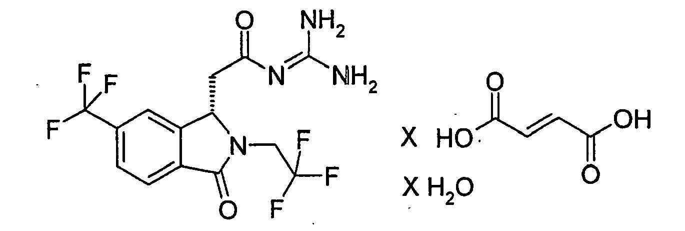

(S)-N-{2-[3-Oxo-2-(2,2,2-trifluorethyl)-6-trifluormethyl-2,3-dihydro-1 H-isoindol-1-yl]-acetyl}-guanidin [Einschlussverbindung mit 0,5 Äquivalenten 2-Propanol, (M=412.3), 110g, 266 mmol] wurde in Dimethoxyethan (2 l) gelöst und mit Fumarsäurelösung (0,5 M in Dimethoxyethan/Wasser 9:1, 512 ml) versetzt und die gebildete klare Lösung im Vakuum eingedampft. Der Rückstand wurde mit Dichlormethan (2 l) aufgenommen und das Gemisch erneut im Vakuum eingedampft. Der Rückstand wurde in Wasser (1.5 I) suspendiert, bei Raumtemperatur abgesaugt und über Nacht bei Raumtemperatur an der Luft getrocknet. Man erhielt 125.9 g (95 %) (S)-N-{2-[3-Oxo-2-(2,2,2-trifluorethyl)-6-trifluormethyl-2,3-dihydro-1H-isoindol-1-yl]-acetyl}-guanidin Hydrogenfumarat Hydrat als farblose Kristalle, Mp. 210 °C.

### Bestimmung der NHE-Hemmung

Die Hemmkonzentration IC₅₀ für die NHE-1 Hemmung wurde wie folgt bestimmt:
IC₅₀ für die NHE-1 Hemmung wurden bestimmt in einem FLIPR-Assay mittels Messung der pHᵢ-Erholung in transfizierten Zelllinien, die den humanen NHE-1 exprimieren.

Der Assay wurde im FLIPR (Fluorescent imaging plate reader) mit schwarzwandigen 96-Well-Mikrotiterplatten mit klarem Boden durchgeführt. Die transfizierten Zelllinien, welche die verschiedenen NHE-Subtypen exprimieren (die parentale Zelllinie LAP-1 weist als Folge von Mutagenese und anschließender Selektion keine endogene NHE-Aktivität auf), wurden am Vortag mit einer Dichte von -25.000 Zellen / Weil ausgesät. Das Wachstumsmedium der transfizierten Zellen (Iscove +10 % fötales Kälberserum) enthielt zusätzlich G418 als Selektionsantibiotikum, um die Anwesenheit der transfizierten Sequenzen sicherzustellen.

Der eigentliche Assay begann mit der Entfernung des Wachstumsmediums und Zugabe von 100 µl /Well Beladungspuffer (5 µM BCECF-AM [2',7'-Bis-(Carboxyethyl)-5-(und-6)-Carboxyfluorescein, Acetoxymethyl ester] in 20 mM NH₄Cl, 115 mM Cholinchlorid, 1 mM MgCl₂, 1 mM CaCl₂, 5 mM KCI, 20 mM HEPES, 5 mM Glucose; pH 7,4 [mit KOH eingestellt]). Die Zellen wurden dann 20 Minuten bei 37°C inkubiert. Diese Inkubation führte zur Beladung der Zellen mit dem fluoreszierenden Farbstoff, dessen Fluoreszenzintensität vom pHi abhängt, und mit NH₄Cl, was zu einer leichten Alkalinisierung der Zellen führte.
Die nicht fluoreszierende Farbstoff-Vorstufe BCECF-AM ist als Ester membranpermeabel. Intrazellulär wird durch Esterasen der eigentliche Farbstoff BCECF freigesetzt, der nicht membranpermeabel ist.

Nach dieser 20-minütigen Inkubation wurde der Beladungspuffer, der NH₄Cl und freies BCECF-AM enthielt, durch dreimaliges Waschen im Zellwasher (Tecan Columbus) mit jeweils 400 µl Waschpuffer (133,8 mM Cholinchlorid, 4,7 mM KCI, 1,25 mM MgCl₂, 1,25 mM CaCl₂,0,97 mM K₂HPO₄, 0,23 mM KH₂PO₄, 5 mM HEPES, 5 mM Glucose; pH 7,4 [mit KOH eingestellt]) entfernt. Das in den Wells verbleibende Restvolumen betrug 90 µl (50 -125 µl möglich). Dieser Waschschritt entfernte das freie BCECF-AM und führte als Folge der Entfernung der externen NH₄⁺-Ionen zu einer intrazellulären Ansäuerung (∼ pHᵢ 6.3 - 6.4).
Da das Gleichgewicht von intrazellulärem NH₄⁺ mit NH₃ und H⁺ durch das Entfernen des extrazellulären NH₄⁺ und durch die nachfolgende, augenblicklich ablaufende Passage des NH₃ durch die Zellmembran gestört wurde, führte der Waschprozess dazu, dass intrazellulär H⁺ zurückblieb, was die Ursache für die intrazelluläre Ansäuerung war. Diese kann letztlich zum Zelltod führen, wenn sie lang genug anhält. An dieser Stelle war es wichtig, dass der Waschpuffer natriumfrei (<1 mM) war, da extrazelluläre Natrium-Ionen zu einer augenblicklichen Erholung des pHⱼ durch die Aktivität der klonierten NHE-Isoformen führen würde.
Es war ebenfalls wichtig, dass alle verwendeten Puffer (Beladungspuffer, Waschpuffer, Recoverypuffer) keine HCO₃⁻-Ionen enthielten, da die Anwesenheit von Bicarbonat zur Aktivierung störender bicarbonatabhängiger pHi-Regulationssysteme führen würde, die in der parentalen LAP-1 Zelllinie enthalten sind.

Die Mikrotiterplatten mit den angesäuerten Zellen wurden dann (bis zu 20 Minuten nach der Ansäuerung) zum FLIPR transferiert. Im FLIPR wurde der intrazelluläre Fluoreszenzfarbstoff durch Licht mit einer Wellenlänge von 488 nm, das von einem Argon-Laser erzeugt wurde, angeregt, und die Messparameter (Laserleistung, Belichtungszeit und Blende der im FLIPR eingebauten CCD-Kamera) wurden so gewählt, dass das durchschnittliche Fluoreszenzsignal pro Well zwischen 30000 und 35000 relativen Fluoreszenzeinheiten lag.

Die eigentliche Messung im FLIPR begann damit, dass Software-gesteuert alle zwei Sekunden eine Aufnahme mit der CCD-Kamera gemacht wurde. Nach zehn Sekunden wurde die Erholung des intrazellulären pH's durch Zugabe von 90 µl Recoverypuffer (133,8 mM NaCl, 4,7 mM KCl, 1,25 mM MgCl₂, 1,25 mM CaCl₂, 0,97 mM K₂HPO₄, 0,23 mM KH₂PO₄, 10 mM HEPES, 5 mM Glucose; pH 7.4 [mit NaOH eingestellt]) mittels des im FLIPR eingebauten 96-Well-Pipettierers eingeleitet.
Als Positivkontrollen (100 % NHE-Aktivität) dienten Wells, denen reiner Recoverypuffer zugegeben wurde, Negativkontrollen (0 % NHE-Aktivität) erhielten Waschpuffer. In allen anderen Wells wurde Recoverypuffer mit der zweifach konzentrierten Testsubstanz hinzugegeben. Die Messung im FLIPR endete nach 60 Messpunkten (zwei Minuten).

Die Rohdaten wurden in das Programm ActivityBase exportiert. Mit diesem Programm wurden zunächst die NHE-Aktivitäten für jede getestete Substanzkonzentration und daraus die IC₅₀-Werte für die Substanzen berechnet. Da der Verlauf der pHᵢ-Erholung nicht während des ganzen Experiments linear war, sondern am Ende aufgrund abnehmender NHE-Aktivität bei höheren pHᵢ-Werten abfiel, war es wichtig, für die Auswertung der Messung den Teil auszuwählen, in dem die Fluoreszenzzunahme der Positivkontrollen linear war.

| Substanz | NHE1-Hemmung IC₅₀ [nM] |
|---|---|
| (S)-N-{2-[3-Oxo-2-(2,2,2-trifluorethyl)-6-trifluormethyl-2,3-dihydro-1H-isoindol-1-yl]-acetyl}-guanidin | 0,3 |

## Patentansprüche

1. Verfahren zur Herstellung von Verbindungen der Formel I 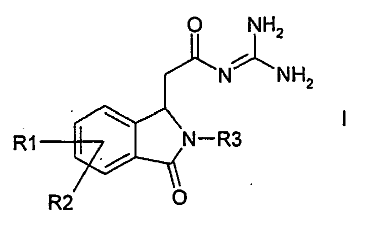 wobei bedeuten
R1 und R2 unabhängig voneinander Wasserstoff, F, Cl, Tritluormethoxy, 2,2,2-Trifluorethoxy, Trifluormethyl, 2,2,2-Trifluorethyl oder Alkyl mit 1, 2, 3 oder 4 C-Atomen;
R3 Alk-R4 oder Trifluormethyl;
Alk Alkyl mit 1, 2, 3 oder 4 C-Atomen;
R4 Wasserstoff, Trifluormethyl oder Cycloalkyl mit 3, 4, 5, 6 oder 7 C-Atomen;
sowie deren Salzen;
**dadurch gekennzeichnet, dass** 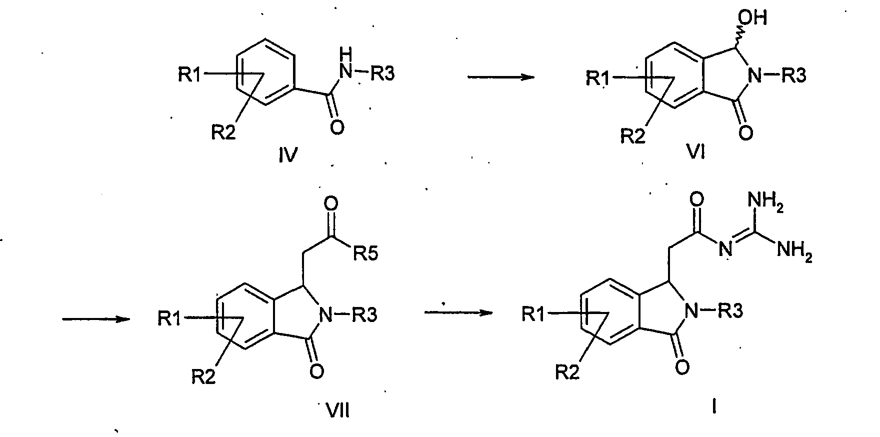
a) das Amid der Formel IV formyliert und dann zur Verbindung der Formel VI cyclisiert wird,
b) die Verbindung der Formel VI mit einem Alkoxycarbonylmethylentriphenylphosphoran, mit einem 1-Alkoxy-1-trimethylsiloxyethylen oder mit einem Trialkylphosphonoacetat zur Verbindung der Formel VII umgesetzt wird und
c) die Verbindung der Formel VII mit Guanidin zur Verbindung der Formel I umgesetzt wird,
wobei in den Verbindungen der Formeln IV, VI und VII
R1 bis R3 definiert sind wie in Formel 1 und
R5 Alkoxy mit 1, 2, 3 oder 4 C-Atomen ist;
sowie deren Salze.

2. Verfahren zur Herstellung von Verbindungen der Formel I nach Anspruch 1 wobei bedeuten
R1 und R2 unabhängig voneinander Wasserstoff, F, Cl, Trifluormethoxy, 2,2,2-Trifluorethoxy, Trifluormethyl, 2,2,2-Trifluorefihyl oder Alkyl mit 1, 2, 3 oder 4 C-Atomen;
R3 Alk-R4 oder Trifluormethyl;
Alk Alkyl mit 1, 2, 3 oder 4 C-Atomen;
R4 Wasserstoff, Trifluormethyl oder Cycloalkyl mit 3, 4, 5, 6 oder 7 C-Atomen;
sowie deren Salzen;
**dadurch gekennzeichnet, dass** 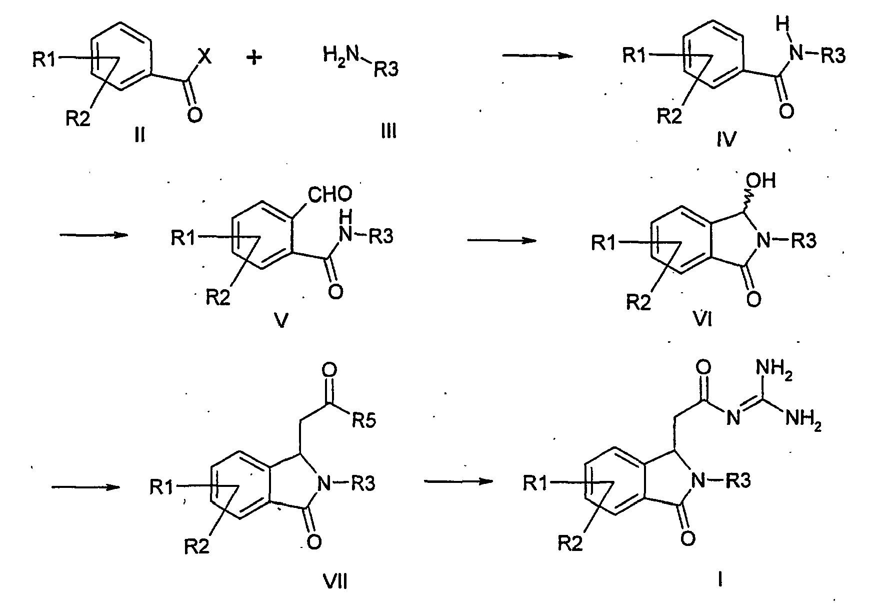
a) die Verbindung der Formel II mit dem Amin der Formel III zum Amid der Formel IV umgesetzt wird,
b) das Amid der Formel IV an ortho-Position zur Amidfunktion zu dem Formyl-amid der Formel V formyliert wird,
c) das Formyl-amid der Formel V zur Verbindung der Formel VI cyclisiert wird,
d) die Verbindung der Formel VI mit einem Alkoxycarbonylmethylentriphenylphosphoran, mit einem 1-Alkoxy-1-trimethylsiloxyethylen oder mit einem Trialkylphosphonoacetat zur Verbindung der Formel VH umgesetzt wird und
e) die Verbindung der Formel VII mit Guanidin zur Verbindung der Formel I umgesetzt wird,
wobei in den Verbindungen der Formeln II, III, IV, V, VI und VII
R1 bis R3 definiert sind wie in Formel I,
R5 Alkoxy mit 1, 2, 3 oder 4 C-Atomen ist und
X Cl, Br, OH oder Alkoxy mit 1, 2, 3 oder 4 C-Atomen ist;
sowie deren Salze

3. Verfahren nach Anspruch 1 und/oder 2, bei dem die Verfahrensschritte unabhängig voneinander kontinuierlich oder diskontinuierlich geführt werden.

4. Verfahren nach einem oder mehreren der Ansprüche 1, 2 oder 3, worin die Verbindung der Formel I definiert ist als N-{2-[3-Oxo-2-(2,2,2-trifluorethyl)-6-trifluormethyl-2,3-dihydro-1H-isoindol-1-yl]-acetyl}guanidin, sowie dessen pharmazeutisch verträgliche Salze.

5. Verfahren zur Herstellung von Verbindungen der Formel I , 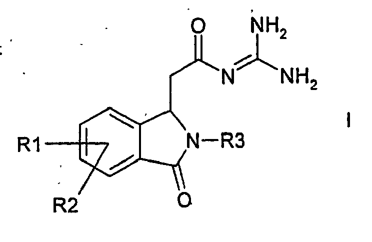 wobei bedeuten
R1 und R2 unabhängig voneinander Wasserstoff, F, Cl, Trifluormethoxy, 2,2,2-Trifluorethoxy, Trifluormethyl, 2,2,2-Trifluorethyl oder Alkyl mit 1, 2, 3 oder 4 C-Atomen;
R3 Alk-R4 oder Trifluormethyl;
Alk Alkyl mit 1, 2, 3 oder 4 C-Atomen;
R4 Wasserstoff, Trifluormethyl oder Cycloalkyl mit 3, 4, 5, 6 oder 7 C-Atomen;
sowie deren Salzen;
**dadurch gekennzeichnet, dass** 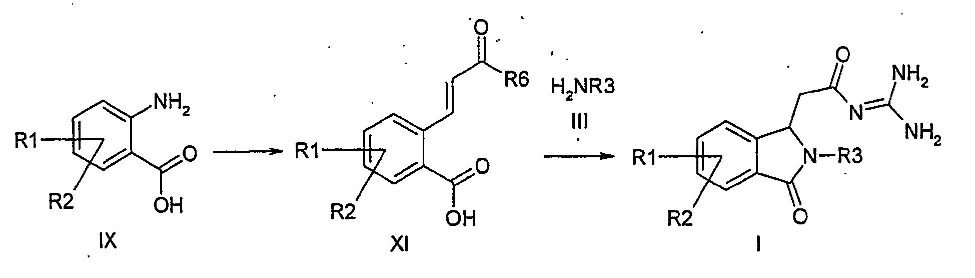
a) das Amin der Formel IX über ein Diazoniumsalz mit einem Acrylsäure-Alkylester zum Zimtsäurederivat der Formel XI umgesetzt wird,
b) die Verbindung der Formel XI mit dem Amin der Formel III und mit Guanidin zum Acylguanidin der Formel I umgesetzt wird,
wobei in den Verbindungen der Formeln III, IX und XI
R1 bis R3 definiert sind wie in Formel und
R6 Alkoxy mit 1, 2, 3 oder 4 C-Atomen ist;
sowie deren Salze.

6. Verfahren zur Herstellung von Verbindungen der Formel I nach Anspruch 5 wobei bedeuten
R1 und R2 unabhängig voneinander Wasserstoff, F, Cl, Trifluormethoxy, 2,2,2-Trifluorethoxy, Trifluormethyl, 2,2,2-Trifluorethyl oder Alkyl mit 1, 2, 3 oder 4 C-Atomen;
R3 Alk-R4 oder Trifluormethyl;
Alk Alkyl mit 1, 2, 3 oder 4 C-Atomen;
R4 Wasserstoff, Trifluormethyl oder Cycloalkyl mit 3, 4, 5, 6 oder 7 C-Atomen;
sowie deren Salzen;
**dadurch gekennzeichnet, dass** 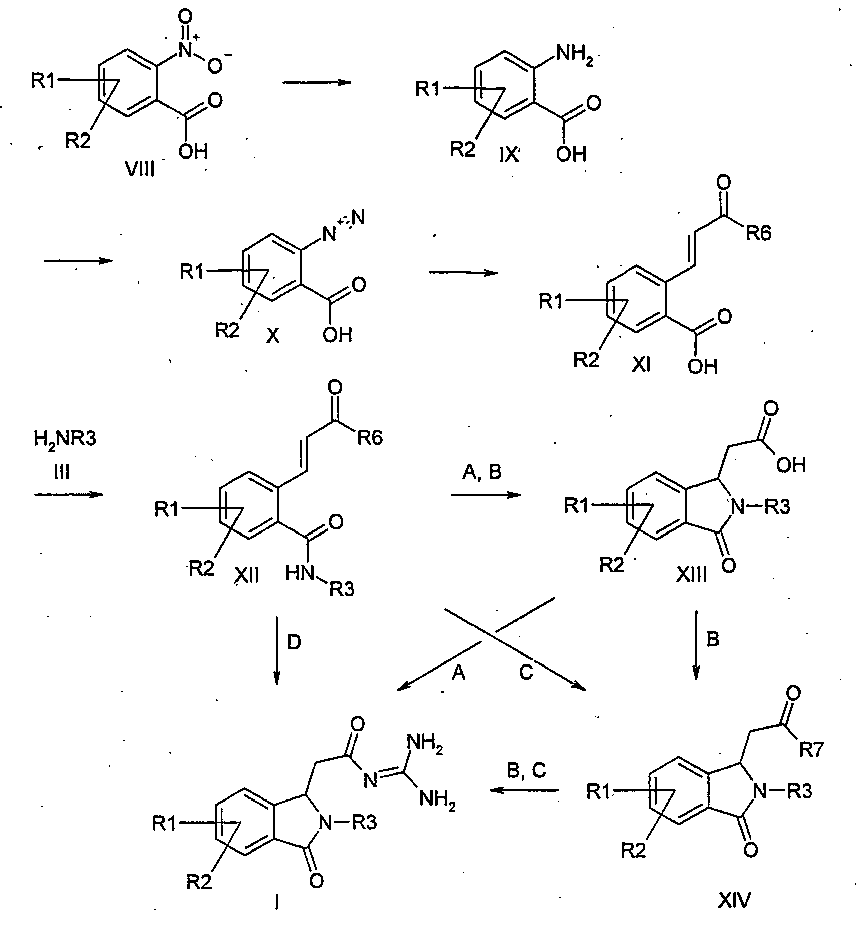
a) die Nitroverbindung der Formel VIII zum Amin der Formel IX umgesetzt wird,
b) das Amin der Formel IX zum Diazoniumsalz der Formel X umgesetzt wird,
c) das Diazoniumsalz der Formel X mit einem Acrylsäure-Alkylester zum Zimtsäurederivat der Formel XI umgesetzt wird,
d) die Verbindung der Formel XI zum Amid der Formel XII umgesetzt wird und
e) die Verbindung der Formel XII zum Acylguanidin der Formel I umgesetzt wird, entweder durch Umsetzung der Verbindung der Formel XII in Gegenwart einer Base zum Isoindolon-Derivat der Formel XIII und anschließend durch Umsetzung mit Guanidin unter Aktivierung zum Acylguanidin der Formel I (Alternative A), oder nach Bildung des Isoindolon-Derivats der Formel XIII in Gegenwart einer Base aus der Verbindung der Formel XII durch Umwandlung der Verbindung der Formel XIII in den Ester der Formel XIV und anschließend durch Umsetzung mit Guanidin zum Acylguanidin der Formel I (Alternative B), oder
durch Umsetzung der Verbindung der Formel XII in Gegenwart einer starken Base zum Ester der Formel XIV und anschließend durch Umsetzung mit Guanidin zum Acylguanidin der Formel I (Alternative C), oder
durch direkte Umsetzung der Verbindung der Formel XII mit Guanidin in Gegenwart einer Base unter gleichzeitig erfolgender Guanylierung und Cyclisierung zum Isoindolon der Formel I (Alternative D),
wobei in den Verbindungen der Formeln VIII, IX, X, XI, XII, XIII und XIV
R1 bis R3 definiert sind wie in Formel I und
R6 und R7 unabhängig voneinander Alkoxy mit 1, 2, 3 oder 4 C-Atomen sind;
sowie deren Salze.

7. Verfahren nach Anspruch 6, wobei im Verfahrensschritt e) die Alternative D verwendet wird.

8. Verfahren nach Anspruch 6 und/oder 7, bei dem die Verfahrensschritte d) und e) im Eintopfverfahren durchgeführt werden.

9. Verfahren nach Anspruch 5 und/oder 6, bei dem die Verfahrensschritte, unabhängig voneinander kontinuierlich oder diskontinuierlich geführt werden.

10. Verfahren nach einem oder mehreren der Ansprüche 5 bis 9, worin die Verbindung der Formel I definiert ist als N-{2-[3-Oxo-2-(2,2,2-trifluorethyl)-6-trifluormethyl-2,3-dihydro-1H-isoindol-1-yl]-acetyl}-guanidin, sowie dessen pharmazeutisch verträgliche Salze.

11. Verbindungen der Formel XII 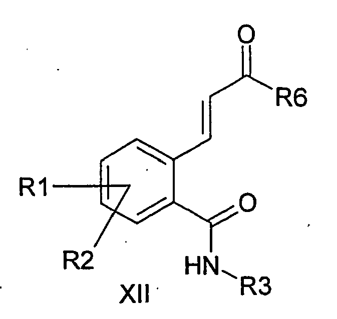 wobei bedeuten
R1 und R2 unabhängig voneinander Wasserstoff, F, Cl, Trifluormethoxy, 2,2,2-Trifluorethoxy, Trifluormethyl, 2,2,2-Trifluorethyl oder Alkyl mit 1, 2, 3 oder 4 C-Atomen;
R3 Alk-R4 oder Trifluormethyl;
Alk Alkyl mit 1,2,3 oder 4 C-Atomen;
R4 Wasserstoff, Trifluormethyl oder Cycloalkyl mit 3, 4, 5, 6 oder 7 C-Atomen;
R6 Alkoxy mit 1, 2, 3 oder 4 C-Atomen;
sowie deren Salze.

12. Verbindungen der Formel XII nach Anspruch 11 zur Verwendung als Syntheseintermediate.

13. Verfahren zur Isolierung von Verbindungen der Formel Ia und Ib 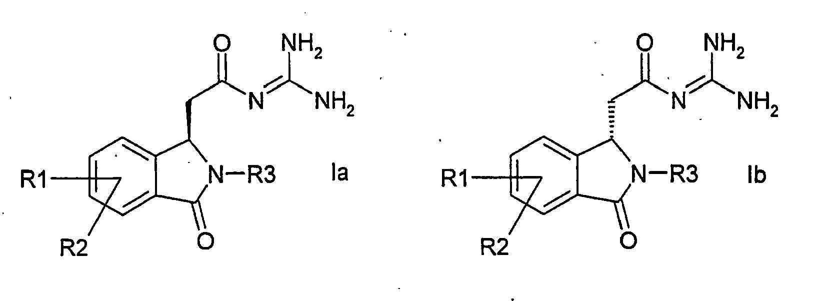 wobei bedeuten
R1 und R2 unabhängig voneinander Wasserstoff, F, Cl, Trifluormethoxy, 2,2,2-Trifluorethoxy, Trifluormethyl, 2,2,2-Trifluorethyl oder Alkyl mit 1, 2, 3 oder 4 C-Atomen;
R3 Alk-R4 oder Trifluormethyl;
Alk Alkyl mit 1, 2, 3 oder 4 C-Atomen;
R4 Wasserstoff, Trifluormethyl oder Cycloalkyl mit 3, 4, 5, 6 oder 7 C-Atomen;
sowie deren Salzen;
**dadurch gekennzeichnet, dass** 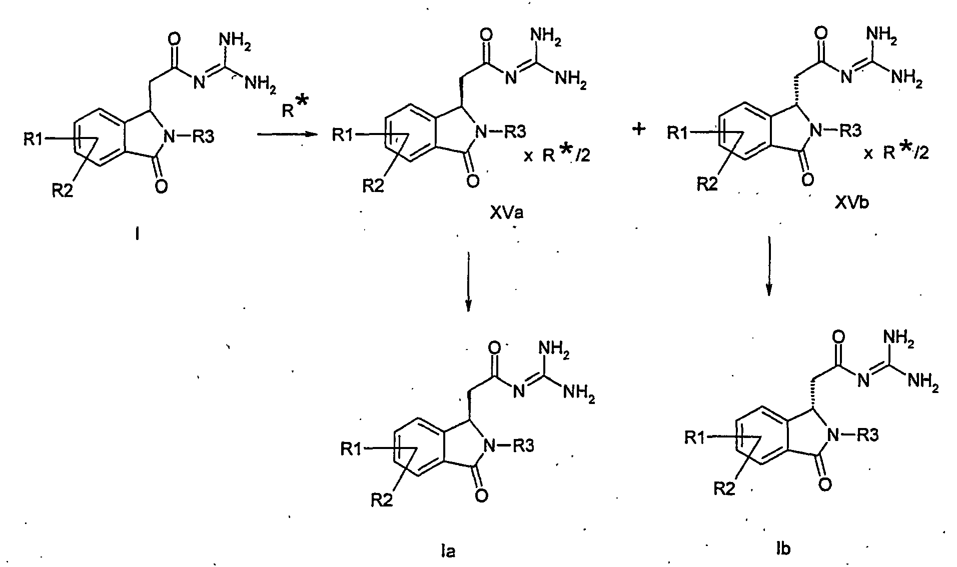
a) die Verbindung der Formel I zu Salzen von einer 2,3-O-acylierteri D- oder L-Weinsäure umgesetzt und durch Kristallisation die beiden Salze der Formeln XVa und XVb getrennt erhalten werden, und
b) die freien Basen der Formeln Ia bzw. Ib aus den beiden Salzen der Formeln XVa bzw. XVb freigesetzt werden,
wobei in den Verbindungen der Formeln I, XVa und XVb
R1 bis R3 definiert sind wie in den Formeln Ia und Ib
R* 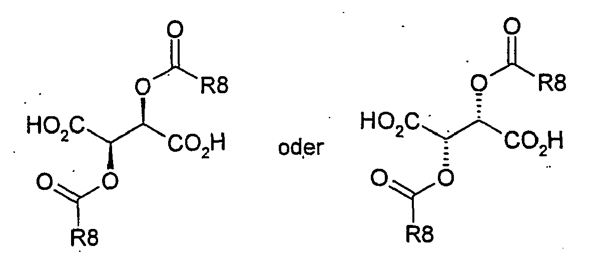 bedeutet
R8 Alkyl mit 1, 2, 3, 4, 5 oder 6 C-Atomen oder Phenyl; das unsubstituiert oder substituiert ist mit 1, 2 oder 3 Substituenten aus der Gruppe F, Cl, Br, I, Alkyl mit 1, 2, 3 oder 4 C-Atomen oder Alkoxy mit 1, 2, 3 oder 4 C-Atomen, bedeutet.

14. Verfahren nach Anspruch 13, wobei das unerwünschte Enantiomer der Formeln Ia oder Ib wieder racemisiert wird.

15. Verfahren nach Anspruch 13 und/oder 14, wobei die Verbindungen der Formeln Ia und Ib (R)-N-{2-[3-Oxo-2-(2,2,2-trifluorethyl)-6-trifluormethyl-2,3-dihydro-1H-isoindol-1-yl]-acetyl}-guanidin und (S)-N-{2-[3-Oxo-2-(2,2,2-trifluorethyl)-6-trifluormethyl-2,3-dihydro-1H-isoindol-1-yl]-acetyl}-guanidin sind.

16. Verbindungen der Formel XVa und/oder XVb 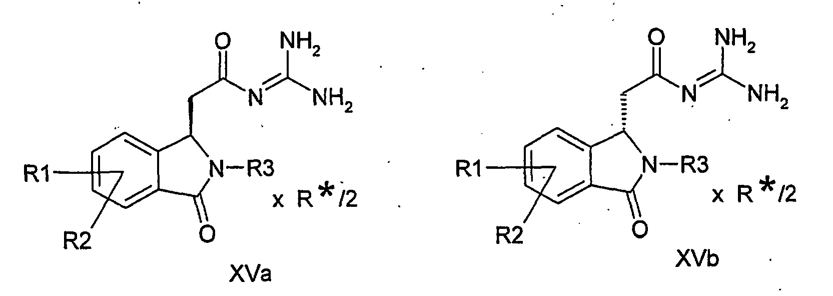 wobei bedeuten
R1 und R2 unabhängig voneinander Wasserstoff, F, Cl, Trifluormethoxy, 2,2,2-Trifluorethoxy, Trifluormethyl, 2,2,2-Trifluorethyl oder Alkyl mit 1, 2, 3 oder 4 C-Atomen;
R3 Alk-R4 oder Trifluormethyl;
Alk Alkyl mit 1, 2, 3 oder 4 C-Atomen;
R4 Wasserstoff, Trifluormethyl oder Cycloalkyl mit 3, 4, 5, 6 oder 7 C-Atomen;
R* 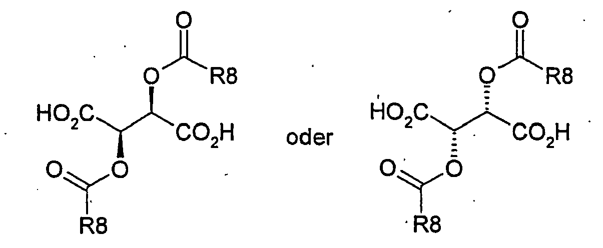
R8 Alkyl mit 1, 2, 3, 4, 5 oder 6 C-Atomen oder Pheny,das unsubstituiert oder substituiert ist mit 1, 2 oder 3 Substituenten aus der Gruppe F, Cl, Br, I, Alkyl mit 1, 2, 3 oder 4 C-Atomen oder Alkoxy mit 1, 2, 3 oder 4 C-Atomen.

17. (S)-N-{2-[3-Oxo-2-(2,2,2-trifluorethyl)-6-trifluormethyl-2,3-dihydro-1 H-isoindol-1-y)]-acety)}-guahidin Hydrogenfumarat Hydrat der Formel XVI.

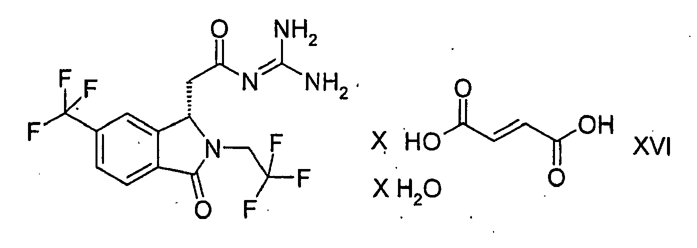

18. Verbindung der Formeln XVa oder XVb nach Anspruch 16 oder der Formel XVI nach Anspruch 17 zur Verwendung als Medikament.

19. Verwendung einer Verbindung der Formeln XVa oder XVb nach Anspruch 16 oder der Formel XVI nach Anspruch 17 allein oder in Kombination mit anderen Arzneimitteln oder Wirkstoffen zur Herstellung eines Medikaments zur Behandlung oder Prophylaxe von akuten oder chronischen Schäden, Erkrankungen oder indirekte Folgeerkrankungen von Organen und Geweben, die durch Ischämie- oder durch Reperfusionsereignisse verursacht werden, zur Behandlung oder Prophylaxe von Arrhythmien, des lebensbedrohlichen Kammerflimmerns des Herzens, des Herzinfarkts, der Angina Pectoris, zur Behandlung oder Prophylaxe von ischämischen Zuständen des Herzens, von ischämischen Zuständen des peripheren und zentralen-Nervensystems oder des Schlaganfalls oder von ischämischen Zuständen peripherer Organe und Gewebe, zur Behandlung oder Prophylaxe von Schockzuständen, von Krankheiten, bei denen die Zellproliferation eine primäre oder sekundäre Ursache darstellt, von Krebs, der Metastasierung, der Prostata-Hypertrophie bzw. der ProstataHyperplasie, der Atherosklerose oder von Störungen des Fettstoffwechsels, des Bluthochdrucks, der essentiellen Hypertonie, von Erkrankungen des Zentralnervensystems, von Erkrankungen, die durch ZNS-Übererregbarkeit resultieren, Epilepsie oder zentral ausgelöste Krämpfe, von Erkrankungen des Zentralnervensystems, insbesondere von Angstzuständen, Depressionen oder Psychosen, zur Behandlung oder Prophylaxe des non insulin dependent diabetes mellitus (NIDDM) oder diabetischer Spätschäden, von Thrombosen, von Erkrankungen infolge endothelialer Dysfunktion, von claudicatio intermittens, zur Behandlung oder Prophylaxe fibrotischer Erkrankungen innerer Organe, fibrotischer Erkrankungen der Leber, fibrotischer Erkrankungen der Niere, fibrotischer Erkrankungen von Gefäßen und fibrotischer Erkrankungen des Herzens, zur Behandlung oder Prophylaxe der Herzinsuffizienz oder des Congestive Heart failure, akuter oder chronischer inftammatorischer Erkrankungen, von Erkrankungen, die durch Protozoen verursacht werden, von Malaria und der Hühnercoccidiose und zum Einsatz bei chirurgischen Operationen und Organtransplantationen, zur Konservierung und Lagerung von Transplantaten für chirurgische Maßnahmen, zum Einsatz bei Bypassoperationen, zum Einsatz bei Wiederbelebung nach Herzstillstand, zur Verhinderung altersbedingter Gewebsveränderung, zur Herstellung eines gegen die Alterung gerichteten Medikaments oder zur Lebensverlängerung, zur Behandlung und Senkung der cardiotoxischen Wirkungen in der Thyreotoxikose oder zur Herstellung eines Diagnostikums.

20. Heilmittel für die humane, veterinäre und/oder phytoprotektive Anwendung enthaltend eine wirksame Menge einer Verbindung der Formeln XVa oder XVb nach Anspruch 16 zusammen mit pharmazeutisch annehmbaren Träger- und Zusatzstoffen.

21. Heilmittel für die humane, veterinäre oder phytoprotektive Anwendung enthaltend eine wirksame Menge einer Verbindung der Formeln XVa oder XVb nach Anspruch 16 zusammen mit pharmazeutisch annehmbaren Träger- und Zusatzstoffen in Kombination mit anderen pharmakologischen Wirkstoffen oder Arzneimitteln.

22. Heilmittel für die humane, veterinäre und/oder phytoprotektive Anwendung enthaltend eine wirksame Menge der Verbindung der Formel XVI nach Anspruch 17 zusammen mit pharmazeutisch annehmbaren Träger- und Zusatzstoffen.

23. Heilmittel für die humane, veterinäre oder phytoprotektive Anwendung enthaltend eine wirksame Menge der Verbindung der Formel XVI nach Anspruch 17 zusammen mit pharmazeutisch annehmbaren Träger- und Zusatzstoffen in Kombination mit anderen pharmakologischen Wirkstoffen oder Arzneimitteln.

## Claims

1. A process for preparing compounds of the formula I 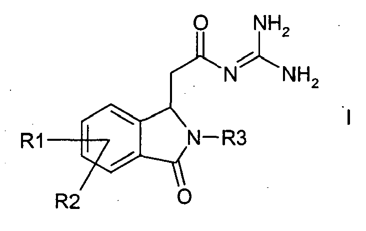 where
R1 and R2 are each independently hydrogen, F, Cl, trifluoromethoxy, 2,2,2-trifluoroethoxy, trifluoromethyl, 2,2,2-trifluoroethyl or alkyl having 1, 2, 3 or 4 carbon atoms;
R3 is Alk-R4 or trifluoromethyl;
Alk is alkyl having 1, 2, 3 or 4 carbon atoms;
R4 is hydrogen, trifluoromethyl or cycloalkyl having 3, 4, 5, 6 or 7 carbon atoms;
and salts thereof;
which comprises 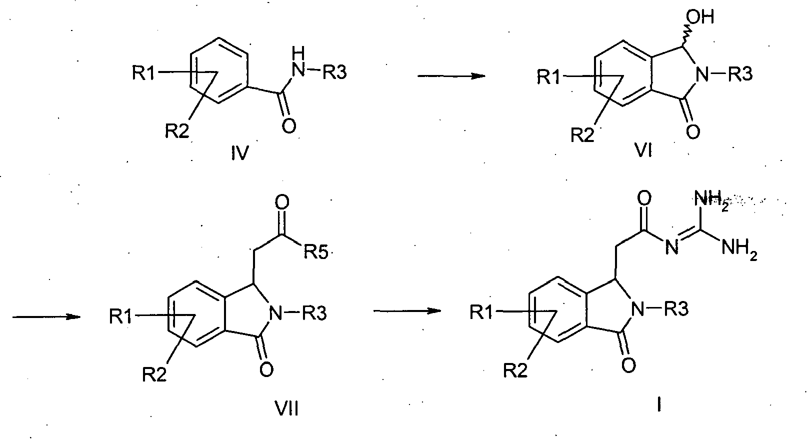
a) formylating the amide of the formula IV and then cyclizing to the compound of the formula VI,
b) reacting the compound of the formula VI with an alkoxycarbonylmethylenetriphenylphosphorane, with a 1-alkoxy-1-trimethylsiloxyethylene or with a trialkyl phosphonoacetate to give the compound of the formula VII, and
c) reacting the compound of the formula VII with guanidine to give the compound of the formula I,
where, in the compounds of the formulae IV, VI and VII,
R1 to R3 are each as defined in formula I and
R5 is alkoxy having 1, 2, 3 or 4 carbon atoms;
and salts thereof.

2. The process for preparing compounds of the formula I as claimed in claim 1 where
R1 and R2 are each independently hydrogen, F, Cl, trifluoromethoxy, 2,2,2-trifluoroethoxy, trifluoromethyl, 2,2,2-trifluoroethyl or alkyl having 1, 2, 3 or 4 carbon atoms;
R3 is Alk-R4 or trifluoromethyl;
Alk is alkyl having 1, 2, 3 or 4 carbon atoms;
R4 is hydrogen, trifluoromethyl or cycloalkyl having 3, 4, 5, 6 or 7 carbon atoms;
and salts thereof;
wherein 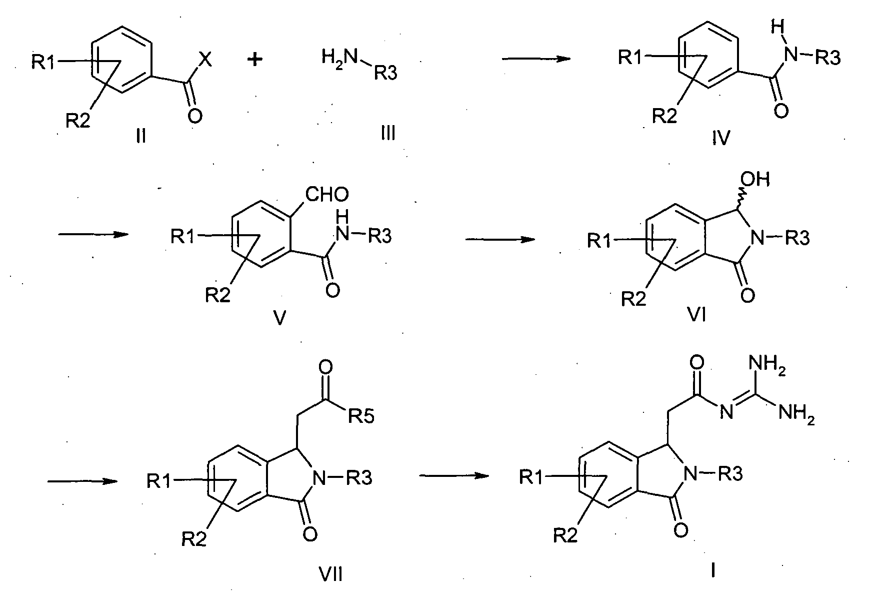
a) the compound of the formula II is reacted with the amine of the formula III to give the amide of the formula IV,
b) the amide of the formula IV is formylated at the ortho-position to the amide function to give the formyl amide of the formula V,
c) the formyl amide of the formula V is cyclized to the compound of the formula VI,
d) the compound of the formula VI is reacted with an alkoxycarbonylmethylenetriphenylphosphorane, with a 1-alkoxy-1-trimethylsiloxyethylene or with a trialkyl phosphonoacetate to give the compound of the formula VII and
e) the compound of the formula VII is reacted with guanidine to give the compound of the formula I,
where, in the compounds of the formulae II, III, IV, V, VI and VII,
R1 to R3 are each as defined in formula I,
R5 is alkoxy having 1, 2, 3 or 4 carbon atoms and
X is Cl, Br, OH or alkoxy having 1, 2, 3 or 4 carbon atoms;
and salts thereof.

3. The process as claimed in claim 1 and/or 2, in which the process steps are each independently conducted continuously or batchwise.

4. The process as claimed in one or more of claims 1, 2 or 3, wherein the compound of the formula I is defined as N-{2-[3-oxo-2-(2,2,2-trifluoroethyl)-6-trifluoromethyl-2,3-dihydro-1H-isoindol-1-yl]acetyl}guanidine, and pharmaceutically acceptable salts thereof.

5. A process for preparing compounds of the formula I 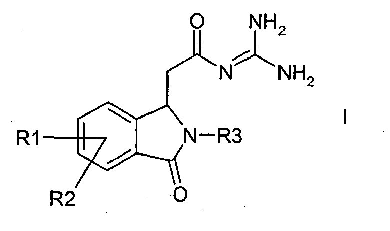 where
R1 and R2 are each independently hydrogen, F, Cl, trifluoromethoxy, 2,2,2-trifluoroethoxy, trifluoromethyl, 2,2,2-trifluoroethyl or alkyl having 1, 2, 3 or 4 carbon atoms;
R3 is Alk-R4 or trifluoromethyl;
Alk is alkyl having 1, 2, 3 or 4 carbon atoms;
R4 is hydrogen, trifluoromethyl or cycloalkyl having 3, 4, 5, 6 or 7 carbon atoms;
and salts thereof;
which comprises 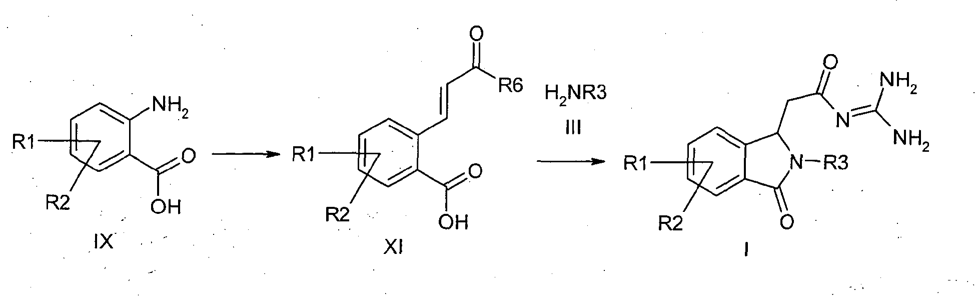
a) reacting the amine of the formula IX via a diazonium salt with an alkyl acrylate to give the cinnamic acid derivative of the formula XI,
b) reacting the compound of the formula XI with the amine of the formula III and with guanidine to give the acylguanidine of the formula I,
where, in the compounds of the formulae III, IX and XI,
R1 to R3 are each as defined in formula I and
R6 is alkoxy having 1, 2, 3 or 4 carbon atoms;
and salts thereof.

6. The process for preparing compounds of the formula I as claimed in claim 5 where
R1 and R2 are each independently hydrogen, F, Cl, trifluoromethoxy, 2,2,2-trifluoroethoxy, trifluoromethyl, 2,2,2-trifluoroethyl or alkyl having 1, 2, 3 or 4 carbon atoms;
R3 is Alk-R4 or trifluoromethyl;
Alk is alkyl having 1, 2, 3 or 4 carbon atoms;
R4 is hydrogen, trifluoromethyl or cycloalkyl having 3, 4, 5, 6 or 7 carbon atoms;
and salts thereof;
wherein 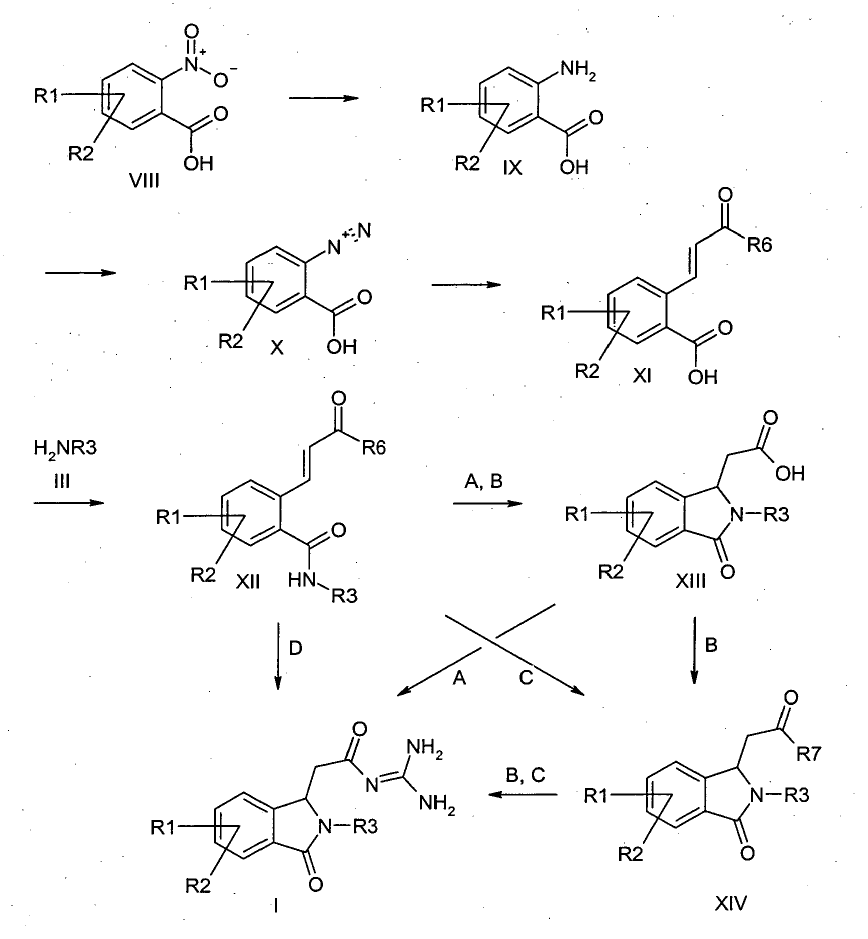
a) the nitro compound of the formula VIII is converted to the amine of the formula IX,
b) the amine of the formula IX is converted to the diazonium salt of the formula X,
c) the diazonium salt of the formula X is reacted with an alkyl acrylate to give the cinnamic acid derivative of the formula XI,
d) the compound of the formula XI is converted to the amide of the formula XII and
e) the compound of the formula XII is converted to the acylguanidine of the formula I, either by converting the compound of the formula XII in the presence of a base to the isoindolone derivative of the formula XIII and subsequently by reaction with guanidine with activation to give the acylguanidine of the formula I (alternative A), or, after formation of the isoindolone derivative of the formula XIII, in the presence of a base, from the compound of the formula XII, by converting the compound of the formula XIII to the ester of the formula XIV and subsequently by reacting with guanidine to give the acylguanidine of the formula I (alternative B), or by converting the compound of the formula XII in the presence of a strong base to the ester of the formula XIV and subsequently by reacting with guanidine to the acylguanidine of the formula I (alternative C), or
by directly reacting the compound of the formula XII with guanidine in the presence of a base with simultaneously proceeding guanylation and cyclization to give the isoindolone of the formula I (alternative D),
where, in the compounds of the formulae VIII, IX, X, XI, XII, XIII and XIV,
R1 to R3 are each as defined in formula I and
R6 and R7 are each independently alkoxy having 1, 2, 3 or 4 carbon atoms;
and salts thereof.

7. The process as claimed in claim 6, wherein, in process step e), alternative D is used.

8. The process as claimed in claim 6 and/or 7, in which process steps d) and e) are carried out in a one-pot process.

9. The process as claimed in claim 5 and/or 6, in which the process steps are each independently conducted continuously or batchwise.

10. The process as claimed in one or more of claims 5 to 9, wherein the compound of the formula I is defined as N-{2-[3-oxo-2-(2,2,2-trifluoroethyl)-6-trifluoromethyl-2,3-dihydro-1 H-isoindol-1-yl]acetyl}guanidine, and pharmaceutically acceptable salts thereof.

11. A compound of the formula XII 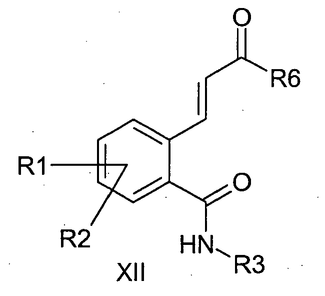 where
R1 and R2 are each independently hydrogen, F, Cl, trifluoromethoxy, 2,2,2-trifluoroethoxy, trifluoromethyl, 2,2,2-trifluoroethyl or alkyl having 1, 2, 3 or 4 carbon atoms;
R3 is Alk-R4 or trifluoromethyl;
Alk is alkyl having 1, 2, 3 or 4 carbon atoms;
R4 is hydrogen, trifluoromethyl or cycloalkyl having 3, 4, 5, 6 or 7 carbon atoms;
R6 is alkoxy having 1, 2, 3 or 4 carbon atoms;
and salts thereof.

12. A compound of the formula XII as claimed in claim 11 for use as a synthetic intermediate.

13. A process for isolating compounds of the formula la and Ib 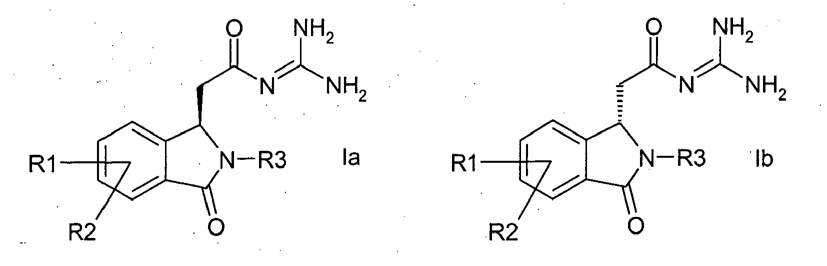 where
R1 and R2 are each independently hydrogen, F, Cl, trifluoromethoxy, 2,2,2-trifluoroethoxy, trifluoromethyl, 2,2,2-trifluoroethyl or alkyl having 1, 2, 3 or 4 carbon atoms;
R3 is Alk-R4 or trifluoromethyl;
Alk is alkyl having 1, 2, 3 or 4 carbon atoms;
R4 is hydrogen, trifluoromethyl or cycloalkyl having 3, 4, 5, 6 or 7 carbon atoms;
and salts thereof;
which comprises 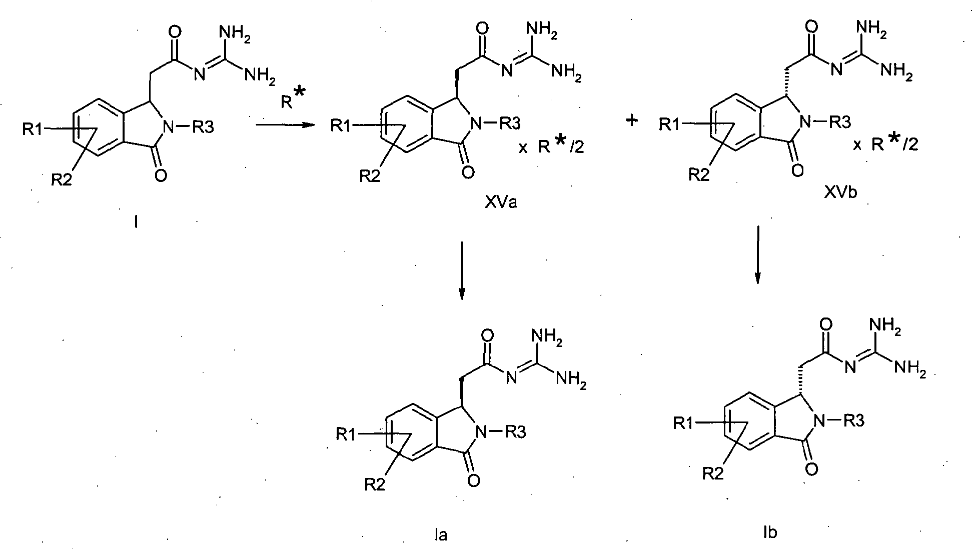
a) converting the compound of the formula I to salts of a 2,3-O-acylated D- or L-tartaric acid and obtaining the two salts of the formulae XVa and XVb separately by crystallization, and
b) releasing the free bases of the formulae la and Ib from the two salts of the formulae XVa and XVb respectively,
where, in the compounds of the formulae I, XVa and XVb,
R1 to R3 are each as defined in the formulae la and Ib
R* is 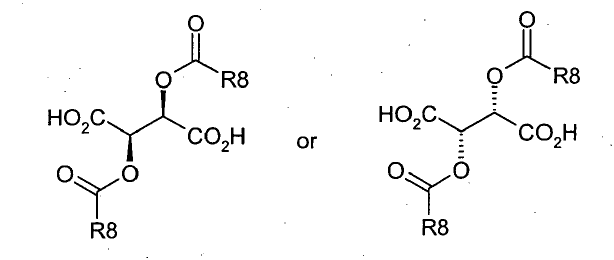 R8 is alkyl having 1, 2, 3, 4, 5 or 6 carbon atoms or phenyl which is unsubstituted or substituted by 1, 2 or 3 substituents from the group of F, Cl, Br, I, alkyl having 1, 2, 3 or 4 carbon atoms or alkoxy having 1, 2, 3 or 4 carbon atoms.

14. The process as claimed in claim 13, wherein the undesired enantiomer of the formulae la or Ib is racemized again.

15. The process as claimed in claim 13 and/or 14, wherein the compounds of the formulae la and Ib are (R)-N-{2-[3-oxo-2-(2,2,2-trifluoroethyl)-6-trifluoromethyl-2,3-dihydro-1 H-isoindol-1-yl]acetyl}guanidine and (S)-N-{2-[3-oxo-2-(2,2,2-trifluoroethyl)-6-trifluoromethyl-2,3-dihydro-1 H-isoindol-1-yl]acetyl}guanidine.

16. A compound of the formula XVa and/or XVb 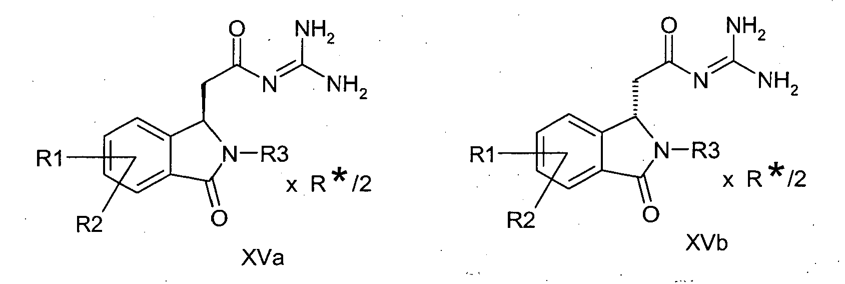 where
R1 and R2 are each independently hydrogen, F, Cl, trifluoromethoxy, 2,2,2-trifluoroethoxy, trifluoromethyl, 2,2,2-trifluoroethyl or alkyl having 1, 2, 3 or 4 carbon atoms;
R3 is Alk-R4 or trifluoromethyl;
Alk is alkyl having 1, 2, 3 or 4 carbon atoms;
R4 is hydrogen, trifluoromethyl or cycloalkyl having 3, 4, 5, 6 or 7 carbon atoms;
R* is 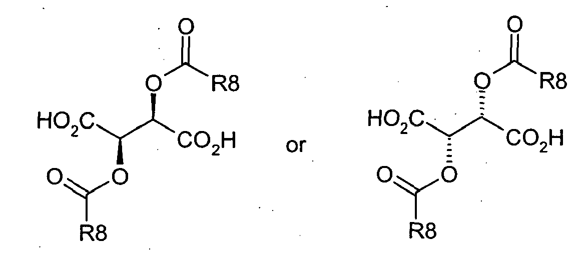
R8 is alkyl having 1, 2, 3, 4, 5 or 6 carbon atoms or phenyl which is unsubstituted or substituted by 1, 2 or 3 substituents from the group of F, Cl, Br, I, alkyl having 1, 2, 3 or 4 carbon atoms or alkoxy having 1, 2, 3 or 4 carbon atoms.

17. (S)-N-{2-[3-oxo-2-(2,2,2-trifluoroethyl)-6-trifluoromethyl-2,3-dihydro-1 H-isoindol-1-yl]acetyl}guanidine hydrogenfumarate hydrate of the formula XVI

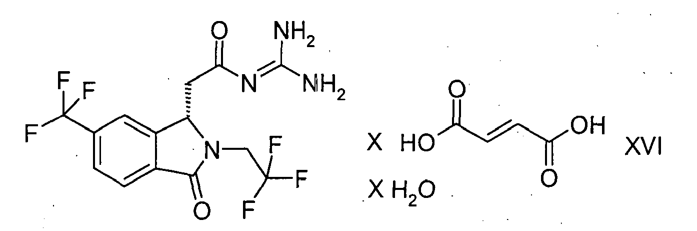

18. A compound of the formulae XVa or XVb as claimed in claim 16 or of the formula XVI as claimed in claim 17 for use as a medicament.

19. The use of a compound of the formulae XVa or XVb as claimed in claim 16 or of the formula XVI as claimed in claim 17, alone or in combination with other medicaments or active ingredients, for producing a medicament for the treatment or prophylaxis of acute or chronic damage, disorders or indirect sequelae of organs and tissues caused by ischemic or by reperfusion events, for the treatment or prophylaxis of arrhythmias, of life-threatening cardiac ventricular fibrillation, of myocardial infarction, of angina pectoris, for the treatment or prophylaxis of ischemic states of the heart, of ischemic states of the peripheral and central nervous system or of stroke or of ischemic states of peripheral organs and tissues, for the treatment or prophylaxis of states of shock, of diseases in which cellular proliferation represents a primary or secondary cause, of cancer, of metastasis, of prostate hypertrophy and of prostate hyperplasia, of atherosclerosis or of disturbances of lipid metabolism, of high blood pressure, of essential hypertension, of disorders of the central nervous system, of disorders resulting from overexcitability of the CNS, epilepsy or centrally induced convulsions, of disorders of the central nervous system, especially of anxiety states, depressions or psychoses, for the treatment or prophylaxis of non-insulin-dependent diabetes mellitus (NIDDM) or late damage from diabetes, of thromboses, of disorders resulting from endothelial dysfunction, of intermittent claudicating, for the treatment or prophylaxis of fibrotic disorders of internal organs, fibrotic disorders of the liver, fibrotic disorders of the kidney, fibrotic disorders of vessels and fibrotic disorders of the heart, for the treatment or prophylaxis of heart failure or of congestive heart failure, of acute or chronic inflammatory disorders, of disorders caused by protozoa, of malaria or of coccidiosis in poultry, and for use for surgical operations and organ transplantations, for preserving and storing transplants for surgical procedures, for use in bypass operations, for use in resuscitation after cardiac arrest, for preventing age-related tissue change, for producing a medicament directed against aging or for prolonging life, for the treatment and reduction of cardiotoxic effects in thyrotoxicosis or for producing a diagnostic aid.

20. A medicine for human, veterinary and/or phytoprotective use, comprising an effective amount of a compound of the formulae XVa or XVb as claimed in claim 16 together with pharmaceutically acceptable excipients and additives.

21. A medicine for human, veterinary or phytoprotective use, comprising an effective amount of a compound of the formulae XVa or XVb as claimed in claim 16 together with pharmaceutically acceptable excipients and additives in combination with other pharmacological active ingredients or medicaments.

22. A medicine for human, veterinary and/or phytoprotective use, comprising an effective amount of a compound of the formula XVI as claimed in claim 17 together with pharmaceutically acceptable excipients and additives.

23. A medicine for human, veterinary or phytoprotective use, comprising an effective amount of the compound of the formula XVI as claimed in claim 17 together with pharmaceutically acceptable excipients and additives in combination with other pharmacological active ingredients or medicaments.

## Revendications

1. Procédé de préparation de composés de formule I 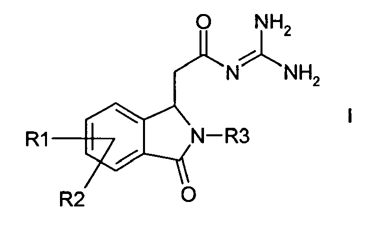 dans laquelle
R1 et R2 sont chacun indépendamment un atome d'hydrogène, un atome de fluor, un atome de chlore, un groupe trifluorométhoxy, un groupe 2,2,2-trifluoroéthoxy, un groupe trifluorométhyle, un groupe 2,2,2-trifluoroéthyle ou un groupe alkyle renfermant 1, 2, 3 ou 4 atomes de carbone ;
R3 est un groupe Alk-R4 ou un groupe trifluorométhyle ;
Alk est un groupe alkyle renfermant 1, 2, 3 ou 4 atomes de carbone ;
R4 est un atome d'hydrogène, un groupe trifluorométhyle ou un groupe cycloalkyle renfermant 3, 4, 5, 6 ou 7 atomes de carbone ;
et de leurs sels;
**caracterisé en ce que** 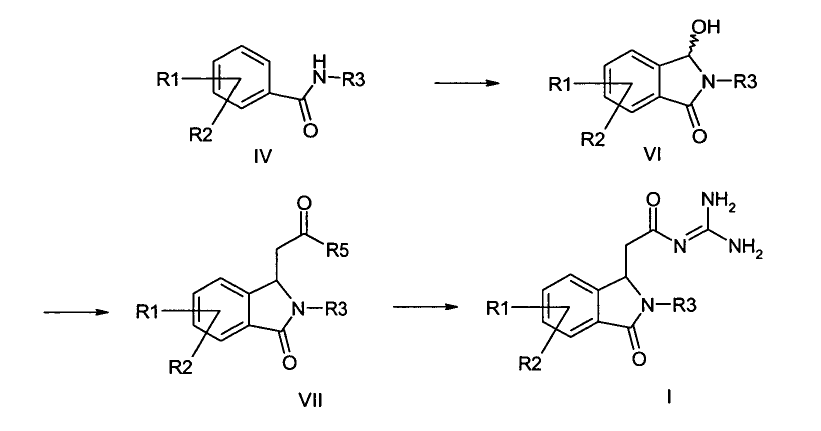
a) l'amide de formule IV est formylé, puis cyclisé pour donner lieu au composé de formule VI,
b) le composé de formule VI est mis à réagir avec un alcoxycarbonylméthylène-triphénylphosphorane, avec un 1-alcoxy-1-triméthylsiloxyéthylène ou avec un phosphonoacétate de trialkyle, pour donner lieu au composé de formule VII, et
c) le composé de formule Vil est mis à réagir avec de la guanidine, pour donner lieu au composé de formule I,
où, dans les composés de formules IV, VI et VII,
R1 à R3 sont chacun tels que définis dans la formule 1, et
R5 est un groupe alcoxy renfermant 1, 2, 3 ou 4 atomes de carbone ;
et des sels de celui-ci.

2. Procédé de préparation de composés de formule I selon la revendication 1, dans laquelle
R1 et R2 sont chacun indépendamment un atome d'hydrogène, un atome de fluor, un atome de chlore, un groupe trifluorométhoxy, un groupe 2,2,2-trifluoroéthoxy, un groupe trifluorométhyle, un groupe 2,2,2-trifluoroéthyle ou un groupe alkyle renfermant 1, 2, 3 ou 4 atomes de carbone ;
R3 est un groupe Alk-R4 ou un groupe trifluorométhyle ;
Alk est un groupe alkyle renfermant 1, 2, 3 ou 4 atomes de carbone ;
R4 est un atome d'hydrogène, un groupe trifluorométhyle ou un groupe cycloalkyle renfermant 3, 4, 5, 6 ou 7 atomes de carbone ;
et de leurs sels;
**caracterisé en ce que** 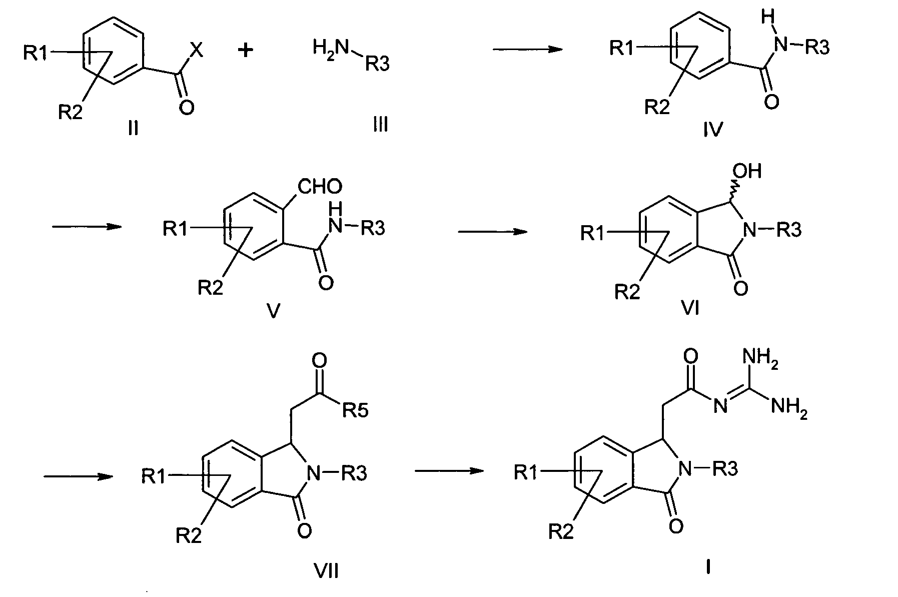
a) le composé de formule II est mis à réagir avec l'amine de formule III pour donner lieu à l'amide de formule IV,
b) l'amide de formule IV est formylé en position ortho par rapport à la fonction amide, pour donner lieu au formylamide de formule V,
c) le formylamide de formule V est cyclisé pour donner lieu au composé de formule VI,
d) le composé de formule VI est mis à réagir avec un alcoxycarbonylméthylènetriphénylphosphorane, avec un 1-alcoxy-1-triméthylsiloxyéthylène ou avec un phosphonoacétate de trialkyle, pour donner lieu au composé de formule VII, et
e) le composé de formule VII est mis à réagir avec de la guanidine pour donner lieu au composé de formule I,
où, dans les composés de formules II, III, IV, V, VI et VII,
R1 à R3 sont chacun tels que définis dans la formule I,
R5 est un groupe alcoxy renfermant 1, 2, 3 ou 4 atomes de carbone, et
X est un atome de chlore, un atome de brome, un groupe OH ou un groupe alcoxy renfermant 1, 2, 3 ou 4 atomes de carbone;
et des sels de celui-ci.

3. Procédé selon la revendication 1 et/ou 2, dans lequel les étapes du procédé sont chacune réalisées indépendamment en continu ou en discontinu.

4. Procédé selon l'une ou plusieurs des revendications 1, 2 ou 3, dans lequel le composé de formule I est défini comme la N-{2-[3-oxo-2-(2,2,2-trifluoroéthyl)-6-trifluorométhyl-2,3-dihydro-1 H-isoindol-1-yl]acétyl}guanidine, et des sels pharmaceutiquement acceptables de celle-ci.

5. Procédé de préparation de composés de formule I 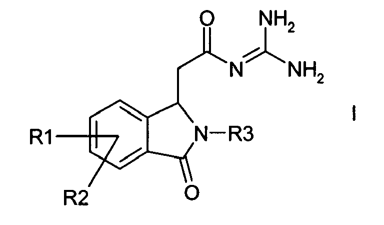 dans laquelle
R1 et R2 sont chacun indépendamment un atome d'hydrogène, un atome de fluor, un atome de chlore, un groupe trifluorométhoxy, un groupe 2,2,2-trifluoroéthoxy, un groupe trifluorométhyle, un groupe 2,2,2-trifluoroéthyle ou un groupe alkyle renfermant 1, 2, 3 ou 4 atomes de carbone ;
R3 est un groupe Alk-R4 ou un groupe trifluorométhyle ;
Alk est un groupe alkyle renfermant 1, 2, 3 ou 4 atomes de carbone ;
R4 est un atome d'hydrogène, un groupe trifluorométhyle ou un groupe cycloalkyle renfermant 3, 4, 5, 6 ou 7 atomes de carbone ;
et de leurs sels ;
**caracterisé en ce que** 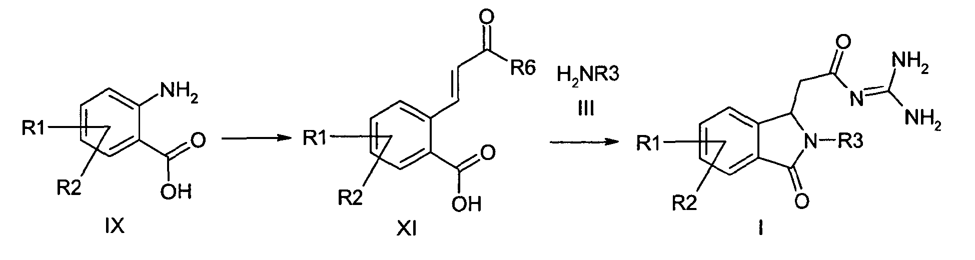
a) l'amine de formule IX est mis à réagir, par l'intermédiaire d'un sel de diazonium, avec un acrylate d'alkyle, pour donner lieu au dérivé de l'acide cinnamique de formule XI,
b) le composé de formule XI est mise à réagir avec l'amine de formule III et avec de la guanidine, pour donner lieu à l'acylguanidine de formule I,
où, dans les composés de formules III, IX et XI,
R1 à R3 sont chacun tels que définis dans la formule I, et
R6 est un groupe alcoxy renfermant 1, 2, 3 ou 4 atomes de carbone ;
et des sels de celle-ci.

6. Procédé de préparation de composés de formule 1 selon la revendication 5, dans laquelle
R1 et R2 sont chacun indépendamment un atome d'hydrogène, un atome de fluor, un atome de chlore, un groupe trifluorométhoxy, un groupe 2,2,2-trifluoroéthoxy, un groupe trifluorométhyle, un groupe 2,2,2-trifluoroéthyle ou un groupe alkyle renfermant 1, 2, 3 ou 4 atomes de carbone ;
R3 est un groupe Alk-R4 ou un groupe trifluorométhyle ;
Alk est un groupe alkyle renfermant 1, 2, 3 ou 4 atomes de carbone ;
R4 est un atome d'hydrogène, un groupe trifluorométhyle ou un groupe cycloalkyle renfermant 3, 4, 5, 6 ou 7 atomes de carbone ;
et de leurs sels ;
**caracterisé en ce que** 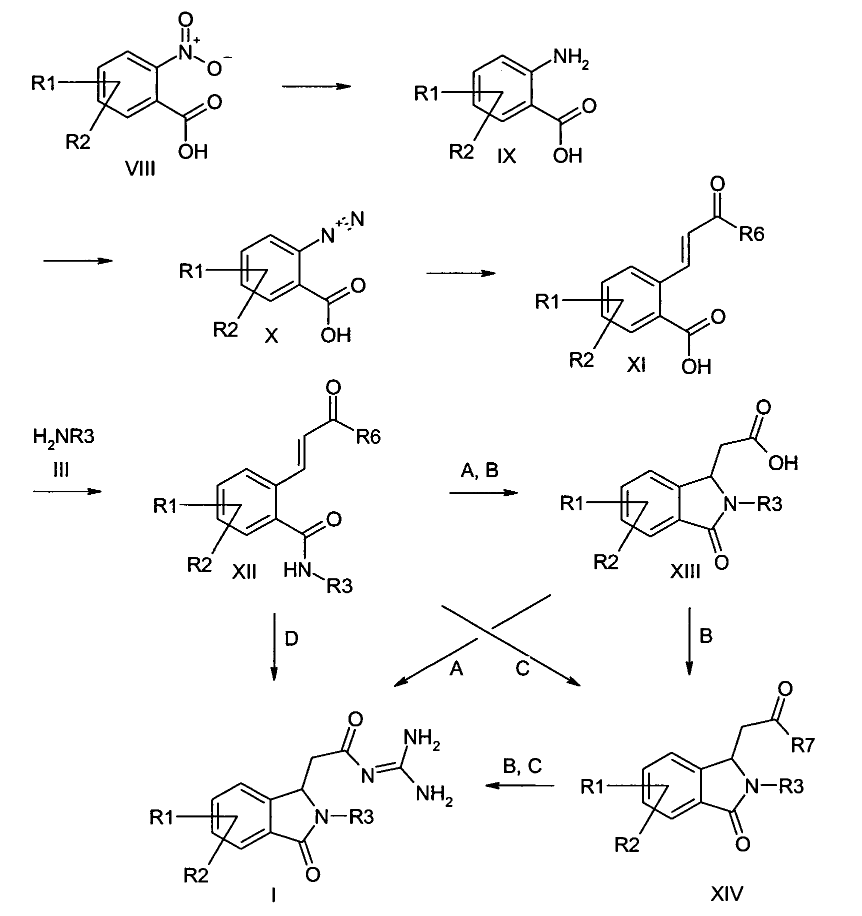
a) le composé nitro de formule VI II est transformé en l'amine de formule IX,
b) l'amine de formule IX est transformée en le sel de diazonium de formule X,
c) le sel de diazonium de formule X est mis à réagir avec un acrylate d'alkyle pour donner lieu au dérivé de l'acide cinnamique de formule XI,
d) le composé de formule XI est transformé en l'amide de formule XII, et
e) le composé de formule XII est transformé en l'acylguanidine de formule I, soit par transformation du composé de formule XI 1, en présence d'une base, pour donner lieu au dérivé de l'iso-indolone de formule XIII, et ensuite par réaction avec de la guanidine avec activation, pour donner lieu à l'acylguanidine de formule I (variante A), soit,
après formation du dérivé de l'iso-indolone de formule XIII, en présence d'une base, à partir du composé de formule XII, par transformation du composé de formule XIII, pour donner lieu à l'ester de formule XIV, et ensuite par réaction avec de la guanidine pour donner lieu à l'acylguanidine de formule I (variante B), soit
par transformation du composé de formule XII, en présence d'une base forte, pour donner lieu à l'ester de formule XIV, et ensuite par réaction avec de la guanidine pour donner lieu à l'acylguanidine de formule I (variante C), soit
par réaction directe du composé de formule XII avec de la guanidine, en présence d'une base, avec une guanylation et une cyclisation ayant lieu simultanément, pour donner lieu à l'isoindolone de formule I (variante D),
où, dans les composés de formules VIII, IX, X, XI, XII, XIII et XIV,
R1 à R3 sont chacun tels que définis dans la formule I, et
R6 et R7 sont chacun indépendamment un groupe alcoxy renfermant 1,2,3 ou 4 atomes de carbone ;
et des sels de celles-ci.

7. Procédé selon la revendication 6, dans lequel, dans l'étape e) du procédé, la variante D est employée.

8. Procédé selon la revendication 6 et/ou 7, dans lequel les étapes d) et e) du procédé sont réalisées dans un procédé en un seul récipient.

9. Procédé selon la revendication 5 et/ou 6, dans lequel les étapes du procédé sont chacune réalisées indépendamment en continu ou en discontinu.

10. Procédé selon l'une ou plusieurs des revendications 5 à 9, dans lequel le composé de formule I est défini comme la N-{2-[3-oxo-2-(2,2,2-trifluoroéthyl)-6-trifluorométhyl-2,3-dihydro-1 H-isoindol-1-yl]acétyl}guanidine, et les sels pharmaceutiquement acceptables de celle-ci.

11. Composés de formule XII 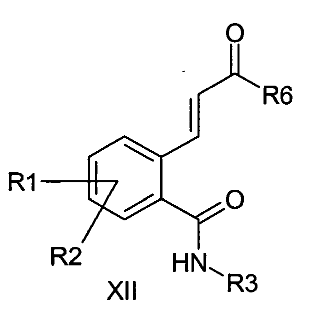 dans laquelle
R1 et R2 sont chacun indépendamment un atome d'hydrogène, un atome de fluor, un atome de chlore, un groupe trifluorométhoxy, un groupe 2,2,2-trifluoroéthoxy, un groupe trifluorométhyle, un groupe 2,2,2-trifluoroéthyle ou un groupe alkyle renfermant 1, 2, 3 ou 4 atomes de carbone ;
R3 est un groupe Alk-R4 ou un groupe trifluorométhyle ;
Alk est un groupe alkyle renfermant 1, 2, 3 ou 4 atomes de carbone ;
R4 est un atome d'hydrogène, un groupe trifluorométhyle ou un groupe cycloalkyle renfermant 3, 4, 5, 6 ou 7 atomes de carbone ;
R6 est un groupe alcoxy renfermant 1, 2, 3 ou 4 atomes de carbone ;
et leurs sels.

12. Composés de formule XII selon la revendication 11, destiné à être utilisé en tant qu'intermédiaire de synthèse.

13. Procédé d'isolation de composés de formules Ia et Ib 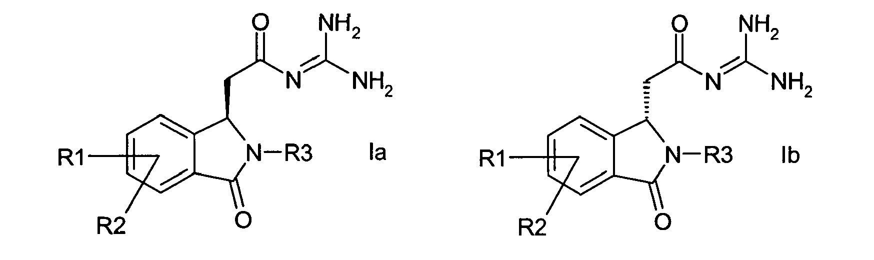 dans lesquelles
R1 et R2 sont chacun indépendamment un atome d'hydrogène, un atome de fluor, un atome de chlore, un groupe trifluorométhoxy, un groupe 2,2,2-trifluoroéthoxy, un groupe trifluorométhyle, un groupe 2,2,2-trifluoroéthyle ou un groupe alkyle renfermant 1, 2, 3 ou 4 atomes de carbone ;
R3 est un groupe Alk-R4 ou un groupe trifluorométhyle ;
Alk est un groupe alkyle renfermant 1, 2, 3 ou 4 atomes de carbone ;
R4 est un atome d'hydrogène, un groupe trifluorométhyle ou un groupe cycloalkyle renfermant 3, 4, 5, 6 ou 7 atomes de carbone ;
et de leurs sels ;
**caracterisé en ce que** 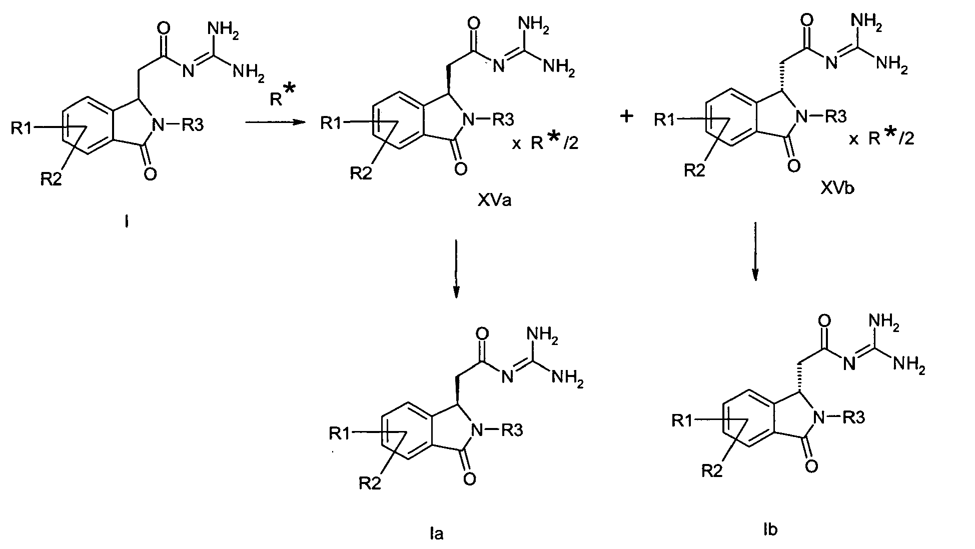
a) le composé de formule I est transformé en sels d'un acide D-tartrique ou L-tartrique 2,3-O-acylé et les deux sels de formules XVa et XVb sont obtenus séparément par cristallisation, et
b) les bases libres de formules Ia et Ib sont libérées à partir des deux sels de formules XVa et XVb, respectivement,
où, dans les composés de formules I, XVa et XVb,
R1 à R3 sont chacun tels que définis dans les formules Ia et Ib,
R* est 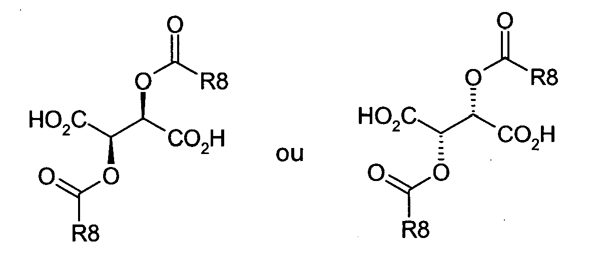 R8 est un groupe alkyle renfermant 1, 2, 3, 4, 5 ou 6 atomes de carbone, ou un groupe phényle qui est non substitué ou substitué par 1, 2 ou 3 substituants parmi le groupe constitué d'un atome de fluor, d'un atome de chlore, d'un atome de brome, d'un atome d'iode, d'un groupe alkyle renfermant 1, 2, 3 ou 4 atomes de carbone ou d'un groupe alcoxy renfermant 1, 2, 3 ou 4 atomes de carbone.

14. Procédé selon la revendication 13, dans lequel l'énantiomère non souhaité de formules Ia ou Ib est de nouveau racémisé.

15. Procédé selon la revendication 13 et/ou 14, dans lequel les composés de formules Ia et Ib sont la (R)-N-{2-[3-oxo-2-(2,2,2-trifluoroéthyl)-6-trifluorométhyl-2,3-dihydro-1H-isoindol-1-yl]acétyl}guanidine et la (S)-N-{2-[3-oxo-2-(2,2,2-trifluoroéthyl)-6-trifluorométhyl-2,3-dihydro-1H-isoindol-1-yl]acétyl}guanidine.

16. Composés de formules XVa et/ou XVb 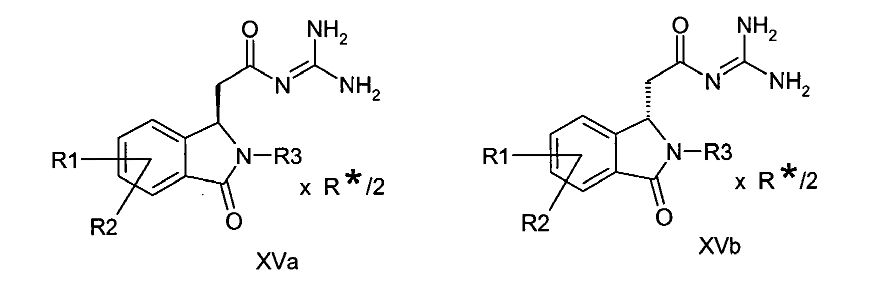 dans lesquelles
R1 et R2 sont chacun indépendamment un atome d'hydrogène, un atome de fluor, un atome de chlore, un groupe trifluorométhoxy, un groupe 2,2,2-trifluoroéthoxy, un groupe trifluorométhyle, un groupe 2,2,2-trifluoroéthyle ou un groupe alkyle renfermant 1, 2, 3 ou 4 atomes de carbone ;
R3 est un groupe Alk-R4 ou un groupe trifluorométhyle ;
Alk est un groupe alkyle renfermant 1, 2, 3 ou 4 atomes de carbone ;
R4 est un atome d'hydrogène, un groupe trifluorométhyle ou un groupe cycloalkyle renfermant 3, 4, 5, 6 ou 7 atomes de carbone ;
R* is 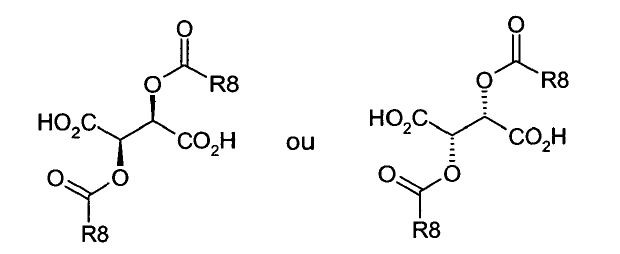
R8 est un groupe alkyle renfermant 1, 2, 3, 4, 5 ou 6 atomes de carbone, ou un groupe phényle qui est non substitué ou substitué par 1, 2 ou 3 substituants parmi le groupe constitué d'un atome de fluor, d'un atome de chlore, d'un atome de brome, d'un atome d'iode, d'un groupe alkyle renfermant 1, 2, 3 ou 4 atomes de carbone, ou d'un groupe alcoxy renfermant 1, 2, 3 ou 4 atomes de carbone.

17. Hydrate d'hydrogénofumarate de (S)-N-{2-[3-oxo-2-(2,2,2-trifluoroéthyl)-6-trifluorométhyl-2,3-dihydro-1H-isoindol-1-yl]acétyl}guanidine de formule XVI

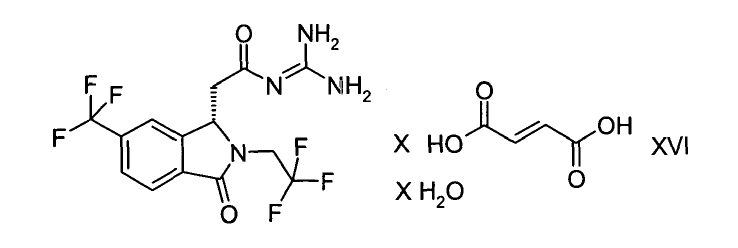

18. Composé de formules XVa ou XVb selon la revendication 16 ou de formule XVI selon la revendication 17, destiné à être utilisé en tant que médicament.

19. Utilisation d'un composé de formules XVa ou XVb selon la revendication 16 ou de formule XVI selon la revendication 17, seul ou en combinaison avec d'autres médicaments ou ingrédients actifs, pour la production d'un médicament destiné au traitement ou à la prophylaxie d'une atteinte aiguë ou chronique, de troubles ou de séquelles indirectes d'organes et de tissus, causés par des événéments ischémiques ou de reperfusion, pour le traitement ou la prophylaxie d'arythmies, d'une fibrillation ventriculaire cardiaque menaçant le pronostic vital, d'un infarctus du myocarde, d'une angine de poitrine, pour le traitement ou la prophylaxie d'états ischémiques du coeur, d'états ischémiques du système nerveux périphérique et central, ou d'un accident vasculaire cérébral ou d'états ischémiques d'organes et de tissus périphériques, pour le traitement ou la prophylaxie d'états de choc, de maladies pour lesquelles une prolifération cellulaire représente une cause primaire ou secondaire, d'un cancer, d'une métastase, d'une hypertrophie de la prostate et d'une hyperplasie de la prostate, d'une athérosclérose ou de troubles du métabolisme lipidique, de l'hypertension, de l'hypertension essentielle, de troubles du système nerveux central, de troubles résultant d'une surexcitabilité du SNC, de l'épilepsie ou de convulsions provoquées centralement, de troubles du système nerveux central, en particulier d'états d'anxiété, de dépressions ou de psychoses, pour le traitement ou la prophylaxie du diabète sucré non insulino-dépendent (NIDDM) ou d'une atteinte tardive due au diabète, de thromboses, de troubles résultant d'un dysfonctionnement endothélial, d'une claudication intermittante, pour le traitement ou la prophylaxie de troubles fibrotiques d'organes internes, de troubles fibrotiques du foie, de troubles fibrotiques du rein, de troubles fibrotiques de vaisseaux et de troubles fibrotiques du coeur, pour le traitement ou la prophylaxie d'une insuffisance cardiaque ou d'une insuffisance cardiaque congestive, de troubles inflammatoires aigus ou chroniques, de troubles causés par des protozoaires, de la malaria ou de la coccidiose chez la volaille, et pour une utilisation pour des opérations chirurgicales et des greffes d'organes, pour la conservation et le stockage de greffes pour des interventions chirurgicales, pour une utilisation dans des opérations de pontage, pour une utilisation en réanimation après un arrêt cardiaque, pour la prévention d'un changement tissulaire lié à l'âge, pour la production d'un médicament orienté contre le vieillissement ou pour prolonger la vie, pour le traitement et la réduction d'effets cardiotoxiques dans le cas d'une thyrotoxicose ou pour la production d'une aide diagnostique.

20. Médicament destiné à une utilisation chez l'homme, vétérinaire et/ou phytoprotectrice, comprenant une quantité efficace d'un composé de formules XVa ou XVb selon la revendication 16, conjointement avec des excipients et des additifs pharmaceutiquement acceptables.

21. Médicament destiné à une utilisation chez l'homme, vétérinaire et/ou phytoprotectrice, comprenant une quantité efficace d'un composé de formules XVa ou XVb selon la revendication 16, conjointement avec des excipients et des additifs pharmaceutiquement acceptables, en combinaison avec d'autres ingrédients actifs pharmacologiques ou médicaments.

22. Médicament destiné à une utilisation chez l'homme, vétérinaire et/ou phytoprotectrice, comprenant une quantité efficace du composé de formule XVI selon la revendication 17, conjointement avec des excipients et des additifs pharmaceutiquement acceptables.

23. Médicament destiné à une utilisation chez l'homme, vétérinaire et/ou phytoprotectrice, comprenant une quantité efficace du composé de formule XVI selon la revendication 17, conjointement avec des excipients et des additifs pharmaceutiquement acceptables, en combinaison avec d'autres ingrédients actifs pharmacologiques ou médicaments.
